# EUROPEAN PATENT APPLICATION

(11) **EP 4 674 855 A1**
(43) Date of publication of application: **07.01.2026**
(21) Application number: 24185671.5
(22) Date of filing: 01.07.2024
(51) Int. Cl.: C07F 5/00, C07D 201/00, C07F 5/02, C07F 5/06, C07F 7/08, C07F 7/30, C07F 9/6564, C07F 9/6568, C07F 9/6571, C07F 9/6578, C07F 9/6584, C07F 9/6596, C09K 11/06, H10K 50/11, H10K 85/00

(54) **A COMPOUND, AN ORGANIC LIGHT EMITTING DIODE COMPRISING THE DERIVATES, A USE OF THE DIODE, AND A CONSUMER PRODUCT**

(71) Applicant: Noctiluca S.A., 81-100 Torun (PL)
(72) Inventor: Zielinska, Alicja, 87-100 Torun (PL); Bosiak, Mariusz, 87-148 Papowo Torunskie (PL)
(74) Representative: Kowalkiewicz, Zuzanna

(57) **Abstract**

The subject of the invention is a compound, an organic light emitting diode comprising the compound, a use of the diode, and a consumer product. The compound can be a compound according to Formula I. Furthermore, the organic light emitting diode (OLED) comprising an anode, cathode, and organic emissive layer, disposed between the anode and cathode, wherein the organic emissive layer includes the compound according to Formula I. The invention also encloses hyper-fluorescence diode (HF-OLED), wherein the emissive layer comprises a fluorescent dopant - N7,N7,N13,N13,5,9,11,15-octaphenyl-5,9,11,15-tetrahydro-5,9,11,15-tetraaza-19b,20b-diboradinaphtho[3,2,1-de:1',2',3'-jk]pentacene-7,13-diamine (v-DABNA) as a final emitter to improve the colour purity in pure emission region. The invention belongs to the field of light-emitting diodes/devices.

## Description

### FIELD

The present disclosure generally relates to a compound, an organic light emitting diode comprising the compound, a use of the diode, and a consumer product. The invention belongs to the field of light-emitting diodes/devices.

### BACKGROUND

Light-emitting devices comprise organic light-emitting diodes (OLED) that are also described as organic light-emitting devices in the state of the art. OLED comprises two electrodes, between which there are organic or organometallic layers, of which at least one is an emissive layer containing at least one chemical compound - an emitter

Organic compounds operating on the basis of the thermally activated delayed fluorescence (TADF) mechanism, belonging to the third generation of emitters, have many advantages over the emitters currently used in OLED. They can be purely organic compounds, need not contain heavy metals, can be more resistant to the burn-in effect and it is possible to develop an efficient and durable emitter. In addition, it is possible to replace the second-generation emitters used today with cheaper TADF emitters in the same production lines.

Conventional TADF emitters possess molecular skeleton with the combination of donor and acceptor derivatives in an appropriate connection. The number of donors and acceptors in molecular design differs based on the requirement, such as emission wavelength and photo-physical parameters. TADF emitters are mainly depends on intra molecular charge transfer (ICT), therefore the emission spectra are notably wide and poor colour purity. A particular group among TADF emitters are multiresonant TADF (MR-TADF) compounds - fused polycyclic aromatic compounds having electron-donating moiety (a donor) and electron-deficient moiety (an acceptor) disposed to each other in a fused planar polycyclic aromatic framework. Conventional TADF emitters constructed using various acceptor moieties are being reported, such as cyano, benzonitrile, sulfone, pyrimidine, triazine, triaryl-boron and oxygen-bridged boron (DBA). DBA acceptor derivatives-based TADF emitters are immensely studied due to their rigid molecular nature and tuneable acceptor strength with certain modifications at the peripheral region. Obtaining high efficiency in pure OLEDs using conventional donor-acceptor type TADF or MR-TADF is challenging in the state of the art.

Since TADF emitters mainly depend on ICT, the emission spectra are notably broad and have poor colour purity to become applicable in next-generation displays. Therefore, a new OLED strategy known as hyper-fluorescence (HF) technology has been proposed to overcome such issues. Here, conventional TADF emitters act as TADF-sensitized host (TSH) for final emitters. Since TSH utilises singlet and triplet excitons, an effective energy conversion through long-range Förster resonance energy transfer (FRET) to the final emitter is an essential phenomenon in HF technology. Therefore, spectral overlap between the emission of TSH and the absorption of the final emitter is requisite to make an efficient energy transfer The final emitter can be either a simple fluorescence or multi-resonance type of fluorescence emitter.

One object of the disclosure is to provide a compound, which is suitable for a use in the diode according to the invention due to good TADF properties such as high photoluminescence quantum yield (PLQY). The subsequent objective of the disclosure is to provide an organic light emitting diode achieving high colour purity and good performances as well as a use of this diode. Yet another aim of the disclosure is to provide a consumer product containing the diode described herein.

### SUMMARY OF THE INVENTION

There is disclosed a compound according to Formula I: 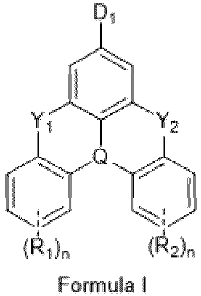 wherein:
Y₁, Y₂, together or independently, are O, S, Se, NR₉, C(R₉)₂ or Si(R₉)₂;
Q, together or independently, are B, P; P=O, Al, Ge, In;
R₁, R₂, R₃, R₄, R₅, R₆, R₇, R₈, together or independently, are hydrogen, deuterium, halogen, cyano, nitro, C₁-C₂₀ alkyl, C₂-C₂₀ alkenyl, C₂-C₂₀ alkynyl, C₁-C₆₀ alkoxy group, C₃-C₆₀ cycloalkyl, C₃-C₆₀ cycloalkenyl, C₁-C₆₀ heterocycloalkyl, C₁-C₆₀ heterocycloalkenyl group, C₃-C₆₀ cycloalkynyl group, C₁-C₆₀ heterocycloalkynyl group, hydroxyl, C₆-C₆₀ aryl, C₁-C₆₀ heteroaryl group, C₅-C₆₀ aryloxy, C₁-C₆₀ heteroaryloxy group, C₂-C₆₀ ether group, C₆-C₆₀ thioaryl, C₁-C₆₀ heterothioaryl, C₁-C₆₀ amino, C₁-C₆₀ alkylamino, C₁-C₆₀ dialkylamino, C₆-C₆₀ arylamino, C₆-C₆₀ diarylamino, C₆-C₆₀ heteroarylamino group, C₆-C₆₀ heterodiarylamino group, C₄-C₆₀ non-aromatic fused polycyclic group, C₁-C₆₀ non-aromatic fused heteropolycyclic group or deuterated analogues thereof;
R₉, R₁₀, together or independently, are hydrogen, deuterium, C₁-C₂₀ alkyl, C₆-C₆₀ aryl group, C₁-C₆₀ heteroaryl group, C₅-C₆₀ aryloxy, C₁-C₆₀ heteroaryloxy group, C₆-C₆₀ arylamino, C₆-C₆₀ heteroarylamino, C₆-C₆₀ diarylamino, C₆-C₆₀ heteroarylamino group, C₆-C₆₀ diheterodiarylamino group, C₆-C₆₀ thioaryl group, C₁-C₆₀ heterothioaryl or deuterated analogues thereof;
an index n ranging from 0 - 4 indicates how many R₁ and R₂ may be attached to a particular ring of Formula I;
D₁ is selected from Formula IIa - Formula IIl: 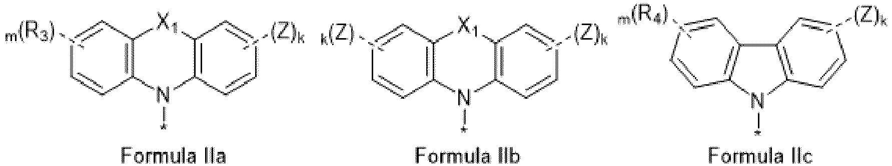 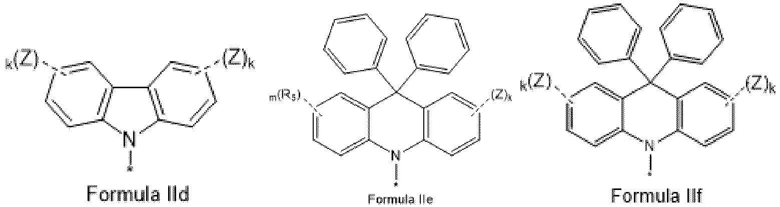 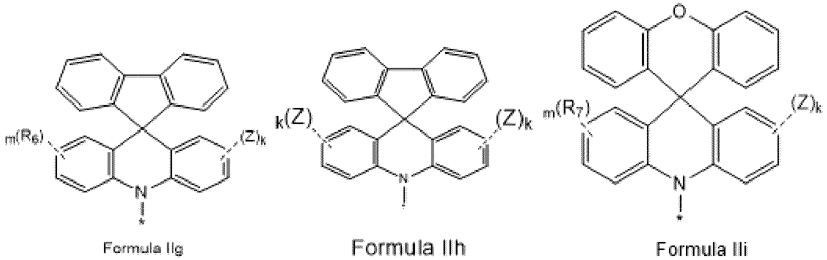 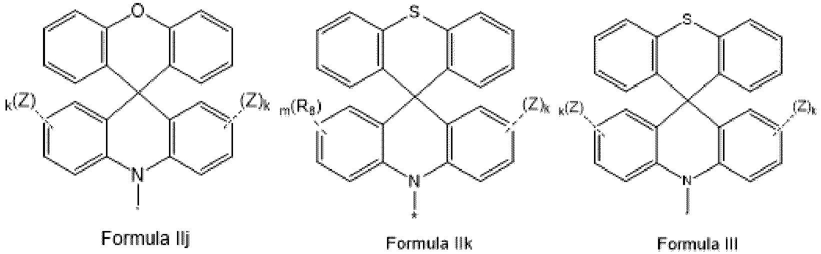 wherein:
   X₁ is selected from C(R₁₀)₂, Si(R₁₀)₂, S, O, NR₁₀;
   Z is: 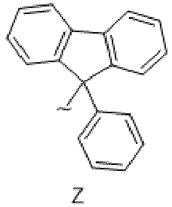
   an index m ranging from 0 - 4 indicates how many R₃, R₄, R₅, R₆, R₇, R₈ may be attached to a particular ring of Formula IIa, Formula IIb, Formula IIc, Formula IId, Formula IIe, Formula IIf, Formula IIg, Formula IIh, Formula IIi, Formula IIj, Formula IIk, or Formula IIl;
   an index k ranging from 0 - 4 indicates how many Z may be attacked to a particular ring of Formula IIa, Formula IIb, Formula IIc, Formula IId, Formula IIe, Formula IIf, Formula IIg, Formula IIh, Formula IIi, Formula IIj, Formula IIk, or Formula IIl;
   ~ each indicate a binding site of Z to Formula IIa, Formula IIb, Formula IIc, Formula IId, Formula IIe, Formula IIf, Formula IIg, Formula IIh, Formula IIi, Formula IIj, Formula IIk, and Formula IIl;
   * each indicate a binding site of Formula IIa, Formula IIb, Formula IIc, Formula IId, Formula IIe, Formula IIf, Formula IIg, Formula IIh, Formula IIi, Formula IIj, Formula IIk, and Formula III to Formula I.
In a preferred embodiment of the invention, the compound according to the invention is selected from 1 - 483.
An organic light emitting diode (OLED) according to the invention comprising an anode, cathode, and organic emissive layer, disposed between the anode and cathode, wherein the organic emissive layer comprising a compound according to the invention.
In a preferred embodiment of the invention, the organic emissive layer comprising v-DABNA as a fluorescent dopant. In a preferred embodiment of the invention, the diode comprising a host is selected from H1 - H163 and deuterated analogues thereof.
In a preferred embodiment of the invention, the diode comprising at least one of layer between the anode and cathode selected from a hole injection layer; hole transport layer; electron transport layer; electron blocking layer; hole blocking layer; electron injection layer; or a combination thereof.
In a preferred embodiment of the invention, the diode comprising a matrix, preferably the matrix is passive or active. In a preferred embodiment of the invention, the diode emits light with a maximum wavelength of 350 nm to 2 500 nm, more preferably with a maximum wavelength of 400 nm to 750 nm.
Use of the diode according to the invention as a display screen or illumination device, an electroluminescent display device, an organic photovoltaic device, or a sensing device.
A consumer product comprises the diode according to the invention, wherein the product is selected from a flat panel display, a computer monitor, a medical monitor, a television, a billboard, a light for interior or exterior illumination/signalling, a heads-up display, a fully or partially transparent display, a flexible display, a laser printer, a telephone, a cell phone, tablet, a phablet, a personal digital assistant, a wearable device, a laptop computer, a watch, a backlight, a digital camera, a camcorder, a 3-D display, a virtual reality or augmented reality display, a vehicle, a video walls comprising multiple displays tiled together, a theatre, stadium screen, and a sign.

One of significant issues associated with emitters is aggregation-caused quenching due to the planarity of the molecules. In film state, most reported emitters show strong aggregation caused by π-π interaction between the (inter)molecules, which results in spectral broadening, bathochromic shifted emission and poor PLQY. Consequently, such effects worsen with an increment of doping concentration and lead to severe efficiency roll-off during the device operations. Therefore, TADF emitters are usually embedded in low concentration (~25 wt%) with the host matrix to suppress the device's exciton annihilation and emission quenching.

Unexpectedly, the compound according to the invention having an acceptor moiety and a carbazole, acridine, or spiro-acridine derivate donor moiety that is substituted with 9-phenyl-9-fluorenyl without disturbing the donor strength much (other photo-physical parameters) in the comparison to a donor-acceptor moiety unsubstituted with 9-phenyl-9-fluorenyl. There are designed pure emitters in unsymmetrical or symmetrical molecular skeletons through extending the donor moiety trough aryl-methylation with 9-phenyl-9-fluorenyl in cooperation with the acceptor moiety. This molecular design causes the compound to undergo further twisting between the acceptor moiety and the donor moiety compared to that of the donor-acceptor moiety unsubstituted with 9-phenyl-9-fluorenyl. The introduction of phenyl-9-fluoronyl unit, the intermolecular distance between the molecules is assumed to be increased, as a result, the aggregation behaviour of the molecule can be minimized.

The emitters according to the invention can enhance a device efficiency, without changing the main photo-physical parameters. If the donor strength does not change significantly, then is suitable for HF system through effective spectral overlapping. Unexpectedly, the designed compounds according to the invention can also be employed as TADF sensitized host (TSH) for effective hyper-fluorescence system, where fluorescent dopant (FD) is N7,N7,N13,N13,5,9,11,15-octaphenyl-5,9,11,15-tetrahydro-5,9,11,15-tetraaza-19b,20b-diboradinaphtho[3,2,1-de: 1',2',3'-jk]pentacene-7,13-diamine (v-DABNA) as a final emitter to improve the colour purity in pure emission region.

### General definition of terms and substituents

As used herein, the terms "or" and "and/or" include any and all combinations of one or more of the associated listed items. The term "or" is not an exclusive term, e.g., A or B means: A, B, or A and B. The expression "at least one" comprises at least one, two, three, four, five and more mentioned elements (etc.).

It should be understood that the terms "comprises," "comprising," "includes," "including," "has", "have," "having," "contains," "containing," and the like are intended to specify the presence of stated features, integers, steps, operations, elements, components, or combinations thereof in the disclosure, but do not preclude the presence or addition of one or more other features, integers, steps, operations, elements, components, or combinations thereof.

Certain chemical compounds can have one or more hydrogen atoms of the compound replaced by one or more deuterium atoms including perdeuterated analogues. Further, it should be noted that the term "deuterated analogue" as used herein refers to compounds where at least one hydrogen atom has been replaced by a deuterium atom. The term "deuterated" as used herein alone or as part of a group, means substituted deuterium atoms. When a particular position is designated as holding deuterium (stated as "D" or "deuterium"), it is understood that the abundance of deuterium at that position is substantially greater than the natural abundance of deuterium, which is 0.015% (i.e., at least 50.1% incorporation of deuterium). The deuterated analog of the present disclosure may be a fully or partially deuterium substituted derivative. For example, the deuterium substituted compound of the present disclosure can hold a fully or partially deuterium substituted alkyl, aryl or heteroaryl group. In one embodiment, the deuterium substituted compound of the present disclosure holds a fully or partially deuterium substituted alkyl group, e.g., -CD₃, CD₂, CD₃, -CD₂, and the like. In another embodiment, the deuterium substituted compound of the present disclosure holds a fully or partially deuterium substituted aryl, such as phenyl, e.g., CeDs or a fully or partially deuterium substituted heteroaryl, e.g., pyridyl-d₃, and the like.

The term "Ph" used herein refers to a phenyl group.

Organic groups include both substituted and unsubstituted forms of such groups unless otherwise denoted. The term "substituted" refers to at least one hydrogen replaced by deuterium, halogen, C₁-C₆₀ alkyl, C₂-C₆₀ alkenyl, C₂-C₆₀ alkynyl, C₁-C₆₀ alkoxy group, C₃-C₆₀ cycloalkyl, C₃-C₆₀ cycloalkenyl, C₁-C₆₀ heterocycloalkyl, C₁-C₆₀ heterocycloalkenyl, C₃-C₆₀ cycloalkynyl group, C₁-C₆₀ heterocycloalkynyl group, hydroxyl, C₂-C₆₀ ether group, C₅-C₆₀ aryloxy, C₆-C₆₀ thioaryl, C₁-C₆₀ heterothioaryl, C₁-C₆₀ amino, C₁-C₆₀ alkylamino, C₁-C₆₀ dialkylamino, C₆-C₆₀ arylamino, cyano, nitro, hydrazine, hydrazone, C₆-C₆₀ diarylamino, C₆-C₆₀ heteroarylamino group, C₆-C₆₀ heterodiarylamino group, C₆-C₆₀ aryl, C₁-C₆₀ heteroaryl group, C₁-C₆₀heteroaryloxy group, C₄-C₆₀ non-aromatic fused polycyclic group, C₁-C₆₀ non-aromatic fused heteropolycyclic group, or deuterated analogues thereof.

The term "C₁-C₆₀ alkyl group" as used herein refers to a linear or branched aliphatic saturated hydrocarbon group having 1 to 60 carbon atoms, and examples thereof include a methyl group, an ethyl group, a propyl group, an isopropyl group, a butyl group, an isobutyl group, a sec-butyl group, a *tert*-butyl group, a pentyl group, an isoamyl group, a neopentyl group, a hexyl group, a 2-methylpentyl group, a 3-methylpentyl group, a heptyl group, a 4-methylhexyl group, a 2,2-dimethylpentyl group, an octyl group, a 3-methylheptyl group, a nonyl group, a 4-methyloctyl group, a 3-ethylheptyl group, a decyl group, a 2-methylnonyl group, a 3,3-dimethyloctyl group, an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a heptatriacontyl group, an octatriacontyl group, a nonatriacontyl group, a tetracontyl group, a hentetracontyl group, a dotetracontyl group, a tritetracontyl group, a tetratetracontyl group, a pentatetracontyl group, a hexatetracontyl group, a heptatetracontyl group, an octatetracontyl group, a nonatetracontyl group, a pentacontyl group, a hentapentacontyl group, a dotriacontyl group, a tritriacontyl group, a tetratetracontyl group, a pentapentacontyl group, a hexapentacontyl group, a heptapentacontyl group, a octapentacontyl group, a nonapentacontyl group, a hexacontyl group and others.

The term "C₁-C₂₀ alkyl group" as used herein refers to a linear or branched aliphatic saturated hydrocarbon group having 1 to 20 carbon atoms. The term "C₆-C₁₀ alkyl group" as used herein refers to a linear or branched saturated hydrocarbon group having 6 to 10 carbon atoms.

The term "C₂-C₆₀ alkenyl group" as used herein refers to a linear or branched aliphatic unsaturated hydrocarbon group having at least one carbon-carbon double bond in the middle or at the terminus of the C₂-C₆₀ alkyl group, and examples thereof include an ethenyl group, a propenyl group, a isopropenyl group, a butenyl group, a 2-methyl-1-propenyl group, a 2-methyl-2-butenyl group, a 3-methyl-2-butenyl group, a pentenyl group, a 4-methyl-2-butenyl group, a hexenyl group, a 2,3-dimethyl-2-butenyl group, a 4,4-dimethyl-1-butenyl group, a heptenyl group, a 3,4-dimethyl-3-butenyl group, an octenyl group, a 2,3-dimethyl-2-hexenyl group, a nonenyl group, a 2-methyl-3-heptenyl group, a decenyl group, a 4-ethyl-4-octenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, an eicosenyl group, a heneicosenyl group, a docosenyl group, a tricosenyl group, a tetracosenyl group, a pentacosenyl group, a hexacosenyl group, a heptacosenyl group, an octacosenyl group, a nonacosenyl group, a triacontenyl group, a hentriacontenyl group, a dotriacontenyl group, a tritriacontenyl group, a tetratriacontenyl group, a pentatriacontenyl group, a hexatriacontenyl group, a heptatriacontenyl group, an octatriacontenyl group, a nonatriacontenyl group, a tetracontenyl group, a hentetracontenyl group, a dotetracontenyl group, a tritetracontenyl group, a tetratetracontenyl group, a pentatetracontenyl group, a hexatetracontenyl group, a heptatetracontenyl group, an octatetracontenyl group, a nonatetracontenyl group, a pentacontenyl group, a hentapentacontenyl group, a dotriacontenyl group, a tritriacontenyl group, a tetratetracontenyl group, a pentapentacontenyl group, a hexapentacontenyl group, a heptapentacontenyl group, an octapentacontenyl group, a nonapentacontenyl group, a hexacontenyl group and others.

The term "C₂-C₂₀ alkenyl group" as used herein refers to a linear or branched aliphatic unsaturated hydrocarbon group having 2 to 20 carbon atoms, wherein at least one carbon-carbon double bond is in the middle or at the terminus of the C₂-C₂₀ alkyl group. The term "C₆-C₁₀ alkenyl group" as used herein refers to a linear or branched aliphatic unsaturated hydrocarbon group having 6 to 10 carbon atoms, wherein at least one carbon-carbon double bond is in the middle or at the terminus of the C₂-C₁₀ alkyl group.

The term "C₂-C₆₀ alkynyl group" as used herein refers to a linear or branched aliphatic unsaturated hydrocarbon group having at least one carbon-carbon triple bond in the middle or at the terminus of the C₂-C₆₀ alkyl group, and examples thereof include an ethynyl group, and a propynyl group, a butynyl group, , a pentynyl group, a 3-ethyl-1-butynyl group, a hexynyl group, a 3,3-dimethyl-1-butynyl group, a heptynyl group, a 3-propyl-1-pentynyl group, an octynyl group,a 3,3-dimethyl-4-hexynyl group, a nonynyl group, a 3,3-dimethyl-4-heptynyl group, a decynyl group, a 2-ethyl-3-octynyl group, an undecynyl group, a dodecynyl group, a tridecynyl group, a tetradecynyl group, a pentadecynyl group, a hexadecynyl group, a heptadecynyl group, an octadecynyl group, a nonadecynyl group, an eicosynyl group, a heneicosynyl group, a docosynyl group, a tricosynyl group, a tetracosynyl group, a pentacosynyl group, a hexacosynyl group, a heptacosynyl group, an octacosynyl group, a nonacosynyl group, a triacontynyl group, a hentriacontynyl group, a dotriacontynyl group, a tritriacontynyl group, a tetratriacontynyl group, a pentatriacontynyl group, a hexatriacontynyl group, a heptatriacontynyl group, an octatriacontynyl group, a nonatriacontynyl group, a tetracontynyl group, a hentetracontynyl group, a dotetracontynyl group, a tritetracontynyl group, a tetratetracontynyl group, a pentatetracontynyl group, a hexatetracontynyl group, a heptatetracontynyl group, an octatetracontynyl group, a nonatetracontynyl group, a pentacontynyl group, a hentapentacontynyl group, a dopentacontynyl group, a tritpentacontynyl group, a tetrapentacontynyl group, a pentapentacontynyl group, a hexapentacontynyl group, a heptapentacontynyl group, an octapentacontynyl group, a nonapentacontynyl group, a hexacontynyl group, and others.

The term "C₂-C₂₀ alkynyl group" as used herein refers to a linear or branched aliphatic unsaturated hydrocarbon group having 2 to 20 carbon atoms, wherein at least one carbon-carbon triple bond is in the middle or at the terminus of the C₂-C₂₀ alkynyl group. The term "C₆-C₁₀ alkynyl group" as used herein refers to a linear or branched aliphatic unsaturated hydrocarbon group having 6 to 10 carbon atoms, wherein at least one carbon-carbon triple bond is in the middle or at the terminus of the C₂-C₁₀ alkynyl group.

The term "C₃-C₆₀ cycloalkyl group" as used herein refers to a monocyclic or polycyclic and non-aromatic saturated group that has 3 to 60 carbon atoms. When the C₃-C₆₀ cycloalkyl group includes two or more rings, the rings may be fused to each other. The examples thereof include a cyclopropyl group, a cyclobutyl group, a cyclopentyl group, a cyclohexyl group, and a cycloheptyl group, a cyclooctyl group, a cyclononyl group, a cyclodecyl group, a cycloundecyl group, a cyclododecyl group, a cyclotridecyl group, a cyclotetradecyl group, a cyclopentadecyl group, a cyclohexadecyl group, a cycloheptadecyl group, a cyclooctadecyl group, a cyclononadecyl group, a cycloeicosyl group, a cycloheneicosyl group, a cyclodocosyl group, a cyclotricosyl group, a cyclotetracosyl group, a cyclopentacosyl group, a cyclohexacosyl group, a cycloheptacosyl group, a cyclooctacosyl group, a cyclononacosyl group, a cyclotriacontyl group, a cyclohentriacontyl group, a cyclodotriacontyl group, a cyclotritriacontyl group, a cyclotetratriacontyl group, a cyclopentatriacontyl group, a cyclohexatriacontyl group, a cycloheptatriacontyl group, a cyclooctatriacontyl group, a cyclononatriacontyl group, a cyclotetracontyl group, a cyclohentetracontyl group, a cyclodotetracontyl group, a cyclotritetracontyl group, a cyclotetratetracontyl group, a cyclopentatetracontyl group, a cyclohexatetracontyl group, a cycloheptatetracontyl group, a cyclooctatetracontyl group, a cyclononatetracontyl group, a cyclopentacontyl group, a cyclohentapentacontyl group, a cyclodopentacontyl group, a cyclotritpentacontyl group, a cyclotetrapentacontyl group, a cyclopentapentacontyl group, a cyclohexapentacontyl group, a cycloheptapentacontyl group, a cyclooctapentacontyl group, a cyclononapentacontyl group, a cyclohexacontyl group, and others.

The term "C₁-C₆₀ heterocycloalkyl group" as used herein refers to a cycloalkyl group having at least one heteroatom selected from N, O, Si, P, and S as a ring-forming atom and 1 to 60 carbon atoms. When C₁-C₆₀ heterocycloalkyl group includes two or more rings, the rings may be fused to each other. The examples thereof include 1,2,3,4-oxatriazolidinyl group, a tetrahydrofuranyl group, and a tetrahydrothiophenyl group, a pyrrolidinyl group, an oxetanyl group, a thiiranyl group, a piperidinyl group, an azetidinyl group, a silacyclopropyl group, a furan group, a pyridinyl group, a dithianyl group, a phospholanyl group, a thiazolyl group, a siloxanyl group, a morpholinyl group, an oxazolyl group, a thiophenyl group, and an aziridinyl group, tetrahydropyranyl group, a silacyclohexyl group, a phosphabicycloheptyl group, and others.

The term "C₃-C₆₀ cycloalkenyl group" used herein refers to a monocyclic or polycyclic and non-aromatic unsaturated group that has 3 to 60 carbon atoms and at least one carbon-carbon double bond in the ring thereof. When the C₃-C₆₀ cycloalkenyl group includes two or more rings, the rings may be fused to each other. The examples thereof include a cyclopropenyl group, a cyclobutenyl group, a cyclopentenyl group, a cyclohexenyl group, a cycloheptenyl group, a cyclooctenyl group, a cyclononenyl group, a cyclodecenyl group, a cycloundecenyl group, a cyclododecenyl group, a cyclotridecenyl group, a cyclohexadecenyl group, a cyclooctadecenyl group, a cyclononadecenyl group, a cycloeicosenyl group, a cycloheneicosenyl group, a cyclodocosenyl group, a cyclotriacosenyl group, a cyclotetracosenyl group, a cyclopentacosenyl group, a cyclohexacosenyl group, a cycloheptacosenyl group, a cyclooctacosenyl group, a cyclononacosenyl group, a cyclotriacontenyl group, a cyclohentriacontenyl group, a cyclodotriacontenyl group, a cyclotritriacontenyl group, a cyclotetratriacontenyl group, a cyclopentatriacontenyl group, a cyclohexatriacontenyl group, a cycloheptatriacontenyl group, a cyclooctatriacontenyl group, a cyclononatriacontenyl group, a cyclotetracontenyl group, a cyclohentetracontenyl group, a cyclodotetracontenyl group, a cyclotritetracontenyl group, a cyclotetratetracontenyl group, a cyclopentatetracontenyl group, a cyclohexatetracontenyl group, a cycloheptatetracontenyl group, a cyclooctatetracontenyl group, a cyclononatetracontenyl group, a cyclopentacontenyl group, a cyclohentapentacontenyl group, a cyclodopentacontenyl group, a cyclotritpentacontenyl group, a cyclotetrapentacontenyl group, a cyclopentapentacontenyl group, a cyclohexapentacontenyl group, a cycloheptapentacontenyl group, a cyclooctapentacontenyl group, a cyclononapentacontenyl group a cyclohexacontenyl group and others.

The term "C₁-C₆₀ heterocycloalkenyl group" as used herein refers to a cycloalkenyl group that has at least one heteroatom selected from N, O, Si, P, and S as a ring-forming atom, having 1 to 60 carbon atoms, and at least one double bond in its ring. When the C₁-C₆₀ heterocycloalkenyl group includes two or more rings, the rings may be fused to each other. The examples of thereof include a 4,5-dihydro-1,2,3,4-oxatriazolyl group, a 2,3-dihydrofuranyl group, a 2,3-dihydrothiophenyl group, a pyrrolenyl group, a furanyl group, a thiophenyl group, a pyrrolopyridenyl group, an oxetanyl group, an azetidinyl group, a dioxasilolanyl group, a phospholanyl group, a pyrrolizidinyl group, an oxepinyl group, a thiepinyl group, a silacyclohexenyl group, a piperidinylidene group, an oxocyclooctenyl group, an azononacyclohexenyl group and others.

The term "C₃-C₆₀ cycloalkynyl group" used herein refers to a monocyclic or polycyclic and non-aromatic unsaturated group that has 3 to 60 carbon atoms and at least one carbon-carbon triple bond in the ring thereof. When the C₃-C₆₀ cycloalkynyl group includes two or more rings, the rings may be fused to each other. The examples thereof include a cyclopropynyl group, a cyclobutynyl group, a cyclopentynyl group, a cyclohexynyl group, a cycloheptynyl group, a cyclooctynyl group, a cyclononylyl group, a cyclodecynyl group, a cycloundecynyl group, a cyclododecynyl group, a cyclotridecynyl group, a cyclotetradecynyl group, a cyclopentadecynyl group, a cyclohexadecynyl group, a cycloheptadecynyl group, a cyclooctadecynyl group, a cyclononadecynyl group, a cycloeicosynyl group, a cycloheneicosynyl group, a cyclodocosynyl group, a cyclotriacosynyl group, a cyclotetracosynyl group, a cyclopentacosynyl group, a cyclohexacosynyl group, a cycloheptacosynyl group, a cyclooctacosynyl group, a cyclononacosynyl group, a cyclotriacontynyl group, a cyclohentriacontynyl group, a cyclodotriacontynyl group, a cyclotritriacontynyl group, a cyclotetratriacontynyl group, a cyclopentatriacontynyl group, a cyclohexatriacontynyl group, a cycloheptatriacontynyl group, a cyclooctatriacontynyl group, a cyclononatriacontynyl group, a cyclotetracontynyl group, a cyclohentetracontynyl group, a cyclodotetracontynyl group, a cyclotritetracontynyl group, a cyclotetratetracontynyl group, a cyclopentatetracontynyl group, a cyclohexatetracontynyl group, a cycloheptatetracontynyl group, a cyclooctatetracontynyl group, a cyclononatetracontynyl group, a cyclopentacontynyl group, a cyclohentapentacontynyl group, a cyclodopentacontynyl group, a cyclotritpentacontynyl group, a cyclotetrapentacontynyl group, a cyclopentapentacontynyl group, a cyclohexapentacontynyl group, a cycloheptapentacontynyl group, a cyclooctapentacontynyl group, a cyclononapentacontynyl group a cyclohexacontynyl group and others.

The term "C₁-C₆₀ heterocycloalkynyl group" as used herein refers to a cycloalkynyl group that has at least one heteroatom selected from N, O, Si, P, and S as a ring-forming atom, having 1 to 60 carbon atoms, and at least one triple bond in its ring. When the C₁-C₆₀ heterocycloalkynyl group includes two or more rings, the rings may be fused to each other. The examples thereof include a pyridinylcyclopropynyl group, an oxazolylcyclobutynyl group, a thiophenylcyclopentynyl group, a silylcyclohexynyl group, a phosphacycloheptynyl group, a pyrazinylcyclooctynyl group, an oxacyclononynyl group, a thiasilacyclodecynyl group, an isoxazolylcycloheptenyl group, a pyrimidinylcyclopentadienyl group, a triazinylcyclohexadienyl group, a phosphoranylpyridinylcycloheptenyl group, an oxathiacyclononadienyl group, a silylpyrazinylcyclooctadienyl group, a pyridazinylcyclooctatrienyl group, an isothiazolylsilylcyclononatrienyl group, a thiophenylphosphacyclodecatrienyl group, a pyrimidinylsilylcycloheptadienyl group, an oxazinylphosphoranylcyclodecadienyl group, a silylthiasilacyclohexatrienyl group

The term "C₆-C₆₀ aryl group" as used herein refers to a carbocyclic aromatic group having 6 to 60 carbon atoms. When the C₆-C₆₀ aryl group includes two or more rings, the rings may be fused to each other. The examples thereof include a phenyl group, a naphthyl group, an anthracenyl group, a phenanthrenyl group, a pyrenyl group, a chrysenyl group, a biphenyl group, a triphenylenyl group, a fluorenyl group, a perylenyl group, a coronene-ovalenyl group, a dibenzo[a,e]pentalenyl group, a tetrabenzo[a,c,f,h]pentaphenyl group, a hexabenzo[a,c,f,h,k,n]pentadecaphenyl group, an octabenzo[a,c,f,h,k,n,q,u]icosaphenyl group, a toluyl group, a xylyl group, a diphenylmethyl group, a terphenyl group and others.

The term "C₁-C₆₀ heteroaryl group" as used herein refers to an aryl group having a at least one heteroatom selected from N, O, Si, P, and S as a ring-forming atom, and having 1 to 60 carbon atoms. When the C₁-C₆₀ heteroaryl group includes two or more rings, the rings may be fused with each other. The examples thereof include a pyrimidinyl group, a pyrazinyl group, a pyridazinyl group, a triazinyl group, a quinolinyl group, an isoquinolinyl group, a furan-2-yl group, a thiophen-3-yl group, an imidazol-4-yl group, a thiazol-2-yl group, an oxazol-5-yl group, a pyrrole-2-yl group, an indol-3-yl group, a benzofuran-4-yl group, a benzothiophen-2-yl group, a benzimidazol-1-yl group, a benzothiazol-4-yl group, an isoxazol-3-yl group, a pyridin-4-ylmethyl group, a quinoxalin-2-yl group, a naphtho[1,2-d]imidazol-4-yl group, a tetrazol-1-yl group, a 1,2,3-triazol-4-yl group, a pyridin-2-ylmethoxy group, a thieno[2,3-c]pyridin-4-yl group, a 3,4-dihydro-2H-pyran-2-yl group, a 1,3-dioxol-2-yl group, a pyrazolo[1,5-a]pyridin-3-yl group, a benzofuran-2-ylmethyl group, a carbazole group, an indolocarbazole group, a phenoxazine group, a phenothiazine group, a benzimidazoquinazoline group, a pyridocarbazole group, a pyrrolopyrazine group, a quinoxaline-2,3-dione group, an isoxazole-3,5-dione group, an indole-3-carboxylic acid group, a dibenzofuranone group, a pyrroloquinoxaline group, an imidazo[1,2-a]pyrimidine group, a thiazolo[4,5-d]pyrimidine group, a benzothiazole-5-sulfonamide group, a 1H-pyrazolo[3,4-b]quinolin-3-amine group, a furo[3,2-c]pyridin-4(5H)-one group, a 1,3-dihydro-2H-indol-2-one group, a 2H-indazole-3-carboxylic acid group, a pyrazolo[1,5-a]pyrimidin-3-amine group, a 3H-indole-4-carbonitrile group, a benzoxazin-3-one group, a benzothiophene-2-carboxylic acid group, an isothiazole-3-carboxylic acid group, a pyrido[2,3-b]pyrazin-7-one group, a 3H-quinazolin-4-one group, a benzofurazan-5-amine group, a 1,2,4-triazolo[4,3-a]pyridine group, a 1,3,4-oxadiazole-2(3H)-thione group, a 2H-thiopyran-4,6-dione group, a pyrrolo[1,2-a]quinoxaline-4,7-dione group, a silylpyridine group, a phosphorylpyrazole group, an oxiranylpyrimidine group, a thioxanthyl group, a siloxazinyl group, a phosphonaphthyl group, a dioxaphenanthrenyl group, a thiophenylsiloxane group, an oxatriazinyl group, a silylpyrrole-2-carboxylic acid group, a phosphinophenoxazine group, a silathiazolyl group, an oxadithienyl group, a phosphabenzimidazole group, a silaquinolinyl group, an oxepino[2,3-b]pyridinyl group, a dioxaphenanthrenylpyrazinyl group, a phosphorothiazolidinyl group, a silaisoxazolyl group, an oxazolidinylpyridyl group and others.

The term "C₁-C₆₀ alkoxy group" as used herein refers to an alkyl group which is singularly bonded to oxygen forming thus R-O-. The examples thereof include a methoxy group, an ethoxy group, and an isopropyloxy group, a propoxy group, a butoxy group, a tert-butoxy group, a sec-butoxy group, an iso-butoxy group, a pentoxy group, an iso-amyloxy group, a neo-pentoxy group, a hexoxy group, a heptoxy group, an octoxy group, a nonoxy group, a decoxy group, an undecoxy group, a dodecoxy group, a pentadecoxy group, an eicosoxy group, a pentacosoxy group, a triacontoxy group, a pentatriacontoxy group, a tetracontoxy group, a pentatetracontoxy group, a pentacontoxy group, a pentapentacontoxy group, and a hexacontoxy group and others.

The term "C₅-C₆₀ aryloxy group" as used herein refers to an aryl which is singularly bonded to oxygen forming thus Ar-O-. The examples thereof include include a methoxyphenyl group, an ethoxyphenyl group, a propoxyphenyl group, an isopropoxyphenyl group, a butoxyphenyl group, a pentoxyphenyl group, a hexoxyphenyl group, a heptoxyphenyl group, an octoxyphenyl group, a nonoxyphenyl group, a decoxyphenyl group, a dodecoxyphenyl group, a hexadecoxyphenyl group, a triacontaoxyphenyl group, a hexacontaoxyphenyl group, a phenylmethoxy group, a naphthoxy group, a biphenyloxy group, an anisoyloxy group, a toluoyloxy group, a xylyloxy group, a benzoyloxy group, a napththoyloxy group, an anthryloxy group, a perylenyloxy group, a fluorenyloxy group, a triphenyloxy group, a phenoxyphenyl group, a naphthoxyphenyl group, a pyrenyloxy group and others.

The term "C₁-C₆₀ heteroaryloxy group" as used herein refers to an aryloxy group having a at least one heteroatom selected from N, O, Si, P, and S as a ring-forming atom, and having 1 to 60 carbon atoms. The examples thereof include a pyridinyloxy group, a furanoyloxy group, a thiophenoyloxy group, a siloxanoyloxy group, a phosphinoyloxy group, a pyrimidinyloxy group, a pyrazinyloxy group, a thiazolinyloxy group, an isoxazolinyloxy group, a benzoxazinoyloxy group, a benzothiazolinyloxy group, an imidazolinyloxy group, a pyridazinyloxy group, a silathiazolinyloxy group, an isothiazolinyloxy group, an oxazolinyloxy group, a silabenzoxazinoyloxy group, a phosphabenzothiazolinyloxy group, a dibenzofuranyloxy group, a silaisoxazolinyloxy group. and others.

The term "C₂-C₆₀ ether group" as used herein refers to a group containing an ether group - an oxygen atom connected to two selected from: alkyl, alkenyl, alkynyl, and aryl group, where the ether group having 2 to 60 carbon atoms. The examples thereof include: a methyl ether group, an ethyl ether group, an isopropyl ether group, a *tert*-butyl ether group, a phenyl ether group, an anisyl ether group, a benzyl ether group, an allyl ether group, a methoxyethyl ether group, an ethoxyethyl ether group, a methylphenyl ether group, an ethylphenyl ether group, an isobutyl ether group, a cyclohexyl ether group, a methoxymethyl ether group, an ethoxymethyl ether group, a propyl ether group, a butyl ether group, a pentyl ether group, a hexyl ether group, a methoxypropyl ether group, an ethoxypropyl ether group, a dimethyl ether group, a diethyl ether group, a dipropyl ether group, a dibutyl ether group, a dipentyl ether group, a methoxyisopropyl ether group, an ethoxyisopropyl ether group, a methoxy tert-butyl ether group, a methoxyphenyl ether group, an ethoxyphenyl ether group, a p-tolyl ether group, an o-anisyl ether group, a naphthyl ether group, a biphenyl ether group, a 2-methylphenyl ether group, a 4-ethylphenyl ether group, a 3-methoxyphenyl ether group, a 2,4-dimethylphenyl ether group, a 3,5-diethylphenyl ether group, a 2,4,6-trimethylphenyl ether group, a 2,3-dimethoxyphenyl ether group, a 2,4-diethylphenyl ether group, a 4-methoxynaphthyl ether group, a 3,4-diphenylnaphthyl ether group and others.

The term "C₆-C₆₀ thioaryl group" as used herein refers to a group derived from a thioether with R as an aryl group. The group has 6 to 60 carbon atoms. When the C₆-C₆₀ thioaryl group includes two or more rings, the rings may be fused to each other. The examples thereof include a thiophenyl group, a thionaphthyl group, a thioanthryl group, a thiophenanthryl group, a thiopyryl group, a thiobenzofuranyl group, a thioindolyl group, a thioquinolyl group, a thiopyridazinyl group, a thionaphthoquinolyl group, a thiopyrimidinyl group, a thiochromenyl group, a thioacridinyl group, a thiodibenzothiophenyl group, a thiotriphenylmethyl group and others.

The term "C₁-C₆₀ heterothioaryl" as used herein refers to a thioaryl group having 1 to 60 carbon atoms, where two or more rings fused to each other, at least one heteroatom selected from N, O, Si, P, and S, other than carbon atoms, as a ring-forming atom. Examples of the thereof include a pyrimidothioaryl group, a thieno[2,3-b] [1,4]dioxinyl group, a pyrazinothioaryl group, a benzothiazolyl group, a dibenzothiophenyl group, a thiopheno[3,4-b]pyrrolyl group, an isothiazolylthioaryl group, a naphthothiophenyl group, a thianthrenyl group, an indolizinothioaryl group, a dithiolo[3,4-b]pyrrolyl group, a selenopheno[2,3-b]pyridinyl group, a pyrrolo[2,3-b]thiophenyl group, an oxazolo[2,3-b]pyridinylthioaryl group, a pyrrolizinothioaryl group and others.

The term "C₁-C₆₀ amino group" as used herein refers to an organic compound comprises -N(R)₂, where R is, together or independently, selected from: hydrogen, deuterium, alkyl, alkenyl, alkynyl, aryl group, and heteroarylo group. The amino group has 1 to 60 carbon atoms. The term "C₁-C₆₀ alkylamino" as used herein refers to an amino group wherein R is an alkyl group. The term "C₁-C₆₀ dialkylamino" as used herein refers to an amino group wherein both R are alkyl groups. The term "C₆-C₆₀ arylamino group" as used herein refers to an amino group wherein R is an aryl group. The term "C₆-C₆₀ diarylamino group" as used herein refers to an amino group wherein both R are aryl groups. The term "C₆-C₆₀ heteroarylamino group" as used herein refers to an amino group wherein R is a heteroaryl group. The term "C₆-C₆₀ heterodiarylamino group" as used herein refers to an amino group wherein both R are heteroaryl groups. The examples of the amino group includes a methylamino group, a phenylamino group, a diphenylamino group, a naphthylamino group, a 9-methyl-anthracenylamino group, an ammonium group, a dimethylamino group, a diethylamino group, a methylanilino group, an ethylanilino group, an ethylphenylamino group, a diethylphenylamino group, a methoxymethylamino group, a methylbenzylamino group, an anilino group, a phenanthrylamino group, an ethoxyethylamino group, a pyridinylamino group, a quinolinylamino group, an isoquinolinylamino group, an imidazolylamino group, a benzimidazolylamino group, a triazolylamino group, a pyrazinylamino group, a pyrimidinylamino group, a thiazolylamino group, an oxazolylamino group, an isoxazolylamino group, an indolylamino group, a quinoxalinylamino group, a benzothiazolylamino group, a benzoxazolylamino group, a carbamoyl group, a thiocarbamoyl group, a sulfonylamino group, a sulfamoyl group, an amidoaryl group, a hydrazinyl group, a hydrazinoaryl group, a guanidinyl group, a guanidinoaryl group, an amidinyl group, an amidinoaryl group, a thiosemicarbazidyl group, a triazolethioamide group and others.

The term "a cyano group" as used herein refers to a -C≡N functional group.

The term "a nitro group" as used herein refers to a -NO₂ functional group.

The term "a halogen" as used herein refers to fluorine, chlorine, bromine, or iodine.

The term "C₄-C₆₀ non-aromatic fused polycyclic group" as used herein refers to a group, having 4 to 60 carbon atoms, where two or more rings fused with each other, only carbon atoms as ring-forming atoms, and no aromaticity in its entire molecular structure. The examples thereof include a fluorenyl group, a cyclobutylcyclopentyl group, a cyclopentylcyclohexyl group, a decalinyl group, a bicyclo[3.2.1]octanyl group, a dicyclo[4.4.0]decanyl group, a spiro[4.4]nonanyl group, a tetracyclo[5.3.0.0²,⁶.0³,⁵]decanyl group, a pentacyclo[6.4.0.0²,⁶.0³,¹⁰.0⁵,⁹]dodecanyl group, a tricyclo[3.2.1.0²,⁴]octanyl group, a bicyclo[4.3.1]decanyl group, a tetradecahydrophenanthrenyl group, a tricyclo[5.3.1.0²,⁶]dodecanyl group, a dicyclo[6.4.0]tetradecanyl group, a pentacyclo[7.3.1.0²,⁷.0³,⁶]tetradecanyl group, a tetracyclo[8.3.1.0²,⁸.0³,¹⁰.0⁶,⁹]hexadecanyl group, a hexacyclo[9.3.1.0²,⁹.0³,¹¹.0⁶,¹⁰]octadecanyl group and others.

The term "C₁-C₆₀ non-aromatic fused heteropolycyclic group" as used herein refers to a group, having 1 to 60 carbon atoms, where two or more rings fused to each other, at least one heteroatom selected from N, O, Si, P, and S, other than carbon atoms, as a ring-forming atom, and no aromaticity in its entire molecular structure. The examples thereof include an oxazolidinyl group, a silathiophenyl group, a pyrrolidinyl group, a phosphaseleninyl group, a dithiiranyl group, an oxathianyl group, a silaoxazinyl group, a thiaselenopyryl group, a phosphaoxazepinyl group, a pyrimidothiopyranyl group, a siladihydrothiazepinyl group, a pyrazinothiadiazinyl group, a phosphoazepinothiazinyl group, a triazolothiadiazinyl group, a sulfinylpyrazolopyridinyl group, an isoxazinothiopyranyl group, a thiadiazinylthiophenyl group, a dioxasilolanyl group, an oxatriazolothiopyridinyl group.

### DETAILED DESCRIPTION OF THE INVENTION

The present invention is illustrated by the following examples in detail.

### Example 1: A compound according to the invention

Examples of inventive compounds are included in the compounds described according to Formula I and they do not limit the scope of protection of this application. The compound according to Formula I may be selected from:

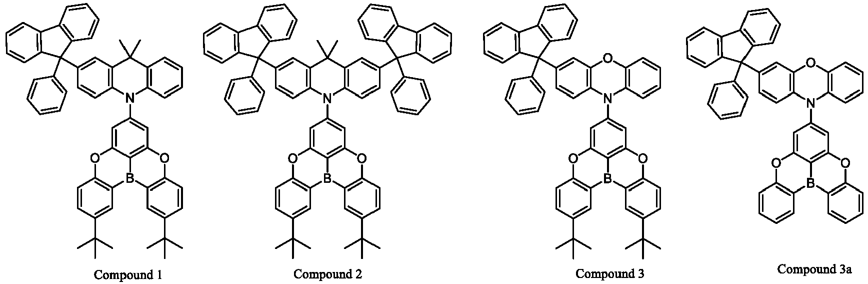

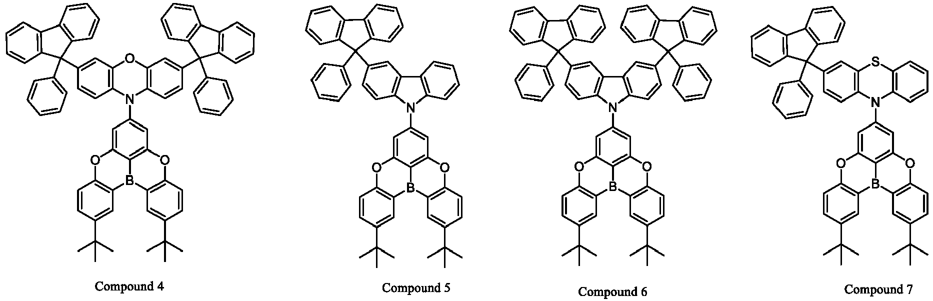

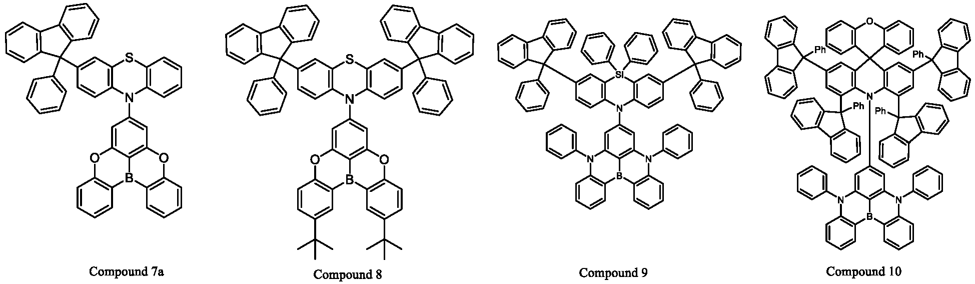

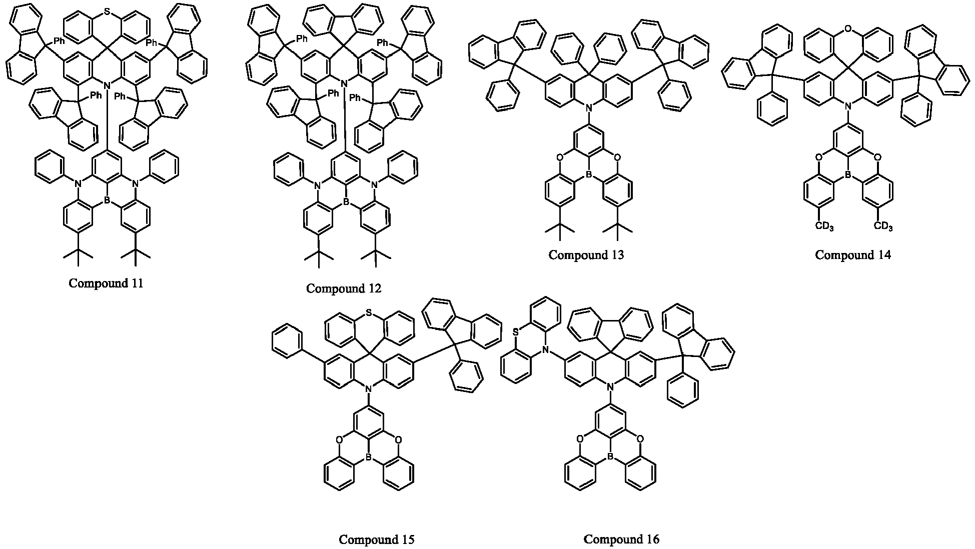

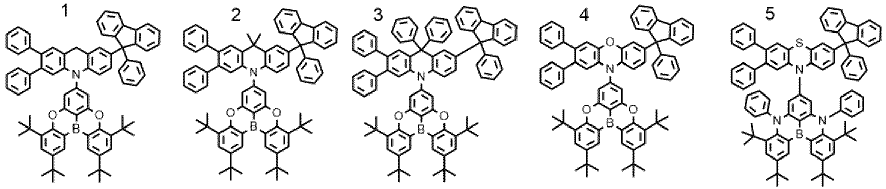

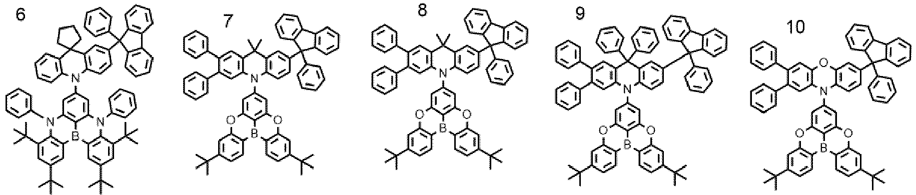

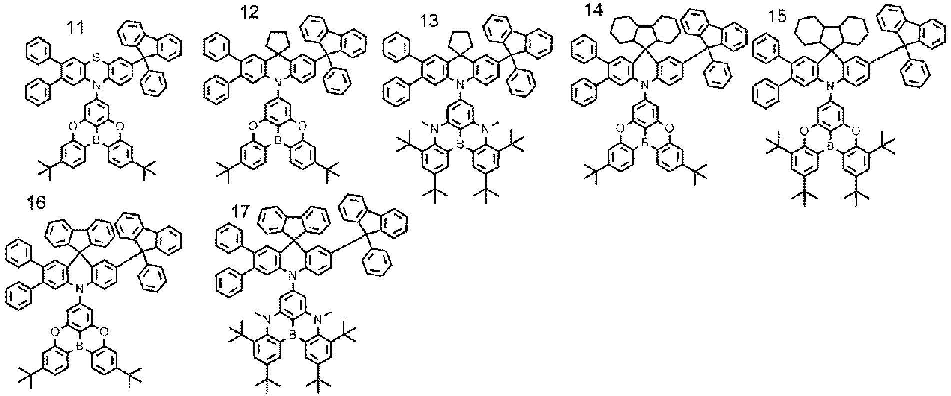

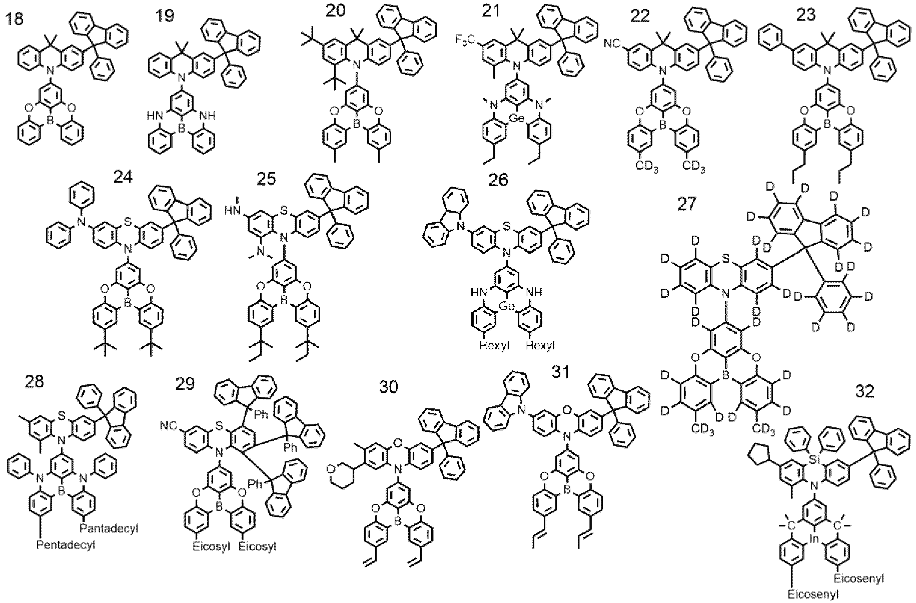

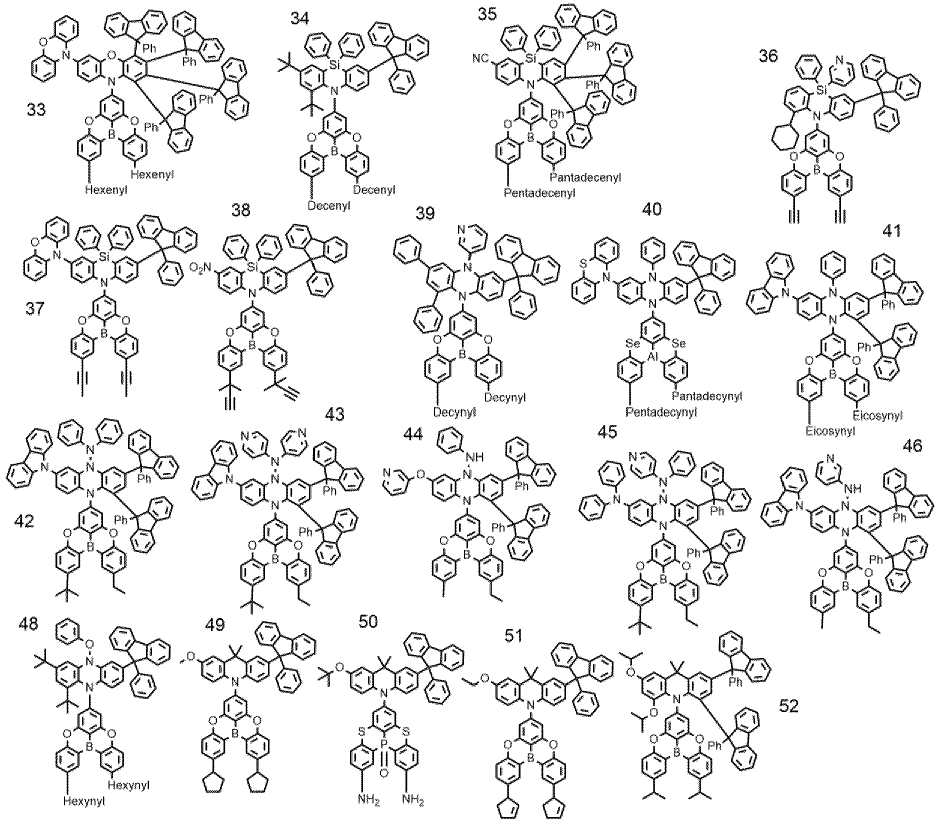

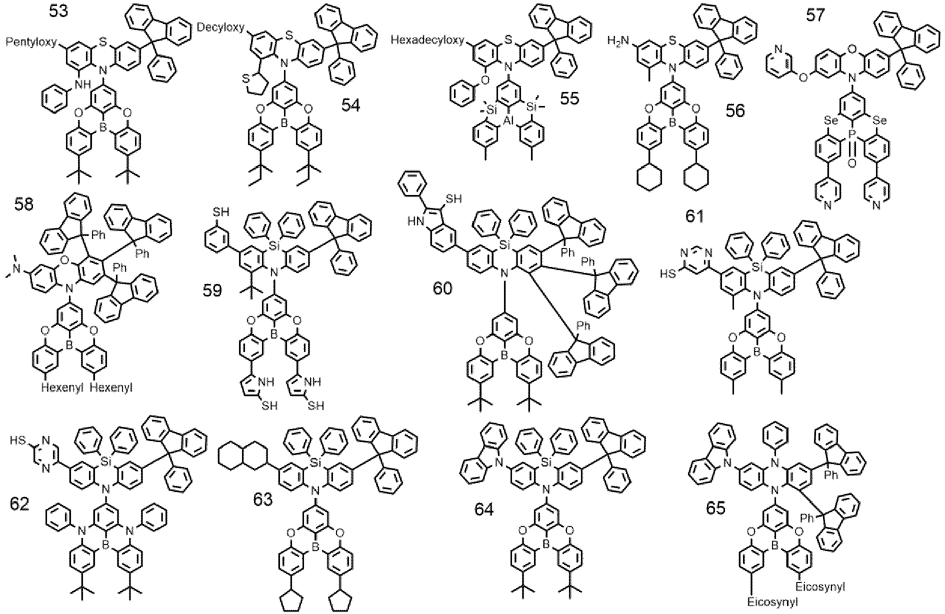

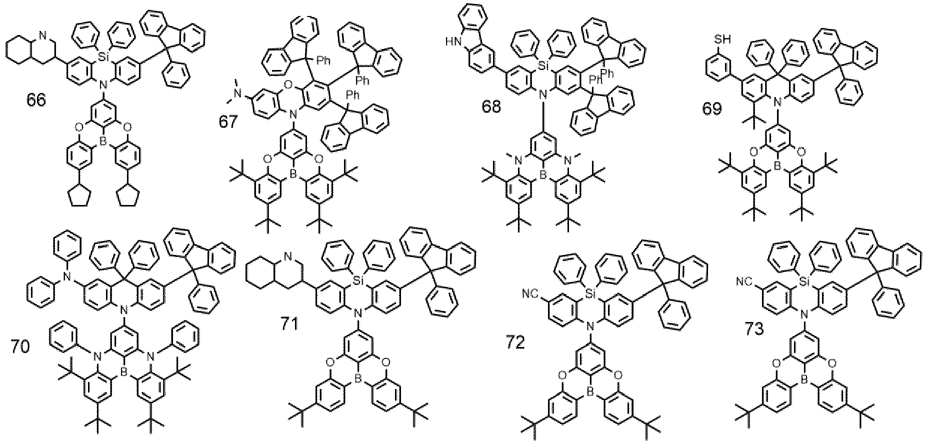

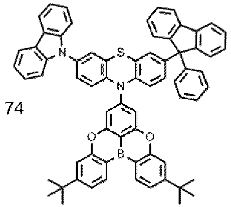

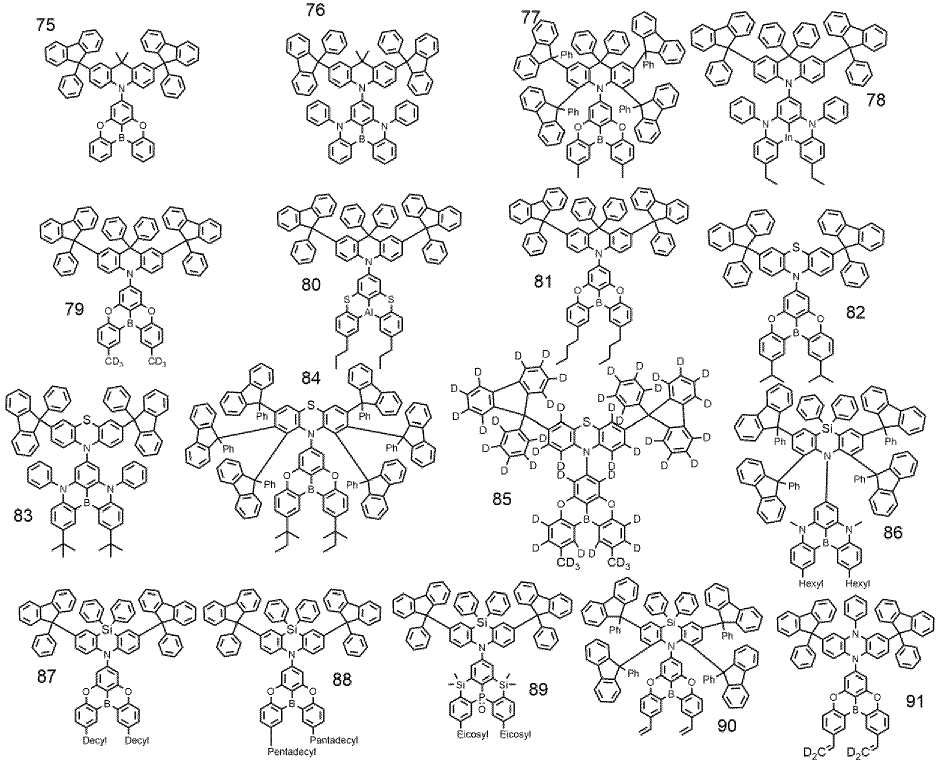

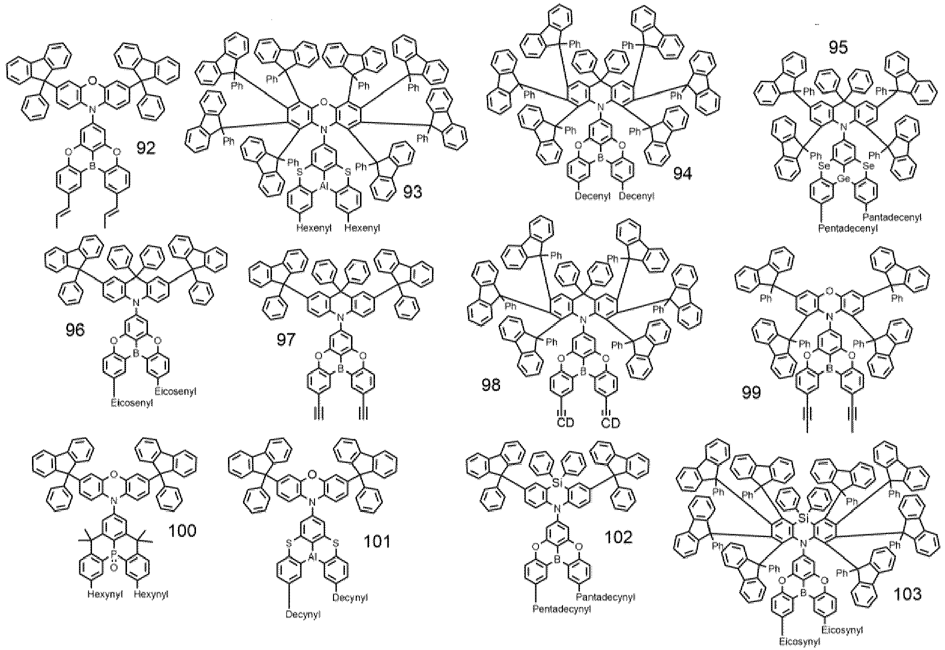

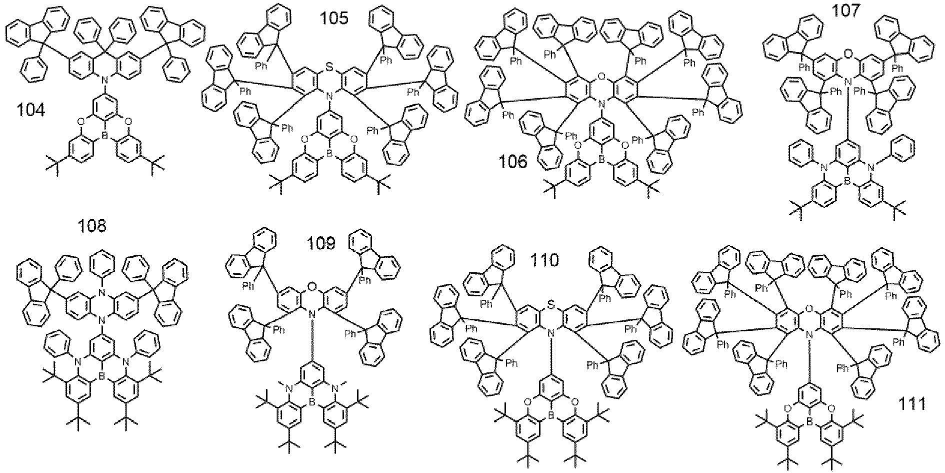

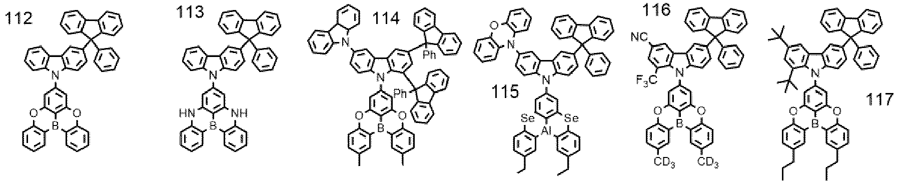

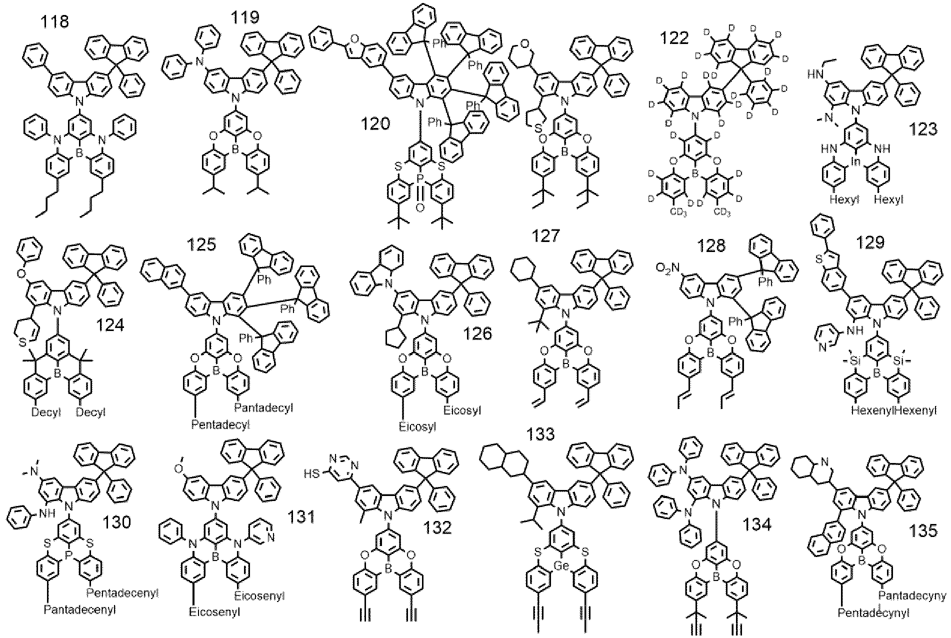

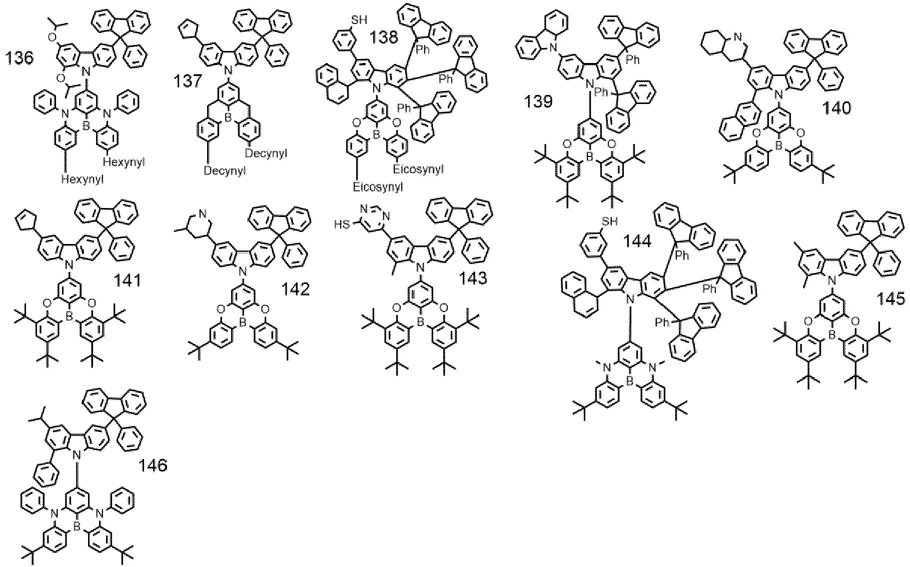

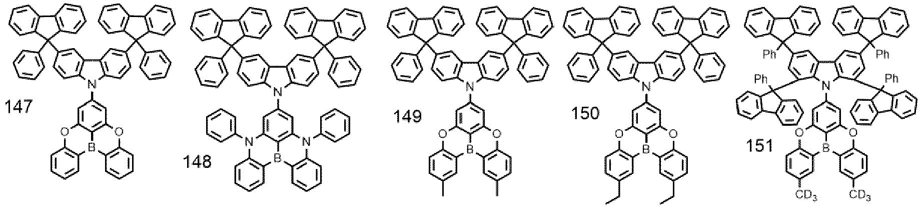

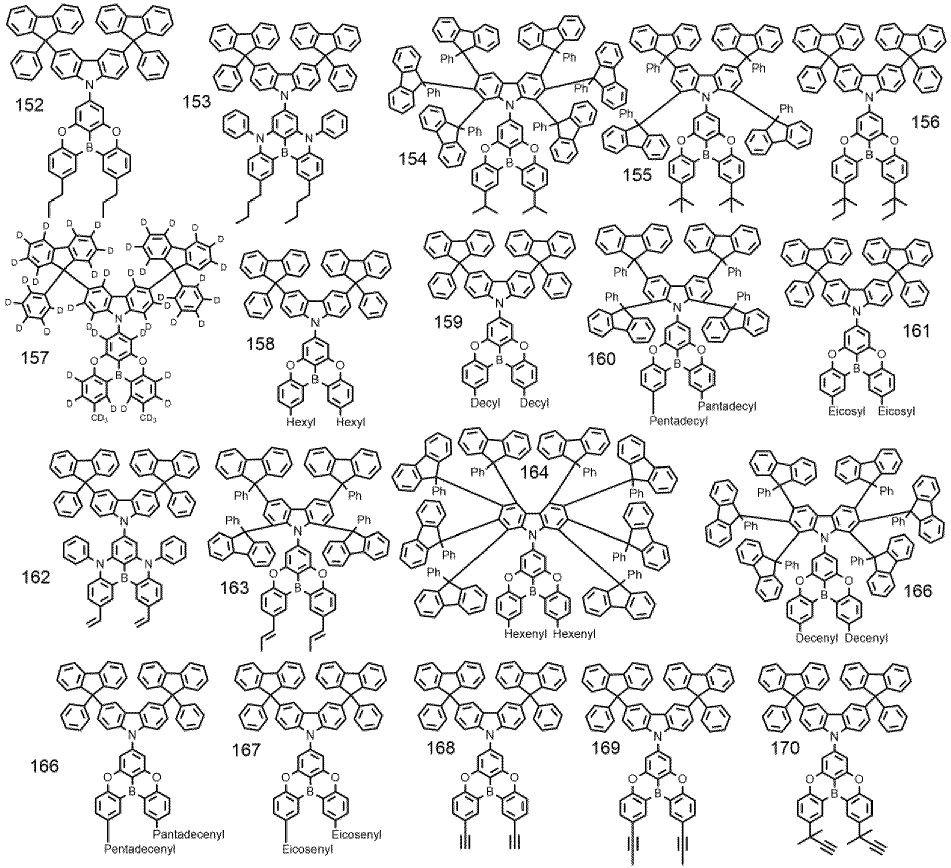

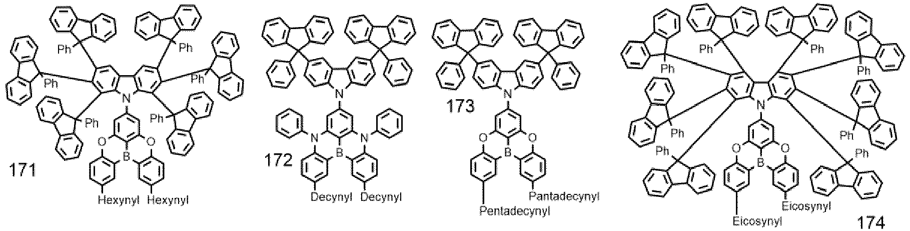

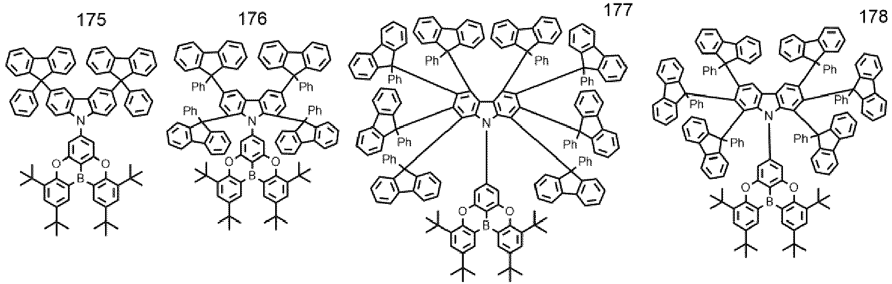

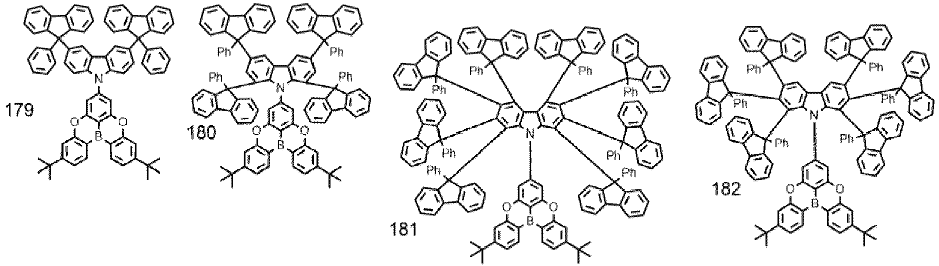

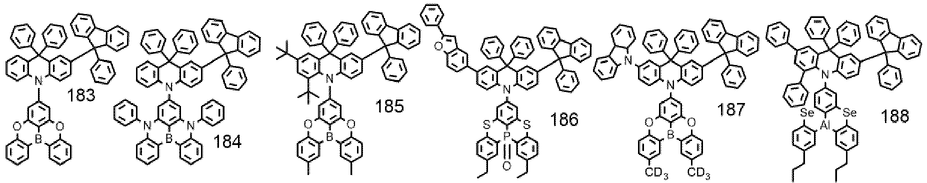

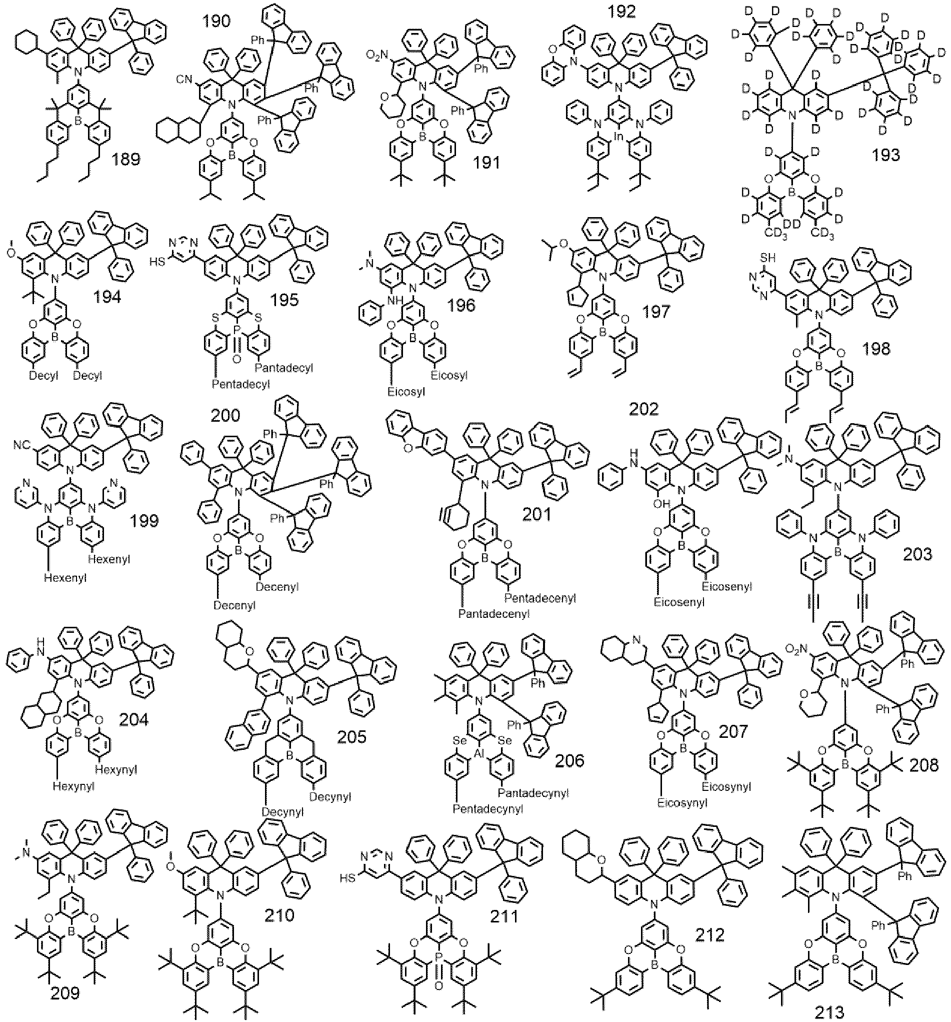

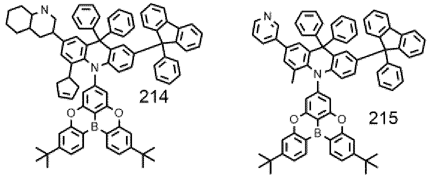

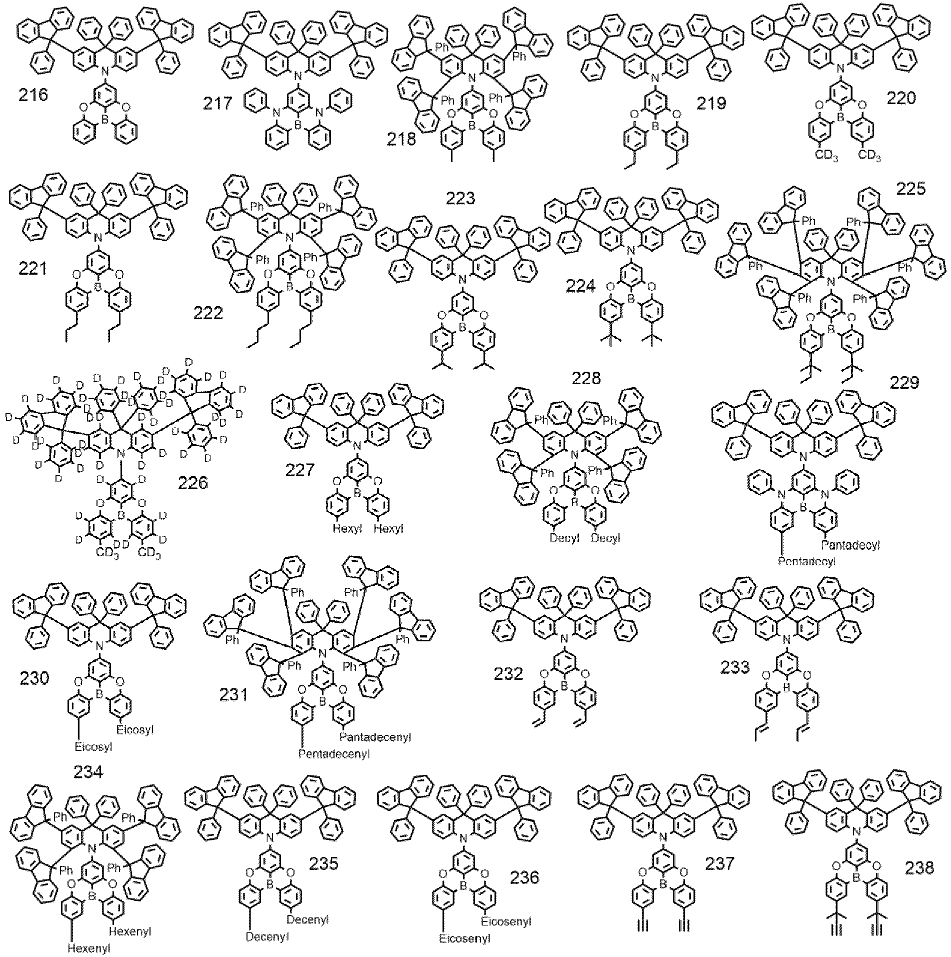

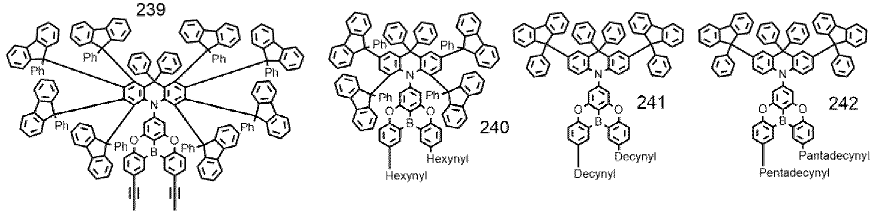

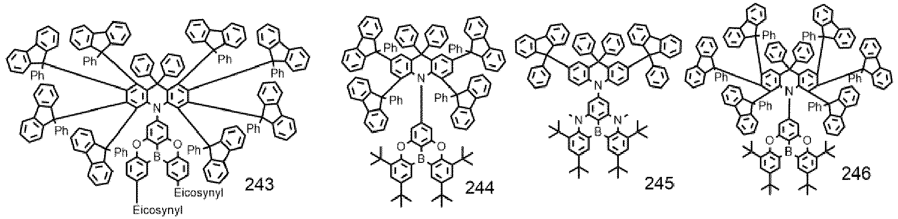

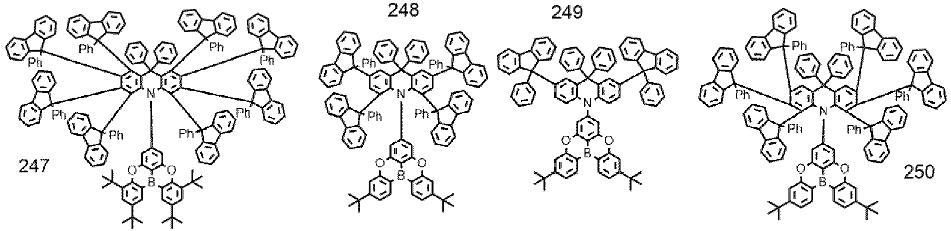

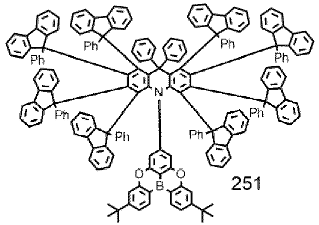

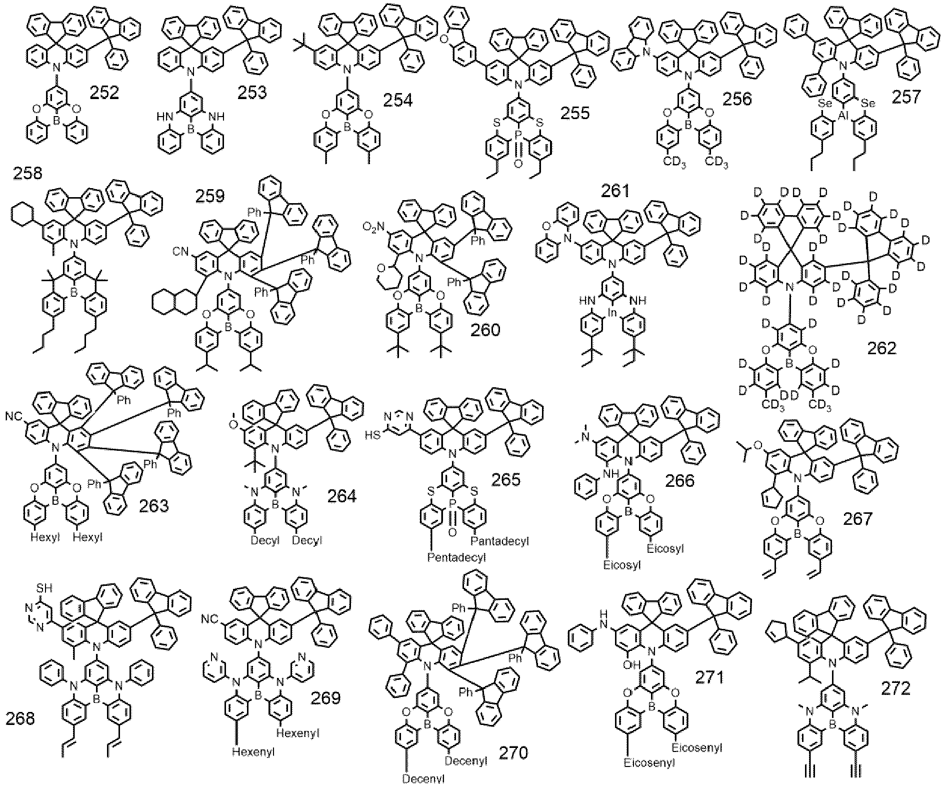

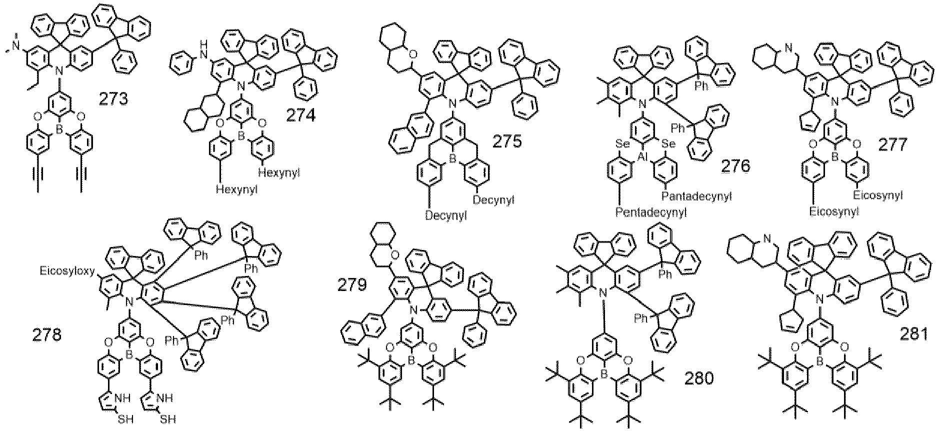

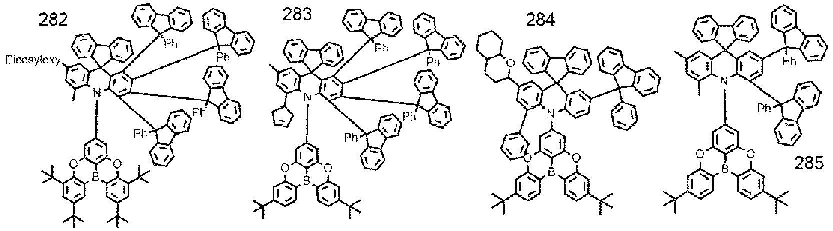

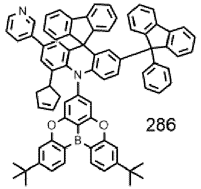

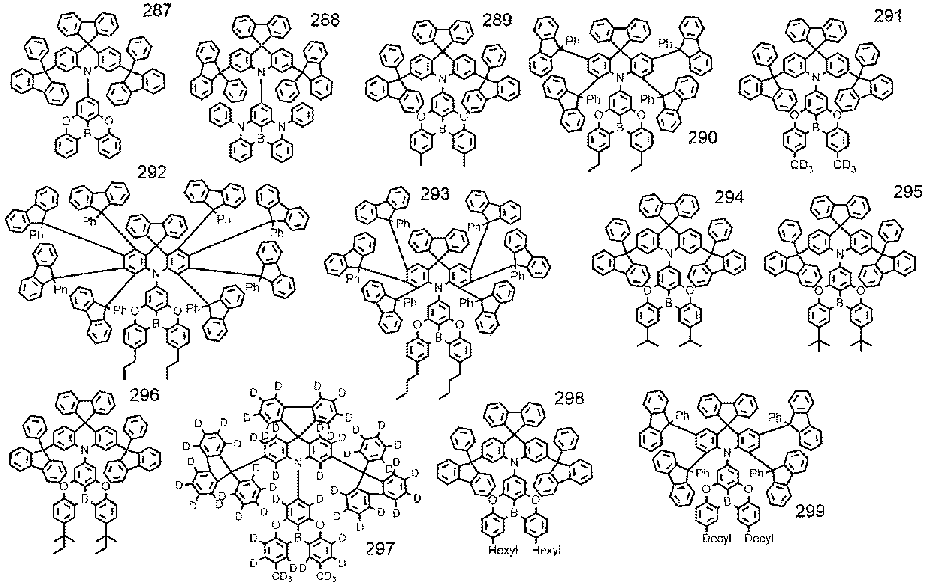

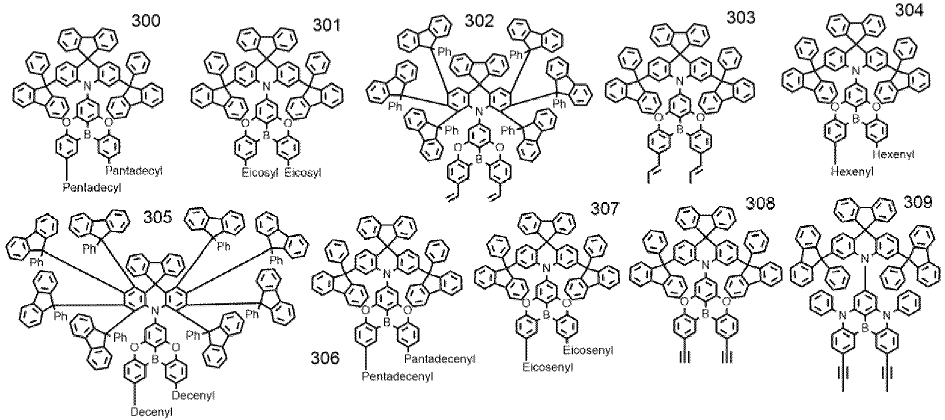

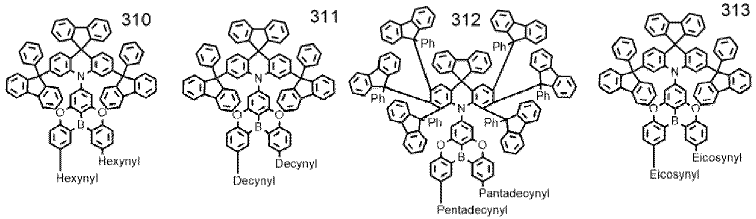

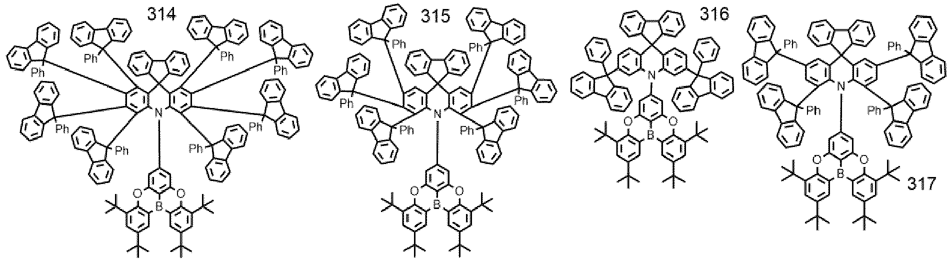

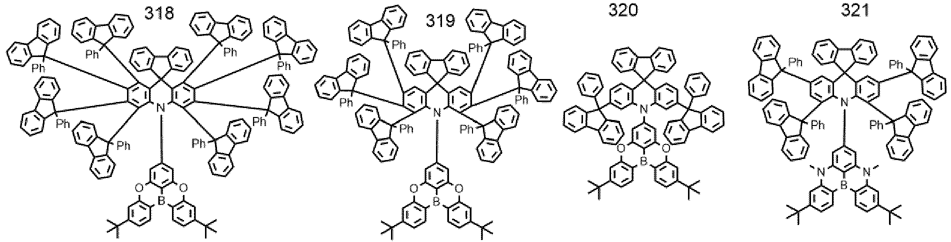

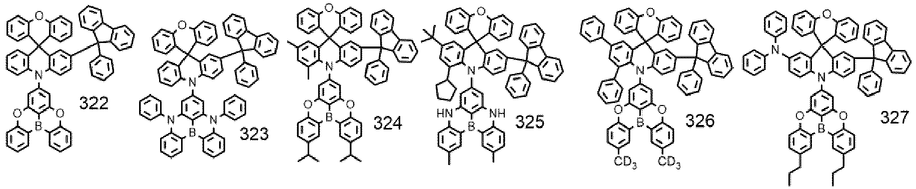

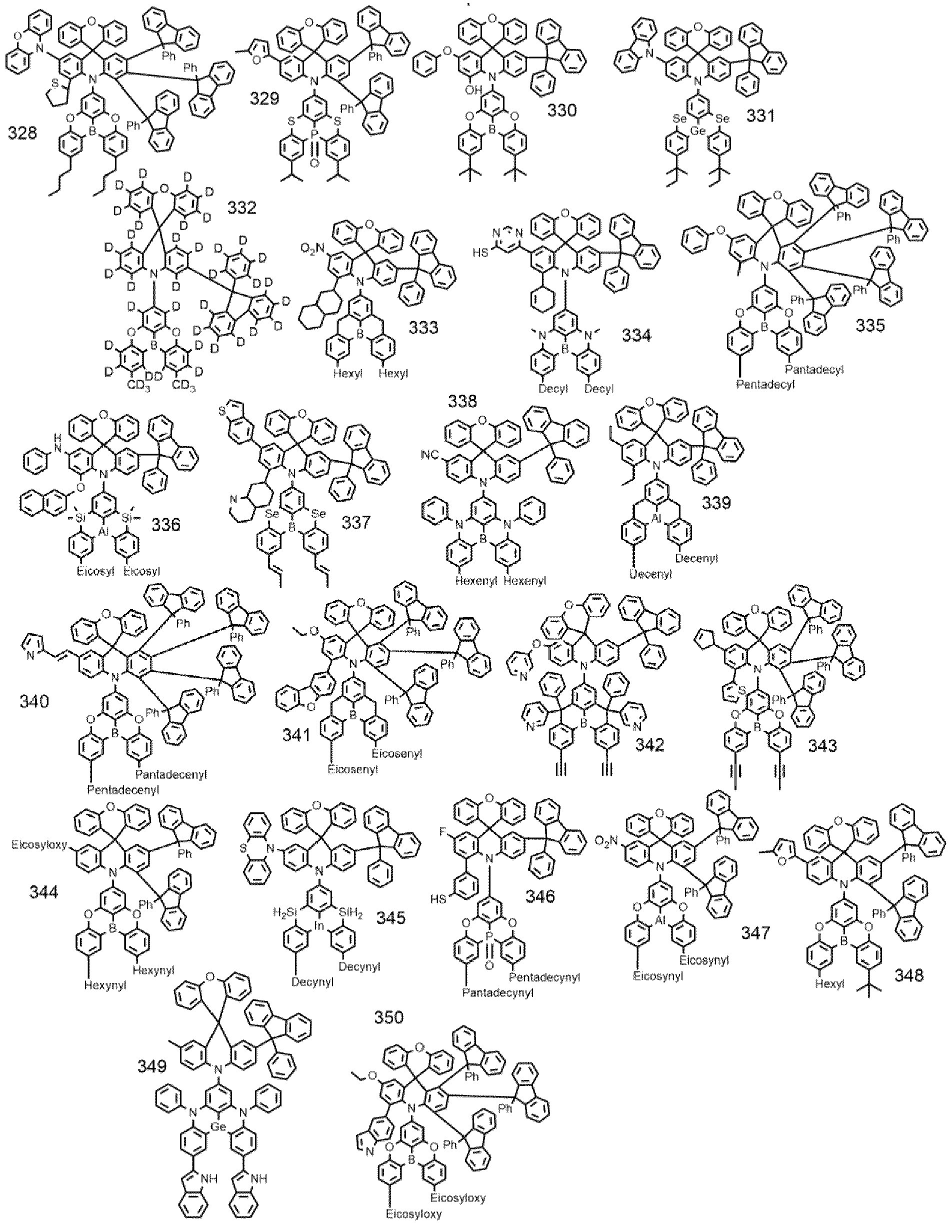

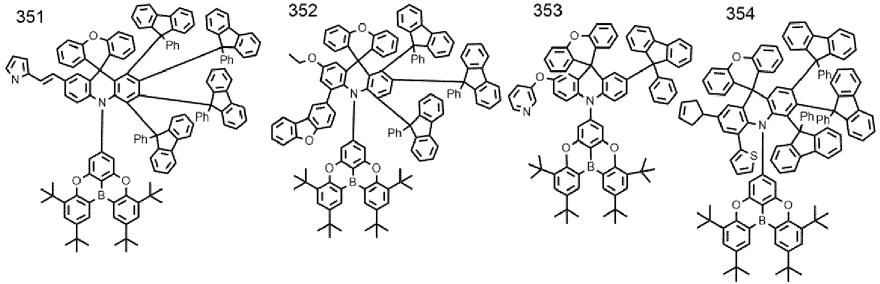

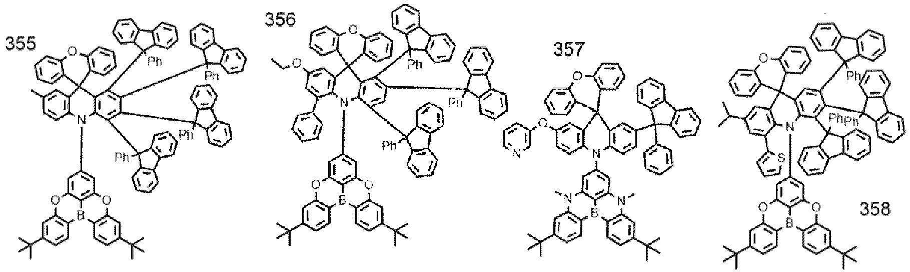

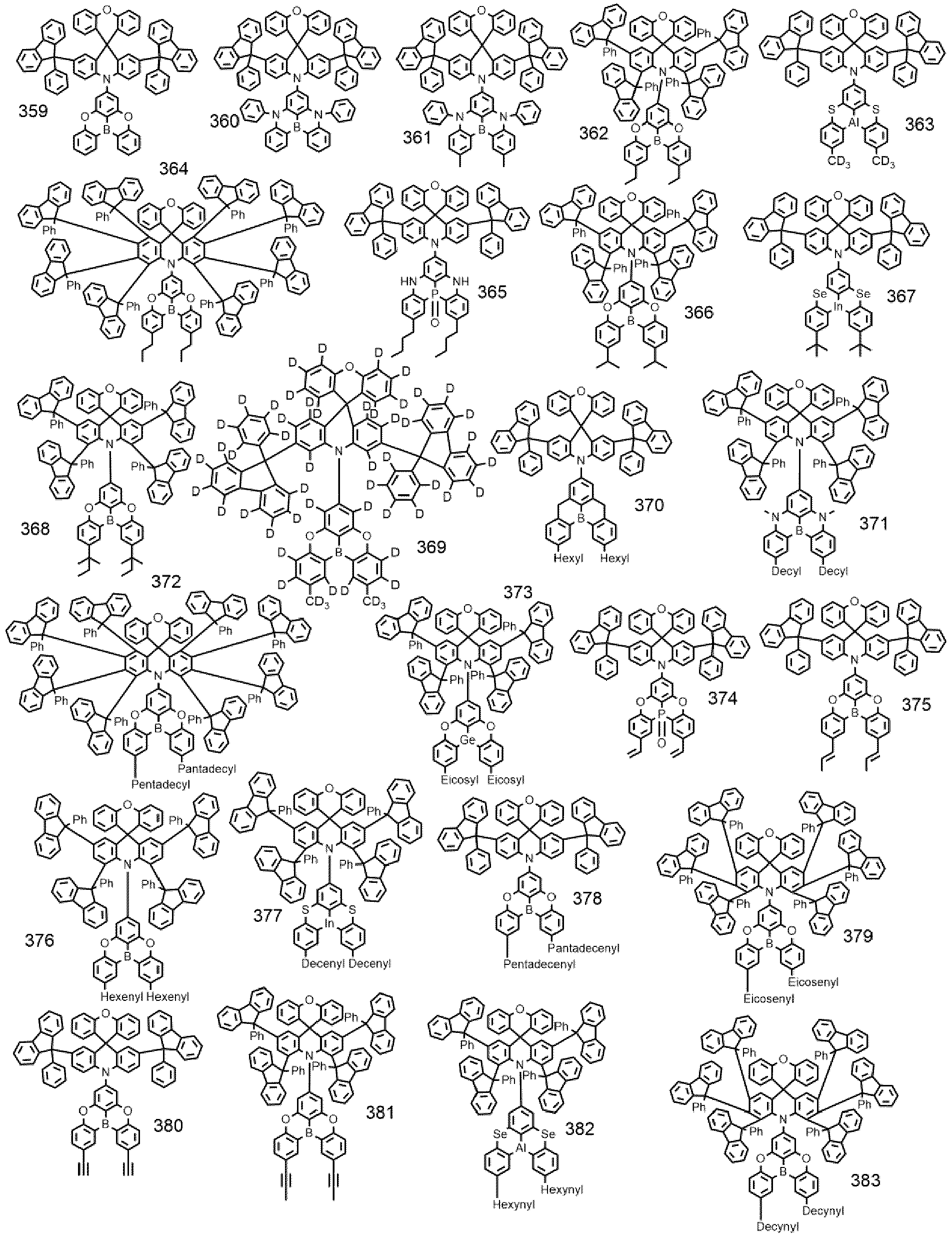

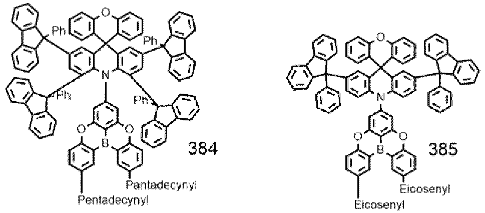

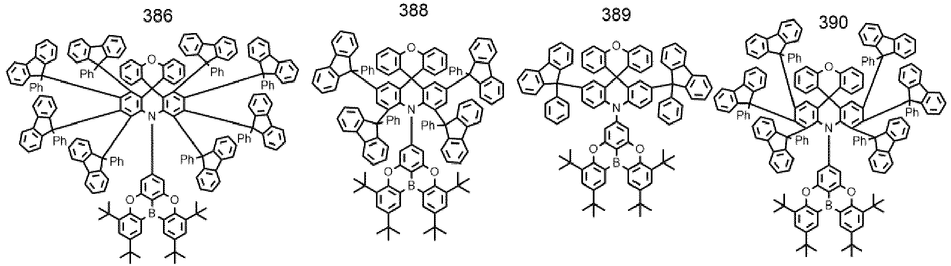

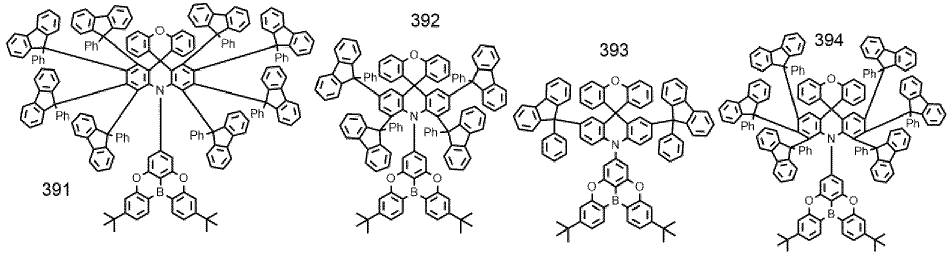

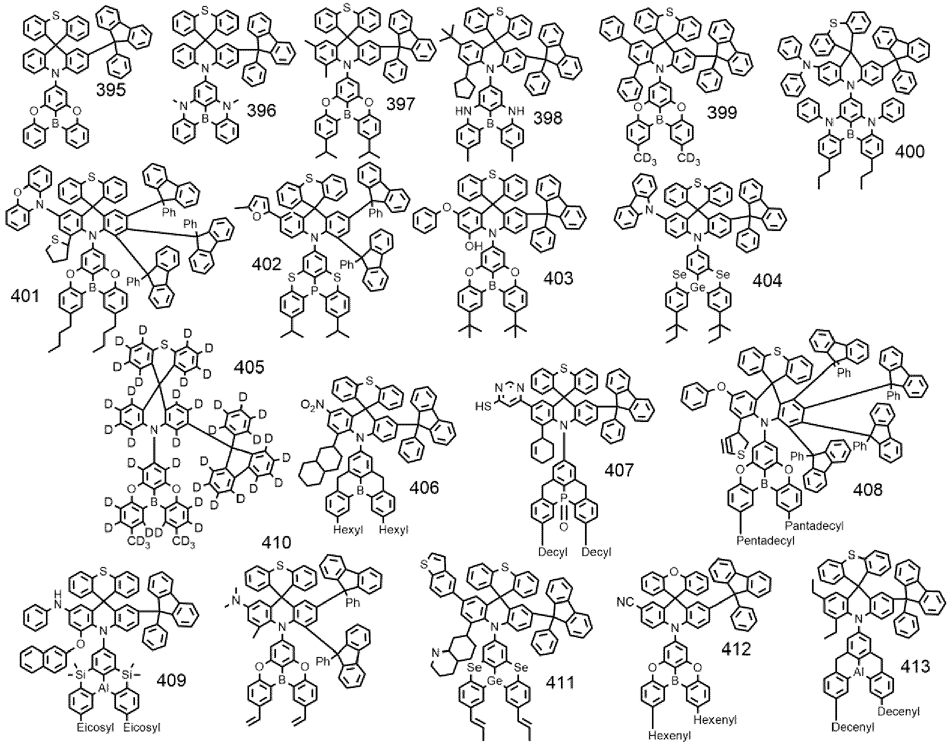

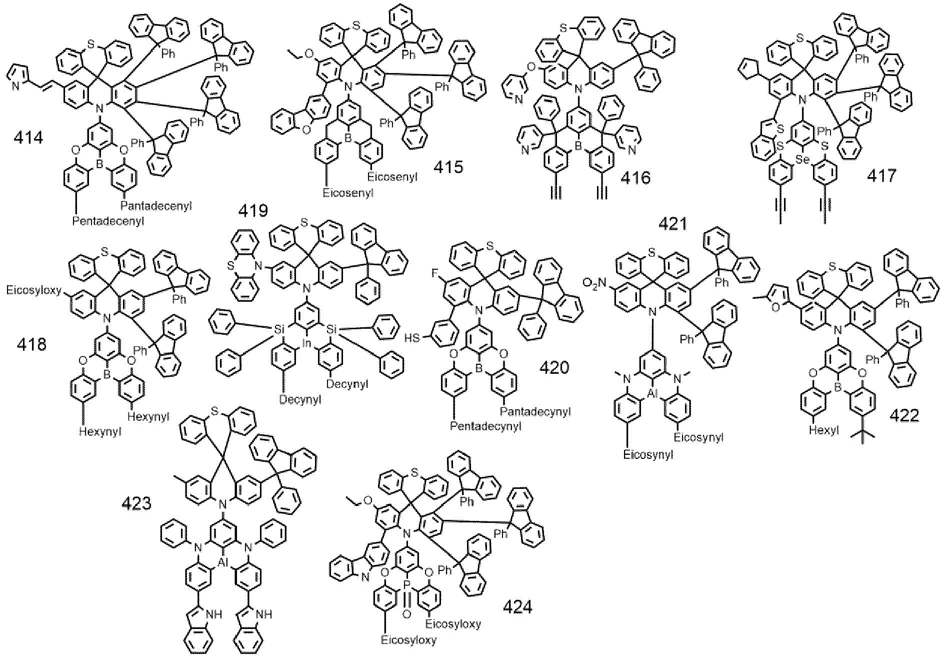

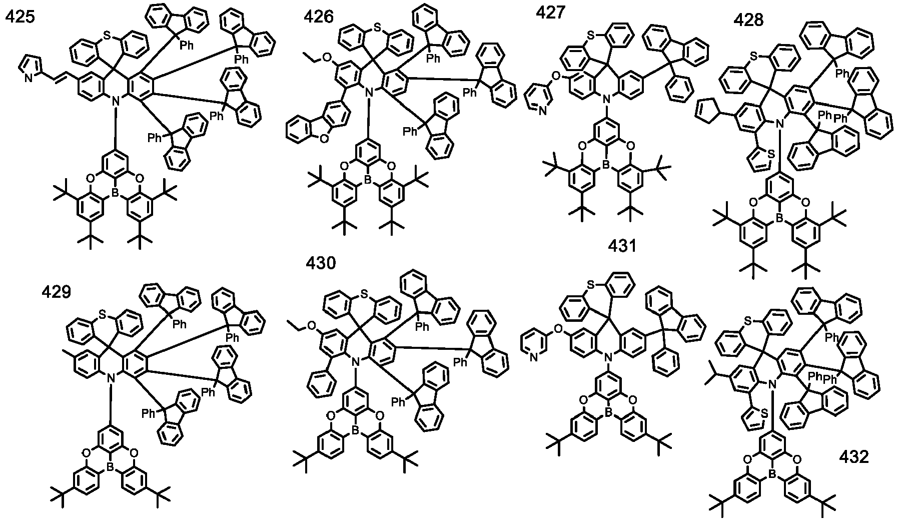

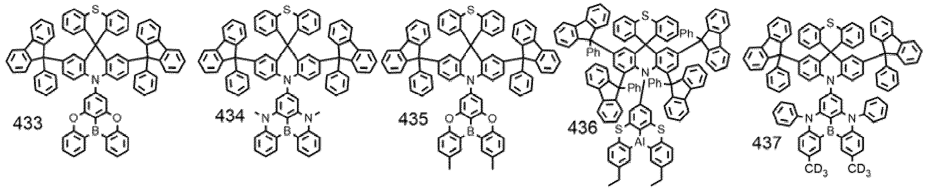

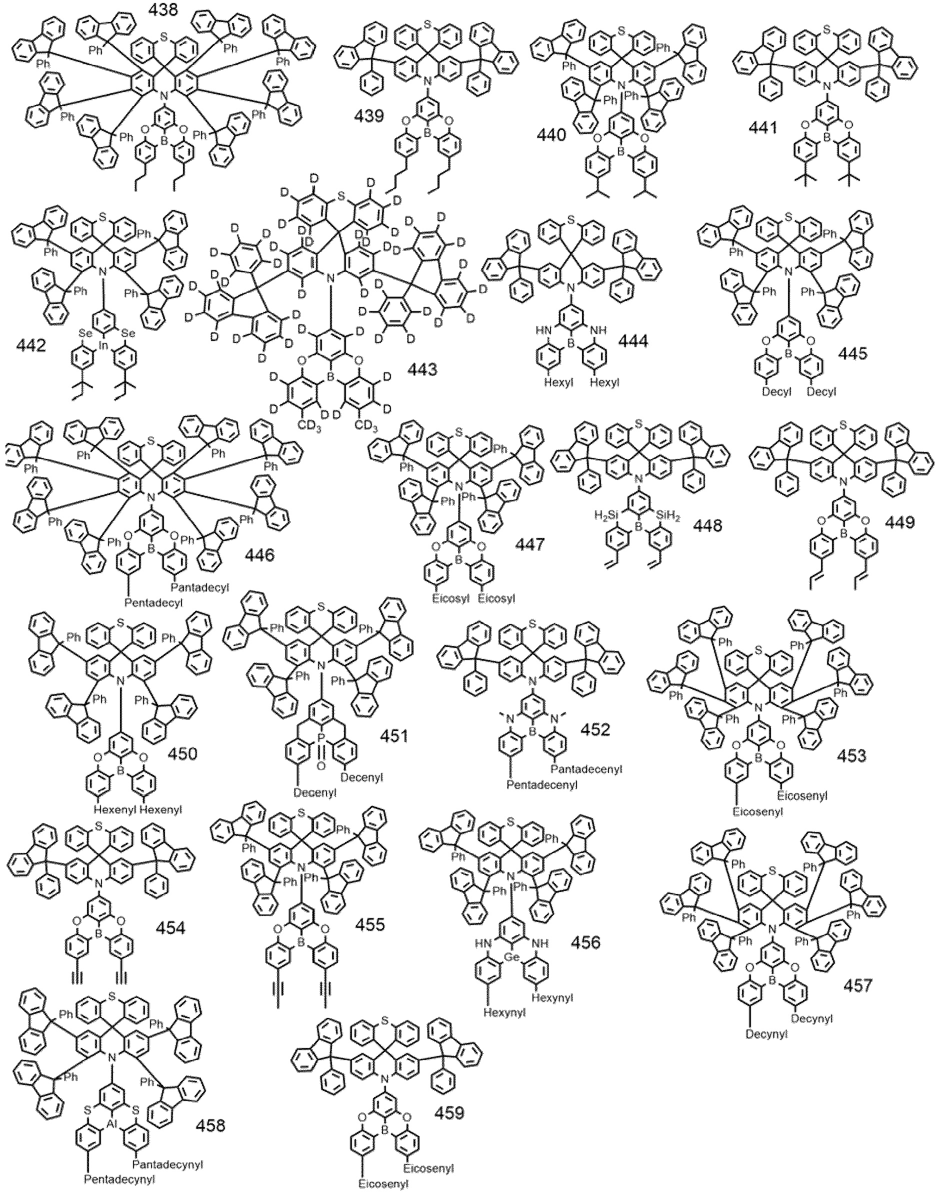

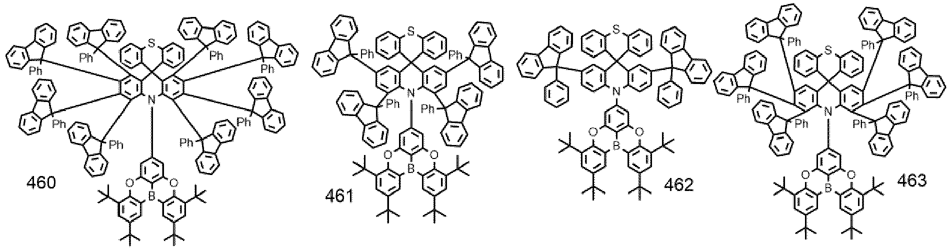

### Example 2: Process for obtaining compounds with the structure according to Formula I

The process of obtaining compounds with the structure represented by Formula I is presented in the following examples, which do not limit its scope of protection. The process of synthesizing the compounds of the embodiment will be exemplified through descriptions of processes for synthesizing **Compounds 1, 2, 3, 3a, 4, 5, 6, 7, 7a, 8, 9, 10, 11** and **12**.

### 1. Synthesis of 9,9-dimethyl-2-(9-phenyl-9H-fluoren-9-yl)-9,10-dihydroacridine (1.1A) and 9,9-dimethyl-2,7-bis(9-phenyl-9H-fluoren-9-yl)-9,10-dihydroacridine (1.1B)

To a stirred solution of 9,9-dimethyl-9,10-dihydroacridine (4.18 g; 20 mmol) in dry DCM (200 ml) BF₃·Et₂O (6.25 g; 5.43 ml; 44 mmol) was added dropwise at room temperature under inert atmosphere. The solution was stirred for 5 minutes. Then 9-phenyl-9*H*-fluoren-9-ol (5.16 g; 20 mmol) in dry DCM (100 ml) was added dropwise within 2 hours. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-ethyl acetate) to yield products: 2.99 g (white powder, 33.2%) of **1.1A** and 2.38 g (white powder, 17.2%) of **1.1B. 1.1A:** ¹H NMR (700 MHz, benzene-d₆): δ [ppm] 7.61 (d, *J* = 7.8 Hz, 2H), 7.52-7.51 (m, 3H), 7.38 (d, *J=* 7.8 Hz, 2H), 7.18-7.15 (m, 2H), 7.13 (d, *J* = 8.4 Hz, 1H), 7.10 (td, *J* = 7.6 Hz, 0.9 Hz, 2H), 7.07-7.03 (m, 3H), 7.02-6.99 (m, 2H), 6.82 (td, *J* = 7.6 Hz, 1.0 Hz, 1H), 6.24 (dd, *J* = 7.6 Hz, 0.9 Hz, 1H), 6.10 (d, *J* = 8.2 Hz, 1H), 5.32 (d, 1H), 1.36 (s, 6H). ¹³C NMR (176 MHz, benzene-d₆): δ [ppm] 152.21, 146.74, 140.29, 138.35, 137.60, 137.15, 128.88, 128.77, 128.26, 128.19, 127.99, 127.31, 126.52, 126.50, 126.22, 125.79, 125.69, 120.51, 120.18, 113.41, 113.29, 65.58, 36.11, 30.71. Anal. Calcd For C₃₄H₂₇N: C, 90.83; H, 6.05; N, 3.12. Found: C, 90.82; H, 6.05; N, 3.13. **1.1B**: ¹H NMR (700 MHz, benzene-d₆): δ [ppm] 7.59 (d, *J* = 7.8 Hz, 4H), 7.47 (d, *J* = 7.7 Hz, 4H), 7.43 (d, *J* = 2.0 Hz, 2H), 7.35-7.34 (m, 4H), 7.16-7.14 (m, 4H), 7.08 (td, *J* = 7.5, 1.1 Hz, 4H), 7.05 (dd, *J* = 8.2, 2.3 Hz, 2H), 7.03-6.97 (m, 6H), 6.09 (d, *J* = 8.4 Hz, 2H), 5.24 (s, 1H), 1.23 (s, 6H). ¹³C NMR (176 MHz, benzene-d₆): δ [ppm] 152.18, 146.68, 140.27, 137.61, 137.25, 128.23, 128.15, 127.98, 127.26, 126.53, 126.45, 126.20, 125.59, 120.14, 113.30, 65.53, 36.26, 30.22. Anal. Calcd For C₅₃H₃₉N: C, 92.27; H, 5.70; N, 2.03. Found: C, 92.20; H, 5.72; N, 2.04.

### 2. Synthesis of 10-(2,12-di-tert-butyl-5,9-dioxa-13b-boranaphtho[3,2,1-de]anthracen-7-yl)-9,9-dimethyl-2-(9-phenyl-9H-fluoren-9-yl)-9,10-dihydroacridine (Compound 1)

In a 10 ml vial, Pd₂dba₃ (0.046 g; 0.05 mmol; 5 mol%) and [*t*-Bu₃PH]BF₄ (0.029 g; 0.10 mmol; 10 mol%) were mixed in dry, degassed toluene (2 ml) and stirred for 10 minutes in an inert gas atmosphere. In a Schlenk flask, **1.1A** (0.540 g; 1.2 mmol) was dissolved in dry, degassed toluene (10 ml), t-BuONa (0.192 g; 2 mmol) was added, and the mixture was stirred for 5 minutes under argon. 7-bromo-2,12-di-tert-butyl-5,9-dioxa-13b-boranaphtho[3,2,1-*de*]anthracene (0.461 g; 1 mmol) was added, followed by the catalyst mixture, and the flask was immersed in an oil bath preheated to 100° C and stirred at this temperature for 1 hour. It was cooled to room temperature and then the mixture reaction was transferred to separatory funnel extracted three times with chloroform (3×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot mixture of acetone/methanol to yield 0.441 g od product (yellow powder, 53.13%).
¹H NMR (700 MHz, benzene-d₆): δ [ppm] 9.00 (d, *J* = 2.4 Hz, 2H), 7.62 (d, *J* = 2.2 Hz, 1H), 7.60 (d, *J* = 7.5 Hz, 2H), 7.56 (dd, *J* = 8.7, 2.4 Hz, 2H), 7.50 (d, *J* = 7.6 Hz, 2H), 7.47 (d, *J* = 8.6 Hz, 2H), 7.38-7.37 (m, 2H), 7.26 (dd, *J* = 7.5, 2.2 Hz, 1H), 7.17-7.14 (m, 4H), 7.10 (td, *J* = 7.4, 1.1 Hz, 2H), 7.04 (tt, *J* = 7.5, 1.1 Hz, 2H), 6.99 (tt, *J* = 7.3, 1.2 Hz, 1H), 6.88 (m, *J* = 7.5 Hz, 2H), 6.84 (dd, *J* = 8.9, 2.2 Hz, 1H), 6.58 (dd, *J* = 7.9, 1.7 Hz, 1H), 6.29 (d, *J* = 8.8 Hz, 1H), 1.52 (s, 6H), 1.40 (s, 18H). ¹³C NMR (176 MHz, benzene-d₆): δ [ppm] 159.46, 159.09, 152.13, 147.69, 146.52, 145.13, 140.56, 140.30, 139.34, 138.17, 131.72, 130.58, 130.41, 130.39, 128.28, 128.18, 127.98, 127.30, 126.63, 126.54, 126.47, 126.25, 125.41, 125.36, 121.11, 120.12, 118.21, 114.93, 114.85, 110.64, 65.46, 36.13, 34.26, 31.24. Anal. Calcd For C₆₀H₅₂BNO₂: C, 86.84; H, 6.32; B, 1.30; N, 1.69; O, 3.86. Found: C, 86.83; H, 6.35; N, 1.70.

### 3. Synthesis of 10-(2,12-di-tert-butyl-5,9-dioxa-13b-boranaphtho[3,2,1-de]anthracen-7-yl)-9,9-dimethyl-2,7-bis(9-phenyl-9H-fluoren-9-yl)-9,10-dihydroacridine (Compound 2)

In a 10 ml vial, Pd₂dba₃ (0.038 g; 0.04 mmol; 5 mol%) and [*t*-Bu₃PH]BF₄ (0.024 g; 0.08 mmol; 10 mol%) were mixed in dry, degassed toluene (2 ml) and stirred for 10 minutes in an inert gas atmosphere. In a Schlenk flask, **1.1B** (0.689 g; 1 mmol) was dissolved in dry, degassed toluene (10 ml), t-BuONa (0.159 g; 1.66 mmol) was added, and the mixture was stirred for 5 minutes under argon. 7-bromo-2,12-di-*tert*-butyl-5,9-dioxa-13b-boranaphtho[3,2,1-*de*]anthracene (0.384 g; 0.83 mmol) was added, followed by the catalyst mixture, and the flask was immersed in an oil bath preheated to 100° C and stirred at this temperature for 2 hours. It was cooled to room temperature and then the mixture reaction was transferred to separatory funnel extracted three times with chloroform (3×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot mixture of acetone/methanol to yield 0.420 g of product (yellow powder, 47.30%).
¹H NMR (700 MHz, benzene-d₆): δ [ppm] 9.00 (d, *J* = 2.5 Hz, 2H), 7.60-7.55 (m, 8H), 7.49 (d, *J* = 8.6 Hz, 2H), 7.46 (d, *J* = 7.7 Hz, 4H), 7.36-7.33 (m, 4H), 7.14 (td, *J* = 7.5, 1.0 Hz, 4H), 7.03-7.00 (m, 4H), 6.99-6.96 (m, 2H), 6.84 (dd, *J* = 8.9, 2.1 Hz, 2H), 6.28 (d, *J* = 8.7 Hz, 2H), 1.40 (s, 6H), 1.39 (s, 18H). ¹³C NMR (176 MHz, benzene-d₆): δ [ppm] 159.40, 159.10, 152.10, 147.46, 146.48, 145.16, 140.27, 139.31, 138.08, 131.79, 130.43, 130.04, 128.26, 128.14, 127.25, 126.68, 126.42, 125.21, 120.09, 118.19, 114.57, 110.90, 65.41, 36.25, 34.26, 31.23, 30.87. Anal. Calcd For C₇₉H₆₄BNO₂: C, 88.66; H, 6.03; B, 1.01; N, 1.31; O, 2.99. Found: C, 88.65; H, 6.08; N, 1.31.

### 4. Synthesis of 3-(9-phenyl-9H-fluoren-9-yl)-10H-phenoxazine (1.1C) and 3,7-bis(9-phenyl-9H-fluoren-9-yl)-1077-phenoxazine (1.1D)

To a stirred solution of 10*H*-phenoxazine (1.0 g; 5.5 mmol) in dry DCM (100 ml) BF₃·Et₂O (1.7 g; 1.5 ml; 12.0 mmol) was added dropwise at room temperature under inert atmosphere. The solution was stirred for 5 minutes. Then 9-phenyl-9*H*-fluoren-9-ol (1.4 g; 5.5 mmol) in dry DCM (100 ml) was added dropwise within 2 hours. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-ethyl acetate) to yield products: 0.405 g (pinkish powder, 18.2%) of **1.1C** and 0.840 g (pinkish powder, 23.6%) of **LID.**
**1.1C:** ¹H NMR (400 MHz, THF-d₈): δ [ppm] 7.82 (d, *J* = 6.9 Hz, 2H), 7.43 (d, *J* = 7.5 Hz, 2 H), 7.35 (td, *J* = 7.4 Hz, 1.4 Hz, 2H), 7.30-7.16 (m, 8H), 6.64 (td, *J* = 7.4 Hz, 1.9 Hz, 1H), 6.55-6.43 (m, 4H), 6.34 (dd, *J* = 7.8 Hz, 1.3 Hz, 1H), 6.22 (d, *J* = 8.2 Hz, 1H). ¹³C NMR (100 MHz, THF-d₈): δ [ppm] 151.30, 145.91, 143.51, 143.30, 140.14, 138.23, 130.49, 128.05, 127.97, 127.85, 127.31, 127.14, 126.25, 125.97, 123.28, 122.92, 120.14, 119.85, 115.22, 114.93, 112.87, 112.30, 64.75. Anal. Calcd For C₃₁H₂₁NO: C, 87.92; H, 5.00; N, 3.31; O, 3.78. Found: C, 87.73, H, 5.17, N, 3.28.
**1.1D:** ¹H NMR (400 MHz, THF-d₈): δ [ppm] 7.79 (d, *J* = 7.3 Hz, 4H), 7.39 (d, *J* = 7.6 Hz, 4H), 7.33 (td, *J* = 7.6 Hz, 1.2 Hz, 4H), 7.27-7.14 (m, 15H), 6.47 (dd, *J* = 7.9 Hz, 2.3 Hz, 2H), 6.35 (d, *J* = 2.3 Hz, 2H), 6.19 (d, *J* = 8.2 Hz, 2H). ¹³C NMR (100 MHz, THF-d₈): δ [ppm] 151.26, 145.82, 143.07, 140.12, 138.46, 138.24, 131.14, 127.95, 127.81, 127.26, 127.10, 126.21, 125.94, 122.83, 119.81, 115.26, 112.25, 64.68. Anal. Calcd For C₅₀H₃₃NO: C, 90.47; H, 5.01; N, 2.11; O, 2.41. Found: C, 85.12, H, 5.89, N, 1.61.

### 5. Synthesis of 10-(2,12-di-tert-butyl-5,9-dioxa-13b-boranaphtho[3,2,1-de]anthracen-7-yl)-3-(9-phenyl-9H-fluoren-9-yl)-10H-phenoxazine (Compound 3)

In a 10 ml vial, Pd₂dba₃ (0.037 g; 0.04 mmol; 5 mol%) and [*t*-Bu₃PH]BF₄ (0.023 g; 0.08 mmol; 10 mol%) were mixed in dry, degassed toluene (2 ml) and stirred for 10 minutes in an inert gas atmosphere. In a Schlenk flask, **1.1C** (0.405 g; 0.96 mmol) was dissolved in dry, degassed toluene (10 ml), t-BuONa (0.184 g; 1.92 mmol) was added, and the mixture was stirred for 5 minutes under argon. 7-bromo-2,12-di-tert-butyl-5,9-dioxa-13b-boranaphtho[3,2,1-de]anthracene (0.367 g; 0.80 mmol) was added, followed by the catalyst mixture, and the flask was immersed in an oil bath preheated to 100° C and stirred at this temperature for 1 hour. It was cooled to room temperature and then the mixture reaction was transferred to separatory funnel extracted three times with chloroform (3 ×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot mixture of acetone/methanol to yield 0.406 g of product (yellowish powder, 62.0%).
**Compound 3:** ¹H NMR (400 MHz, THF-d₈): δ [ppm] 8.85 (d, *J* = 2.5 Hz, 2H), 7.91 (dd, *J* = 9.0 Hz, 2.6 Hz, 2H), 7.82 (d, *J* = 7.5 Hz, 2H), 7.56 (d, *J* = 8.7 Hz, 2H), 7.43 (d, *J* = 7.7 Hz, 2H), 7.35 (td, *J* = 7.5 Hz, 1.0 Hz, 2H), 7.30-7.14 (m, 9H), 6.67-6.56 (m, 4H), 6.49 (dd, *J* = 8.5 Hz, 2.1 Hz, 1H), 6.15 (d, *J* = 8.3 Hz, 1H), 6.04 (d, *J* = 8.4 Hz, 1H), 1.54 (s, 18H). ¹³C NMR (100 MHz, THF-d₈): δ [ppm] 159.43, 158.79, 151.10, 145.61, 145.39, 145.32, 143.96, 143.62, 140.15, 139.78, 133.66, 132.46, 131.92, 130.03, 127.94, 127.90, 127.36, 127.24, 126.33, 125.96, 123.15, 122.92, 121.54, 119.90, 117.84, 115.31, 115.22, 113.65, 113.28, 110.12, 64.66, 34.31, 30.85, 29.36. Anal. Calcd For C₅₇H₄₆BNO₃: C, 85.17; H, 5.77; B, 1.34; N, 1.74; O, 5.97. Found C, 87.31, H, 5,50, N, 2,23.

### 6. Synthesis of 10-(5,9-dioxa-13b-boranaphtho[3,2,1-de]anthracen-7-yl)-3-(9-phenyl-9H-fluoren-9-yl)-10H-phenoxazine (Compound 3a)

Synthesis of **Compound 3a** was obtained according to the method of synthesis **Compound 3** (disclosed in Point 5 above), except that instead of 7-bromo-2,12-di-*tert*-butyl-5,9-dioxa-13b-boranaphtho[3,2,1-*de*]anthracene, 7-bromo-5,9-dioxa-13b-boranaphtho[3,2,1-*de*]anthracene was used.

### 7. Compound 3a: Recorded ¹H NMR spectra confirmed the presence of the desired reaction product.

### Synthesis of 10-(2,12-di-tert-butyl-5,9-dioxa-13b-boranaphtho[3,2,1-de]anthracen-7-yl)-3,7-bis(9-phenyl-9H-fluoren-9-yl)-10H-phenoxazine (Compound 4)

In a 10 ml vial, Pd₂dba₃ (0.046 g; 0.05 mmol; 5 mol%) and [*t*-Bu₃PH]BF₄ (0.029 g; 0.10 mmol; 10 mol%) were mixed in dry, degassed toluene (2 ml) and stirred for 10 minutes in an inert gas atmosphere. In a Schlenk flask, **1.1D** (0.797 g; 1.2 mmol) was dissolved in dry, degassed toluene (10 ml), *t*-BuONa (0.192 g; 2 mmol) was added, and the mixture was stirred for 5 minutes under argon. 7-bromo-2,12-di-*tert*-butyl-5,9-dioxa-13b-boranaphtho[3,2,1-*de*]anthracene (0.461 g; 1 mmol) was added, followed by the catalyst mixture, and the flask was immersed in an oil bath preheated to 100° C and stirred at this temperature for 1 hour. It was cooled to room temperature and then the mixture reaction was transferred to separatory funnel extracted three times with chloroform (3 ×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot mixture of acetone/methanol to yield 0.392 g od product (yellowish powder, 37.0%).
**Compound 4**: ¹H NMR (400 MHz, benzene-d₆): δ [ppm] 8.98 (d, *J* = 2.4 Hz, 2H), 7.59-7.54 (m, 6H), 7.49 (d, *J* = 9.0 Hz, 2H), 7.40 (d, *J* = 7.7 Hz, 4H), 7.36-7.32 (m, 4H), 7.16-7.12 (m, 4H), 7.09-6.94 (m, 12H), 6.85 (d, *J* = 2.2 Hz, 2H), 6.38 (dd, *J* = 8.5 Hz, 2.2 Hz, 2H), 5.76 (d, *J* = 8.5 Hz, 2H), 1.38 (s, 18H). ¹³C NMR (100 MHz, THF-d₈): δ [ppm] 159.35, 158.77, 151.05, 145.53, 145.36, 143.43, 140.12, 139.76, 132.29, 131.88, 130.02, 127.92, 127.86, 127.75, 127.52, 127.33, 127.21, 126.29, 125.93, 122.79, 119.86, 115.33, 113.10, 110.05, 64.61, 34.29, 30.84, 29.63. Anal. Calcd For C₇₆H₅₈BNO₃: C, 87.43; H, 5.60; B, 1.04; N, 1.34; O, 4.60. Found: C, 84.01, H, 5.42, N, 1.15.

### 8. Synthesis of 3-(9-phenyl-9H-fluoren-9-yl)-9H-carbazole (1.1E) and 3,6-bis(9-phenyl-9H-fluoren-9-yl)-9H-carbazole (1.1F)

To a stirred solution of 9*H*-carbazole (0.836 g; 5 mmol) in dry DCM (50 ml) BF₃·Et₂O (0.36 g; 0.31 ml; 2.5 mmol) was added dropwise at room temperature under inert atmosphere. The solution was stirred for 5 minutes. Then 9-phenyl-9*H*-fluoren-9-ol (1.29 g; 5 mmol) in dry DCM (50 ml) was added dropwise within 2 hours. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-ethyl acetate) to yield products: 0.98 g (white powder, 48.1%) of **1.1E** and 0.65 g (white powder, 18.5%) of **1.1F.**
**1.1E:** ¹H NMR (400 MHz, THF-d₈): δ [ppm] 10.23 (s, 1H), 7.91-7.83 (m, 4H), 7.52 (d, *J=* 7.7 Hz, 2H), 7.40-7.43 (m, 3H), 7.32-7.18 (m, 10H), 7.05 (td, *J* = 7.4 Hz, 0.9 Hz, 1H). ¹³C NMR (100 MHz, benzene-d₆): δ [ppm] 152.42, 147.03, 140.32, 139.81, 138.38, 137.06, 126.51, 125.45, 123.40, 123.37, 120.51, 120.16, 119.56, 119.22, 110.36, 110.23, 65.95. Anal. Calcd For C₃₁H₂₁N: C, 91.37; H, 5.19; N, 3.44. Found: C, 91.35, H, 5.24, N, 3.50 .
**1.1F:** ¹H NMR (400 MHz, benzene-d₆): δ [ppm] 8.04 (d, *J* = 1.7 Hz, 2H), 7.52 (d, *J* = 7.4 Hz, 4H), 7.39-7.30 (m, 10H), 7.11 (td, *J* = 7.5 Hz, 1.1 Hz, 4H), 7.03-6.93 (m, 10H), 6.80 (d, *J* = 8.5 Hz, 2H), 6.39 (s, 1H). ¹³C NMR (100 MHz, benzene-d₆): δ [ppm] 152.27, 146.74, 140.20, 138.72, 137.00, 126.68, 126.45, 126.36, 123.28, 120.08, 119.40, 110.41, 65.80. Anal. Calcd For C₅₀H₃₃N: C, 92.70; H, 5.13; N, 2.16. Found: C, 92.14, H, 4.93, N, 2.12.

### 9. Synthesis of 9-(2,12-di-tert-butyl-5,9-dioxa-13b-boranaphtho[3,2,1-de]anthracen-7-yl)-3-(9-phenyl-9H-fluoren-9-yl)-9H-carbazole (Compound 5)

In a 10 ml vial, Pd₂dba₃ (0.046 g; 0.05 mmol; 5 mol%) and [*t*-Bu₃PH]BF₄ (0.029 g; 0.10 mmol; 10 mol%) were mixed in dry, degassed toluene (2 ml) and stirred for 10 minutes in an inert gas atmosphere. In a Schlenk flask, **LIE** (0.489 g; 1.2 mmol) was dissolved in dry, degassed toluene (10 ml), *t*-BuONa (0.192 g; 2 mmol) was added, and t*he* mixture was stirred for 5 minutes under argon. 7-bromo-2,12-di-*tert*-butyl-5,9-dioxa-13b-boranaphtho[3,2,1-de]anthracene (0.461 g; 1 mmol) was added, followed by the catalyst mixture, and the flask was immersed in an oil bath preheated to 100° C and stirred at this temperature overnight. It was cooled to room temperature and then the mixture reaction was transferred to separatory funnel extracted three times with chloroform (3 ×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetonitrile to yield 0.315 g of product (white powder, 40.0%).
**Compound 5:** ¹H NMR (400 MHz, benzene-d₆): δ [ppm] 9.0 (d, *J* = 2.2 Hz, 2H), 8.23 (d, *J* = 1.6 Hz, 1H), 7.67 (d, *J* = 7.3 Hz, 2H), 7.61-7.46 (m, 10H), 7.37-7.31 (m, 4H), 7.22 (td, *J* = 7.4 Hz, 1.1 Hz, 3H), 7.13-7.02 (m, 6H), 1.41 (s, 18H). ¹³C NMR (100 MHz, THF-d₈): δ [ppm] 158.85, 158.62, 151.82, 146.78, 145.34, 143.71, 140.57, 140.25, 139.16, 138.54, 134.49, 134.33, 131.78, 130.04, 129.36, 128.08, 127.41, 127.20, 126.89, 126.31, 126.26, 125.97, 123.92, 123.77, 120.33, 120.15, 119.99, 119.33, 117.83, 109.84, 109.80, 106.14, 65.61, 34.32, 30.87. Anal. Calcd For C₅₇H₄₆BNO₂: C, 86.90; H, 5.89; B, 1.37; N, 1.78; O, 4.06. Found: C, 82.49; H, 5.48; N, 1.71.

### 10. Synthesis of 9-(2,12-di-tert-butyl-5,9-dioxa-13b-boranaphtho[3,2,1-de]anthracen-7-yl)-3,6-bis(9-phenyl-9H-fluoren-9-yl)-9H-carbazole (Compound 6)

In a 10 ml vial, Pd₂dba₃ (0.029 g; 0.03 mmol; 5 mol%) and [*t*-Bu₃PH]BF₄ (0.018 g; 0.06 mmol; 10 mol%) were mixed in dry, degassed toluene (2 ml) and stirred for 10 minutes in an inert gas atmosphere. In a Schlenk flask, **1.1F** (0.486 g; 0.75 mmol) was dissolved in dry, degassed toluene (10 ml), *t*-BuONa (0.120 g; 1.25 mmol) was added, and the mixture was stirred for 5 minutes under argon. 7-bromo-2,12-di-*tert*-butyl-5,9-dioxa-13b-boranaphtho[3,2,1-*de*]anthracene (0.288 g; 0.63 mmol) was added, followed by the catalyst mixture, and the flask was immersed in an oil bath preheated to 100° C and stirred at this temperature overnight. It was cooled to room temperature and then the mixture reaction was transferred to separatory funnel extracted three times with chloroform (3 ×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetone to yield 0.250 g of product (white powder, 38.9%).
**Compound 6:** ¹H NMR (400 MHz, benzene-d₆): δ [ppm] 8.99 (d, *J* = 2.1 Hz, 2H), 8.09-8.07 (m, 2H), 7.59-7.51 (m, 8H), 7.44-7.31 (m, 14H), 7.18-7.12 (m, 4H), 7.09-6.99 (m, 10H), 1.41 (s, 18H). ¹³C NMR (100 MHz, THF-d₈): δ [ppm] 158.84, 159.59, 151.80, 146.82, 145.33, 143.69, 140.19, 139.48, 138.32, 131.72, 130.02, 127.98, 127.86, 127.28, 127.11, 126.85, 126.24, 123.85, 119.92, 119.61, 117.82, 109.66, 106.01, 65.57, 34.30, 30.87. Anal. Calcd For C₇₆H₅₈BNO₂: C, 88.79; H, 5.69; B, 1.05; N, 1.36; O, 3.11. Found: C, 87.91; H, 5.59; N, 1.26.

### 11. Synthesis of 3-(9-fenylo-9H-fluoren-9-ylo)-10H-fenotiazyna (1.1G) and 3-(9-phenyl-9H-fluoren-9-yl)-10H-phenothiazine (1.1H)

To a stirred solution of 10*H*-phenotiazine (0.836 g; 5 mmol) in dry DCM (50 ml) BF₃·Et₂O (0.36 g; 0.31 ml; 2.5 mmol) was added dropwise at room temperature under inert atmosphere. The solution was stirred for 5 minutes. Then 9-phenyl-9*H*-fluoren-9-ol (1.29 g; 5 mmol) in dry DCM (50 ml) was added dropwise within 2 hours. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-ethyl acetate) to yield products: 1.07 g (yellowish powder, 48.7%) of **1.1G** and 0.90 g (yellowish powder, 26.5%) of **1.1H**.
**1.1G:** ¹H NMR (400 MHz, benzene-d₆): δ [ppm] 7.54 (d, *J* = 7.4 Hz, 2H), 7.33 (d, *J* = 7.5 Hz, 2H), 7.28-7.24 (m, 2H), 7.14 -7.10 (m, 8H), 7.05-6.95 (m, 6H), 6.85 (dd, *J* = 8.2 Hz, 2.2 Hz, 1H), 6.73-6.68 (m, 2H), 6.54-6.48 (m, 1H), 5.90 (dd, *J* = 8.3 Hz, 1.5 Hz, 1H), 5.78 (d, *J* = 8.2 Hz, 1H), 4.90 (s, 1H). ¹³C NMR (100 MHz, benzene-d₆): δ [ppm] 151.44, 145.89, 141.72, 140.44, 140.18, 130.30, 127.06, 126.86, 126.80, 126.60, 126.41, 126.16, 122.29, 120.14, 118.56, 118.40, 114.19, 114.17, 64.99. Anal. Calcd For C₃₁H₂₁NS: C, 84.70; H, 4.82; N, 3.19; S, 7.29. Found: C, 80.66, H, 4.84, N, 2.73.
**1.1H:** ¹H NMR (400 MHz, benzene-d₆): δ [ppm] 7.54 (d, *J* = 7.4 Hz, 4H), 7.30 (d, *J* = 7.7 Hz, 4H), 7.27-7.22 )m, 4H), 7.12 (td, *J* = 7.5 Hz, 1.2 Hz, 4H), 7.07-6.94 (m, 12H), 6.86 (dd, *J* = 8.6 Hz, 1.9 Hz, 2H), 5.78 (d, *J* = 7.7 Hz, 2H), 4.80 (s, 1H). 13C NMR (100 MHz, THF-d₈): δ [ppm]. Anal. Calcd For C₅₀H₃₃NS: C, 88.33; H, 4.89; N, 2.06; S, 4.72. Found: C, 87.66, H, 4,82, N, 1.86.

### 12. Synthesis of 10-(2,12-di-tert-butyl-5,9-dioxa-13b-boranaphtho[3,2,1-de]anthracen-7-yl)-3-(9-phenyl-9H-fluoren-9-yl)-10H-phenothiazine (Compound 7)

In a 10 ml vial, Pd₂dba₃ (0.046 g; 0.05 mmol; 5 mol%) and [*t*-Bu₃PH]BF₄ (0.029 g; 0.10 mmol; 10 mol%) were mixed in dry, degassed toluene (2 ml) and stirred for 10 minutes in an inert gas atmosphere. In a Schlenk flask, **1.1G** (0.523 g; 1.2 mmol) was dissolved in dry, degassed toluene (10 ml), *t*-BuONa (0.192 g; 2 mmol) was added, and the mixture was stirred for 5 minutes under argon. 7-bromo-2,12-di-*tert*-butyl-5,9-dioxa-13b-boranaphtho[3,2,1-*de*]anthracene (0.461 g; 1 mmol) was added, followed by the catalyst mixture, and the flask was immersed in an oil bath preheated to 100 °C and stirred at this temperature overnight. It was cooled to room temperature and then the mixture reaction was transferred to separatory funnel extracted three times with chloroform (3 ×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetone to yield 0.290 g of product (yellowish powder, 35.4%).
**Compound 7:** ¹H NMR (400 MHz, benzene-d₆): δ [ppm] 8.95 (d, *J* = 2.4 Hz, 2H), 7.56 (d, *J* = 7.4 Hz, 2H), 7.51 (d, *J* = 2.4 Hz, 1H), 7.49 (d, *J* = 2.3 Hz, 2H), 7.42 (d, *J* = 8.9 Hz, 2H), 7.31 (d, *J* = 7.4 Hz, 2H), 7.26-7.23 (m, 2H), 7.14 (td, *J* = 7.4 Hz, 1.3 Hz, 2H), 7.10 (s, 2H), 7.06-6.96 (m, 6H), 6.91 (dd, *J* = 8.5 Hz, 2.2 Hz, 1H), 6.87 (dd, *J* = 8.0 Hz, 1.2 Hz, 1H), 6.77-6.71 (m, 2H), 6.65 (td, *J* = 7.5 Hz, 1.4 Hz, 1H), 1.38 (s, 1H). ¹³C NMR (100 MHz, THF-d₈): δ [ppm] 158.87, 158.79, 150.81, 150.61, 145.57, 144.88, 143.71, 140.70, 140.22, 131.10, 129.92, 128.13, 128.06, 127.96, 127.58, 127.50, 127.46, 127.31, 127.13, 126.49, 126.10, 125.20, 120.05, 117.51, 99.47, 64.96, 34.22, 30.88. Anal. Calcd For C₅₇H₄₆BNO₂S: C, 85.17; H, 5.77; B, 1.34; N, 1.74; O, 5.97. Found: C, 85.87; H, 5.93; N, 1.19.

### 13. Synthesis of 10-(5,9-dioxa-13b-boranaphtho[3,2,1-de]anthracen-7-yl)-3-(9-phenyl-9H-fluoren-9-yl)-10H-phenoxazine (Compound 7a)

Synthesis of **Compound 7a** was obtained according to the method of synthesis **Compound 7** (disclosed in Point 12 above), except that instead of 7-bromo-2,12-di-*tert*-butyl-5,9-dioxa-13b-boranaphtho[3,2,1-*de*]anthracene, 7-bromo-5,9-dioxa-13b-boranaphtho[3,2,1-*de*]anthracene was used.
**Compound 7a:** Recorded ¹H NMR spectra confirmed the presence of the desired reaction product.

### 14. Synthesis of 10-(2,12-di-tert-butyl-5,9-dioxa-13b-boranaphtho[3,2,1-de]anthracen-7-yl)-3,7-bis(9-phenyl-977-fluoren-9-yl)-10H-phenothiazine (Compound 8)

In a 10 ml vial, Pd₂dba₃ (0.029 g; 0.03 mmol; 5 mol%) and [*t*-Bu₃PH]BF₄ (0.018 g; 0.06 mmol; 10 mol%) were mixed in dry, degassed toluene (2 ml) and stirred for 10 minutes in an inert gas atmosphere. In a Schlenk flask, **1.1H** (0.510 g; 0.75 mmol) was dissolved in dry, degassed toluene (10 ml), t-BuONa (0.120 g; 1.25 mmol) was added, and the mixture was stirred for 5 minutes under argon. 7-bromo-2,12-di-*tert*-butyl-5,9-dioxa-13b-boranaphtho[3,2,1-*de*]anthracene (0.288 g; 0.63 mmol) was added, followed by the catalyst mixture, and the flask was immersed in an oil bath preheated to 100° C and stirred at this temperature overnight. It was cooled to room temperature and then the mixture reaction was transferred to separatory funnel extracted three times with chloroform (3 ×10 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetone to yield 0.302 g of product (yellowish powder, 48.3%).
**Compound** 8: ¹H NMR (400 MHz, benzene-d₆): δ [ppm] 8.96, d, *J* = 2.4 Hz, 2H), 7.57-7.49 (m, 6H), 7.43 (d, *J* = 8.8 Hz, 2H), 7.31-7.26 (m, 6H), 7.24-7.21 (m, 4H), 7.13 (td, *J* = 7.5 Hz, 1.1 Hz, 4H(, 7.06 (s, 2H), 7.01 (td, *J* = 7.5 Hz, 1.1 Hz, 4H), 6.98-6.94 (m, 6H), 6.81 (dd, *J* = 8.6 Hz, 2.3 Hz, 2H), 6.51 (d, *J* = 8.5 Hz, 2H), 1.38 (s, 18H). ¹³C NMR (100 MHz, THF-d₈): δ [ppm] 158.88, 158.78, 150.80, 145.54, 144.96, 143.19, 140.97, 140.18, 131.21, 129.95, 128.46, 128.02, 127.93, 127.54, 127.41, 127.37, 127.09, 126.44, 126.07, 122.32, 120.02, 117.56, 101.39, 64.87, 34.23. Anal. Calcd For C₇₆H₅₈BNO₂S: C, 87.43; H, 5.60; B, 1.04; N, 1.34; O, 4.60. Found: C, 85.87; H, 5.93; N, 1.19.

### 15. Synthesis of 7-chloro-5,9-diphenyl-5,9-dihydro-5,9-diaza-13b-boranaphtho[3,2,1-de]anthracene (DABNA-Cl)

Synthesis of **DABNA-Cl** was obtained according to the method disclosed in Li, H. et al. Regioisomeric effects of dibenzofuran on the properties of boron-nitrogen multiple resonance emissive materials. J. Mater. Chem. C 11, 15548-15554 (2023), DOI: 10.1039/D3TC02708B.
**DABNA-Cl:** Recorded ¹H NMR spectra confirmed the presence of the desired reaction product.

### 16. Synthesis of 10,10-diphenyl-2,8-bis(9-phenyl-9H-fluoren-9-yl)-5,10-dihydrodibenzo[b,e][1,4]azasiline (1.1I)

To a stirred solution of 10,10-diphenyl-5,10-dihydrodibenzo[*b,e*][1,4]azasiline (3.49 g; 10 mmol) in dry DCM (200 ml) BF₃·Et₂O (3.12 g; 2.72 ml; 22 mmol) was added dropwise at room temperature under inert atmosphere. The solution was stirred for 5 minutes. Then 9-phenyl-9*H*-fluoren-9-ol (5.16 g; 20 mmol) in dry DCM (100 ml) was added dropwise within 2 hours. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-ethyl acetate) to yield product: 3.03 g (white powder, 36.5%) of **1.11I.**

### 1.11: Recorded ¹H NMR spectra confirmed the presence of the desired reaction product.

### 17. Synthesis of 7-(10,10-diphenyl-2,8-bis(9-phenyl-9H-fluoren-9-yl)dibenzo[b,e][1,4]azasilin-5(10H)-yl)-5,9-diphenyl-5,9-dihydro-5,9-diaza-13b-boranaphtho[3,2,1-de]anthracene (Compound 9)

In a 10 ml vial, Pd₂dba₃ (0.046 g; 0.05 mmol; 10 mol%) and XPhos (0.048 g; 0.1 mmol; 20 mol%) were mixed in dry, degassed toluene (4 ml) and stirred for 10 minutes under inert atmosphere. In a Schlenk flask, **1.11** (0.829 g; 1 mmol) was dissolved in dry, degassed toluene (25 ml), t-BuONa (0.192 g; 2 mmol) was added, and the mixture was stirred for 5 minutes under argon. **DABNA-Cl** (0.227 g; 0.5 mmol) was added, followed by the catalyst mixture, and the flask was immersed in an oil bath preheated to 100° C and stirred at this temperature overnight. It was cooled to room temperature and the mixture reaction was transferred to separatory funnel, extracted three times with chloroform (3×30 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetone to yield 0.182 g of product - **Compound 9** (yellowish powder, 29.1 %).
**Compound 9**: Recorded ¹H NMR spectra confirmed the presence of the desired reaction product.

### 18. Synthesis of 10-(5,9-diphenyl-5,9-dihydro-5,9-diaza-13b-boranaphtho[3,2,1-de]anthracen-7-yl)-10H-spiro[acridine-9,9'-xanthene] (1.1J)

In a 10 ml vial, Pd₂dba₃ (0.092 g; 0.1 mmol; 10 mol%) and XPhos (0.095 g; 0.2 mmol; 20 mol%) were mixed in dry, degassed toluene (4 ml) and stirred for 10 minutes in an inert gas atmosphere. In a Schlenk flask, 1077-spiro[acridine-9,9'-xanthene] (0.695 g; 2 mmol) was dissolved in dry, degassed toluene (30 ml), t-BuONa (0.384 g; 4 mmol) was added, and the mixture was stirred for 5 minutes under argon. **DABNA-Cl** (0.455 g; 1 mmol) was added, followed by the catalyst mixture, and the flask was immersed in an oil bath preheated to 100° C and stirred at this temperature overnight. It was cooled to room temperature and then the mixture reaction was transferred to separatory funnel extracted three times with chloroform (3×100 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetone to yield 0.286 g of product **- 1.1J** (light yellow powder, 37.4 %).
**1.1J:** Recorded ¹H NMR spectra confirmed the presence of the desired reaction product.

### 19. Synthesis of 10-(5,9-diphenyl-5,9-dihydro-5,9-diaza-13b-boranaphtho[3,2,1-de]anthracen-7-yl)-2,4,5,7-tetrakis(9-phenyl-9H-fluoren-9-yl)-10H-spiro[acridine-9,9'-xanthene] (Compound 10)

To a stirred solution of **1.1J** (0.286 g; 0.37 mmol) in dry DCM (20 ml) BF₃·Et₂O (0.315 g; 0.27 ml; 2.22 mmol) was added dropwise at room temperature under inert atmosphere. The solution was stirred for 5 minutes. Then 9-phenyl-9*H*-fluoren-9-ol (0.765 g; 2.96 mmol) in dry DCM (10 ml) was added dropwise within 30 minutes. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-dichloromethane) to yield product: 0.045 g (white powder, 7.1 %) of **Compound 10.**
**Compound 10**: Recorded ¹H NMR spectra confirmed the presence of the desired reaction product.

### 20. Synthesis of 2,12-di-tert-butyl-7-chloro-5,9-diphenyl-5,9-dihydro-5,9-diaza-13b-boranaphtho[3,2,1-de]anthracene (t-DABNA-Cl)

Synthesis of 2,12-di-*tert*-butyl-7-chloro-5,9-diphenyl-5,9-dihydro-5,9-diaza-13b-boranaphtho[3,2,1-*de*]anthracene **(*t*-DABNA-Cl)** was obtained according to the method disclosed in Mubarok, H., Lee, T., Jung, J. & Lee, M. H. Boron- and nitrogen-embedded blue multi-resonance emitters with low triplet energy. Bull. Korean Chem. Soc. 45, 16-22 (2024), DOI: 10.1002/bkcs.12793, except that instead of 4-*tert*-butylaniline, 4-(*tert*-butyl)-*N*-phenylaniline was used*.*
***t*-DABNA-Cl:** Recorded ¹H NMR spectra confirmed the presence of the desired reaction product.

### 21. Synthesis of 10-(2,12-di-tert-butyl-5,9-diphenyl-5,9-dihydro-5,9-diaza-13b-boranaphtho[3,2,1-de]anthracen-7-yl)-10H-spiro[acridine-9,9'-thioxanthene] (1.1K)

In a 10 ml vial, Pd₂dba₃ (0.092 g; 0.1 mmol; 10 mol%) and XPhos (0.095 g; 0.2 mmol; 20 mol%) were mixed in dry, degassed toluene (4 ml) and stirred for 10 minutes in an inert gas atmosphere. In a Schlenk flask, 10*H-*spiro[acridine-9,9'-thioxanthene] (0.727 g; 2 mmol) was dissolved in dry, degassed toluene (30 ml), t-BuONa (0.384 g; 4 mmol) was added, and the mixture was stirred for 5 minutes under argon. ***t*-DABNA-Cl** (0.567 g; 1 mmol) was added, followed by the catalyst mixture, and the flask was immersed in an oil bath preheated to 100° C and stirred at this temperature overnight. It was cooled to room temperature and then the mixture reaction was transferred to separatory funnel extracted three times with chloroform (3×100 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetone to yield 0.253 g of product - **1.1K** (light brown powder, 28.3 %).

**1.1K**: Recorded ¹H NMR spectra confirmed the presence of the desired reaction product.

### 22. Synthesis of 10-(2,12-di-tert-butyl-5,9-diphenyl-5,9-dihydro-5,9-diaza-13b-boranaphtho[3,2,1-de]anthracen-7-yl)-2,4,5,7-tetrakis(9-phenyl-9H-fluoren-9-yl)-10H-spiro[acridine-9,9'-thioxanthene] (Compound 11)

To a stirred solution of **1.1K** (0.253 g; 0.28 mmol) in dry DCM (20 ml) BF₃·Et₂O (0.238 g; 0.21 ml; 1.68 mmol) was added dropwise at room temperature under inert atmosphere. The solution was stirred for 5 minutes. Then 9-phenyl-9*H*-fluoren-9-ol (0.579 g; 2.24 mmol) in dry DCM (10 ml) was added dropwise within 30 minutes. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-dichloromethane) to yield product: 0.057 g (white powder, 11 %) of **Compound 11**.

**Compound 11:** Recorded ¹H NMR spectra confirmed the presence of the desired reaction product.

### 23. Synthesis of 10-(2,12-di-tert-butyl-5,9-diphenyl-5,9-dihydro-5,9-diaza-13b-boranaphtho[3,2,1-de]anthracen-7-yl)-10H-spiro[acridine-9,9'-fluorene] (1.1L)

In a 10 ml vial, Pd₂dba₃ (0.092 g; 0.1 mmol; 10 mol%) and XPhos (0.095 g; 0.2 mmol; 20 mol%) were mixed in dry, degassed toluene (4 ml) and stirred for 10 minutes in an inert gas atmosphere. In a Schlenk flask, 10*H-*spiro[acridine-9,9'-fluorene] (0.663 g; 2 mmol) was dissolved in dry, degassed toluene (30 ml), *t*-BuONa (0.384 g; 4 mmol) was added, and the mixture was stirred for 5 minutes under argon. ***t*-DABNA-Cl** (0.567 g; 1 mmol) was added, followed by the catalyst mixture, and the flask was immersed in an oil bath preheated to 100° C and stirred at this temperature overnight. It was cooled to room temperature and then the mixture reaction was transferred to separatory funnel extracted three times with chloroform (3×100 ml). The organic layers were washed with saturated NaCl solution and dried (MgSO₄). After removal of the solvent, the crude product was adsorbed on silica gel, purified by flash column chromatography (petroleum ether-dichloromethane) and washed with a hot acetone to yield 0.353 g of product - **1.1L** (greyish powder, 41 %).
**1.1L**: Recorded ¹H NMR spectra confirmed the presence of the desired reaction product.

### 24. Synthesis of 10-(2,12-di-tert-butyl-5,9-diphenyl-5,9-dihydro-5,9-diaza-13b-boranaphtho[3,2,1-de]anthracen-7-yl)-2,4,5,7-tetrakis(9-phenyl-9H-fluoren-9-yl)-10H-spiro[acridine-9,9'-fluorene] (Compound 12)

To a stirred solution of **1.1L** (0.353 g; 0.41 mmol) in dry DCM (20 ml) BF₃·Et₂O (0.349 g; 0.30 ml; 2.46 mmol) was added dropwise at room temperature under inert atmosphere. The solution was stirred for 5 minutes. Then 9-phenyl-9*H*-fluoren-9-ol (0.846 g; 3.28 mmol) in dry DCM (10 ml) was added dropwise within 30 minutes. A completion of the reaction was monitored using HPLC-MS. After 3 hours the mixture reaction was transferred to separatory funnel and washed with three times water. The organic layer was dried (MgSO₄). After removal of the solvent, the crude product was adsorbed on silica gel and purified by flash column chromatography (petroleum ether-dichloromethane) to yield product: 0.069 g (white powder, 9.2 %) of **Compound 12.**

**Compound 12:** Recorded ¹H NMR spectra confirmed the presence of the desired reaction product.

### Example 3: The photophysical properties of compounds with the structure according to Formula I

To verify the photophysical properties of compounds according to the invention, transient photoluminescence measurements are performed in toluene (Tol) and methylene chloride (MC) at room temperature. In toluene, the prompt exciton lifetimes (tₚ) for both **Compound 1** and **Compound 2** are 23.6 ns and 16.2 ns, respectively, and the calculated delayed exciton lifetimes (t_{d}) are 1.67 ms and 1.21 ms, respectively. Such short delayed lifetime can be suitable to alleviate device efficiency roll-off. In polar solvent methylene chloride, both the materials showed very short delayed exciton lifetimes of 1.02 ms and 0.98 ms for **Compound 1** and **Compound 2,** respectively. This is due to the reduction of ΔE_{ST} value because of the stabilization of the S₁ state and strong ICT characteristics in polar conditions. Attaching phenyl-9-fluorenyl substituents at the 2^{nd} and 7^{th} position of the acridine donor does not have any negative impact on TADF properties. Moreover, the tₚ and t_{d} of **Compound 1** and **Compound 2** are comparable to 10-(2,12-di-tert-butyl-5,9-dioxa-13b-boranaphtho[3,2,1-de]anthracen-7-yl)-9,9-dimethyl-9,10-dihydroacridine (TDBA-Ac), which can be attributed to similar molecular skeleton. Subsequently, the PLQY measurements for both emitters in thin film state are determined by doping (10wt%) both emitters in dibenzo[b,d]furan-2,8-diylbis(diphenylphosphine oxide) (DBFPO) host medium. Both emitters **Compound 1** and **Compound 2** revealed high PLQY values of 94.7% and 98.4%, respectively. Such high values compared to that of parent TDBA-Ac (93.1%) can be attributed to the reduced aggregation by the increased intermolecular distance with the addition of 9-methyl-9-fluronyl unit.

The intersystem crossing rate of both emitters are in the order of ~10⁷S⁻¹, but the reverse inter system crossing rate (kRISC) of them are in the order of ~10⁵ S⁻¹. Although, both emitters exhibited small ΔE_{ST}, but the kRISC of **Compound 2** has around 1.6 times lower kRISC (6.0 · 10⁵ S⁻¹) than the unsymmetrically oriented **Compound 1** (9.7 · 10⁵ S⁻¹). knr (non-radiative decay) values for **Compound 1** and **Compound 2** are 0.83 · 10⁵ and 0.32 · 10⁵ S⁻¹, respectively, which is slightly lower than that of parent TDBA-Ac (0.97 · 10⁵ S⁻¹). This result confirms that the reduced non-radiative decay rate is responsible for higher PLQY of **Compound 1** and **Compound 2.** The summary of photophysical and electrochemical properties of above-mentioned compound are in **Table 1.**

**Table 1. Summary of photophysical and electrochemical properties of TDBA-Ac, Compound 1 and Compound 2**

| **Emitter** | **λabs^{a} (nm)** | **λPL max ^{a} (nm)** | **FWHM (Tol) (nm)** | **S₁/T₁ (Tol)^{b} (eV)** | **ΔE_{ST} (eV)** | **HOMO (eV)** | **LUMO (eV)** | **Prompt lifetime (Tol/MC) ^{c} (ns)** | **Delayed lifetime (Tol/MC)^{c} (µs)** |
|---|---|---|---|---|---|---|---|---|---|
| **TDBA-Ac** | 283, 381 | 458 | 50 | 3.11/3.05 | 0.06 | 5.62 | 2.62 | 6.6/- | 1.0/0.9 |
| **Compound 1** | 381 | 467 | 50 | 3.07/2.99 | 0.08 | 5.56 | 2.72 | 23.6/31.7 | 1.67/1.02 |
| **Compound 2** | 381 | 471 | 52 | 3.02/2.96 | 0.06 | 5.53 | 2.72 | 16.2/30.5 | 1.21/0.98 |

| | | | | | | | | | |
|---|---|---|---|---|---|---|---|---|---|
| (a) UV-vis absorption spectra and room temperature photoluminescence (PL) spectra (300 K) measured in toluene at 10⁻⁵ concentration; (b) Calculated from the onset fluorescence and phosphorescence spectra in toluene solution; (c) Measured from transient photoluminescence decay curve in toluene/methylene chloride solution. | | | | | | | | | |

To confirm the concentration quenching of both molecules and to compare with the TDBA-Ac molecule, the PLQY of the film was measured by varying the doping concentrations of 10, 20, 30 and 40 wt%. Regardless of the doping concentration, the materials showed almost similar PLQY values, at different levels of doping, the TDBA-Ac molecule exhibited a more significant decrease in PLQY, while **Compound 1** and **Compound 2** showed a minimal variation in PLQY, which indicates that the concertation quenching behaviour is reduced in these molecules. Such excellent EL performances are attributed to reduction of concentration quenching effect in **Compound 1** and **Compound** 2 whereas the decrease in EQE of the reference TDBA-Ac device at higher doping concentration were due to the higher concentration quenching rate as compared to **Compound 1** based device. Tailoring phenyl-9-fluoronyl group at acridine donor tend to impact on aggregation quenching effect noticeably. These results prove that compounds according to the invention are suitable for a use in OLEDs and exhibit good properties such as high photoluminescence quantum yield.

### Example 4: The emissive layer of diode

The organic light emitting diode comprising an anode, cathode, and organic emissive layer. The emissive layer is disposed between the anode and cathode. The electroluminescent diode can comprise an electrode 1 and electrode 2 that can be made of aluminum (Al), ITO and platinum (Pt), fluorine doped tin oxide (FTO) and silver (Ag), ITO and calcium (Ca), ITO and barium (Ba), ITO and Mg-Al, zinc oxide (ZnO) and Al. The emissive layer can comprise a compound according to Formula I or their deuterated analogues (as presented in the Example 1). The diode can comprise at least one of layer between the anode and cathode selected from: a hole injection layer; a hole transport layer; an electron transport layer, an electron blocking layer, a hole blocking layer, an electron injection layer; or a combination thereof. The hole transport layer can function as an electron blocking layer and the electron transport layer can function as a hole blocking layer. The diode can be made by thermal evaporation in high vacuum (PVD) or by solution methods, e.g. spin-coating, ink-jet printing. Exemplary structures of hole injection compounds in the hole injection layer (HIL) comprise the following:

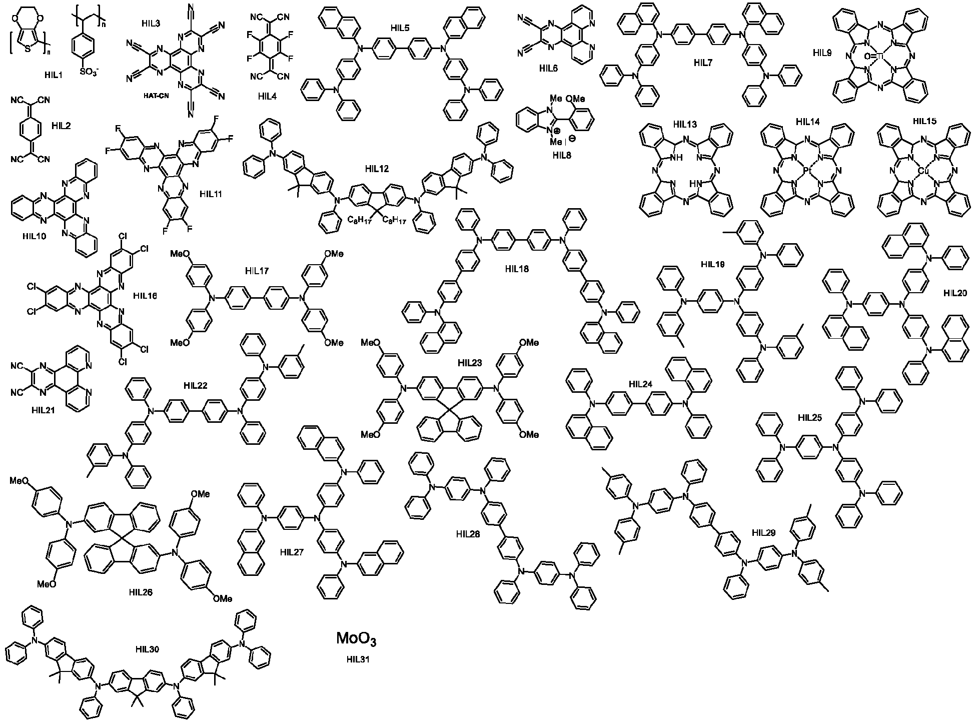

Exemplary structures of compounds of the hole transport layer (HTL):

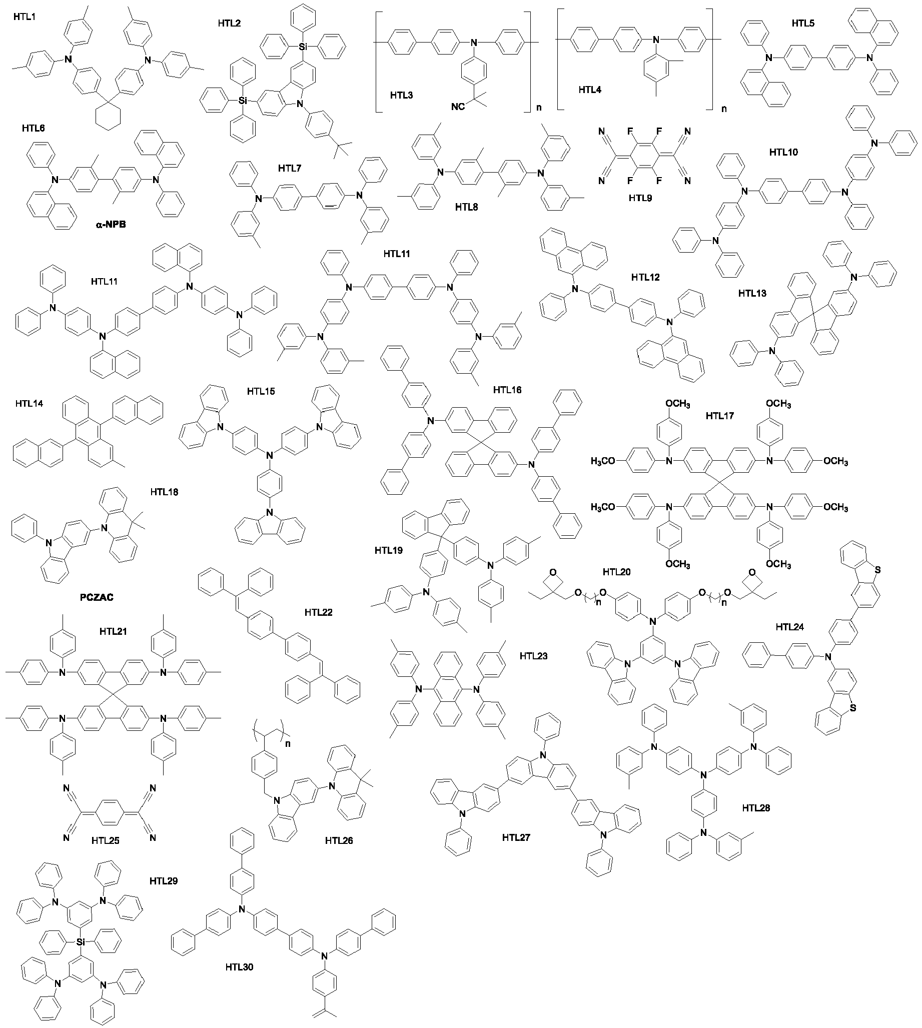

Exemplary structures of electron transporting compounds in the electron transport layer (ETL):

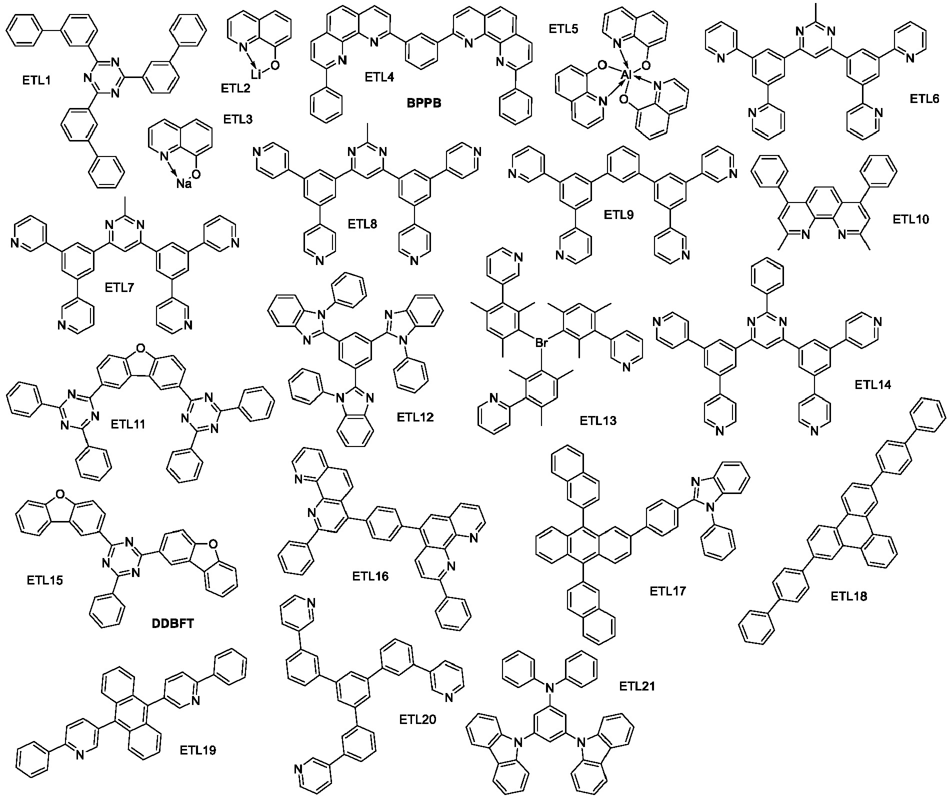

Exemplary structures of electron-injecting materials in the electron injection layer (EIL):

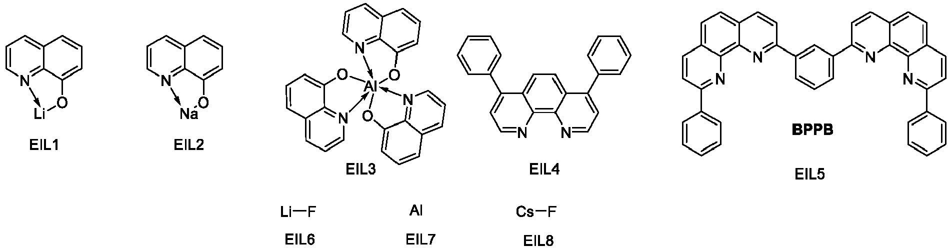

The examples of host (H) can be 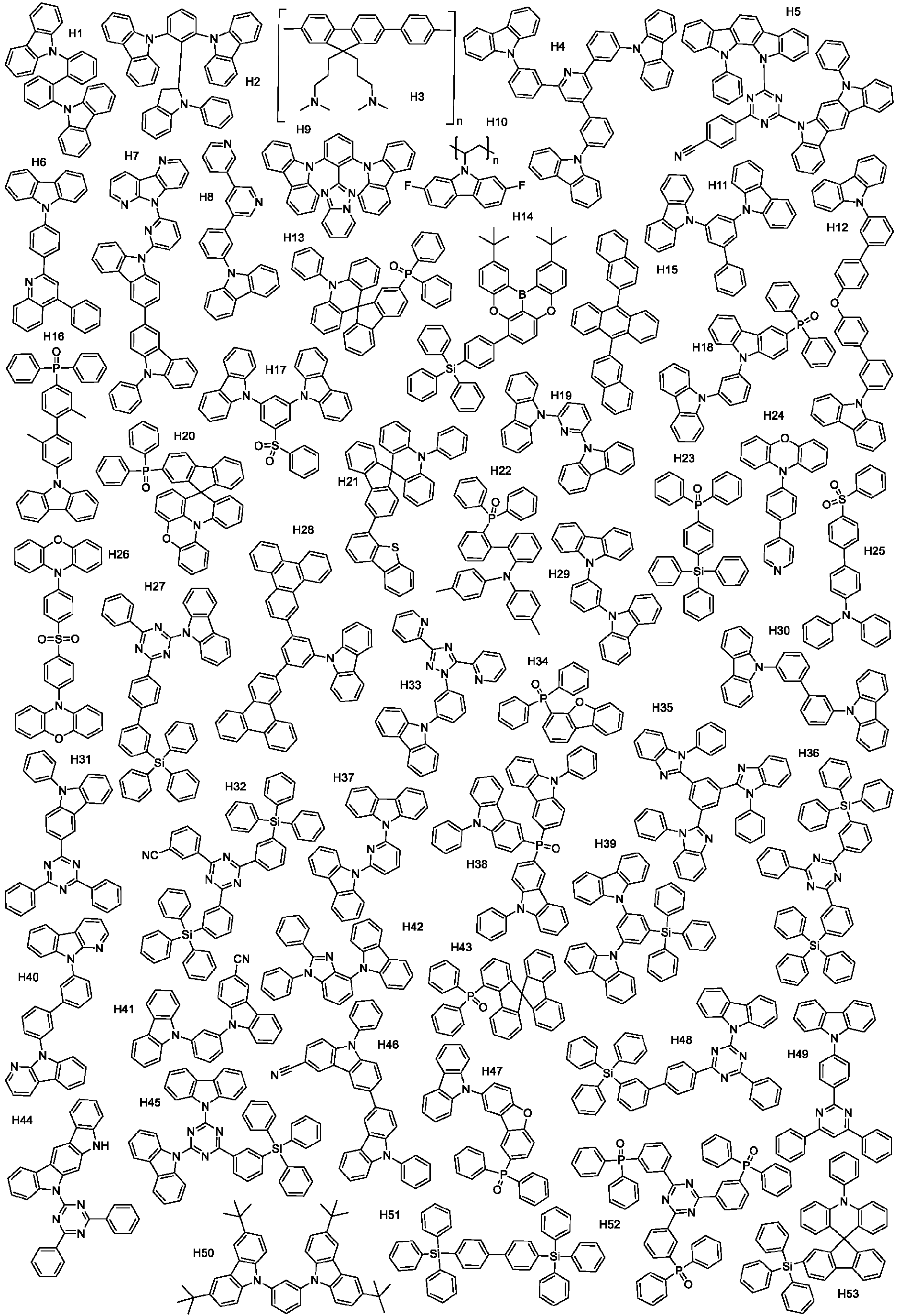 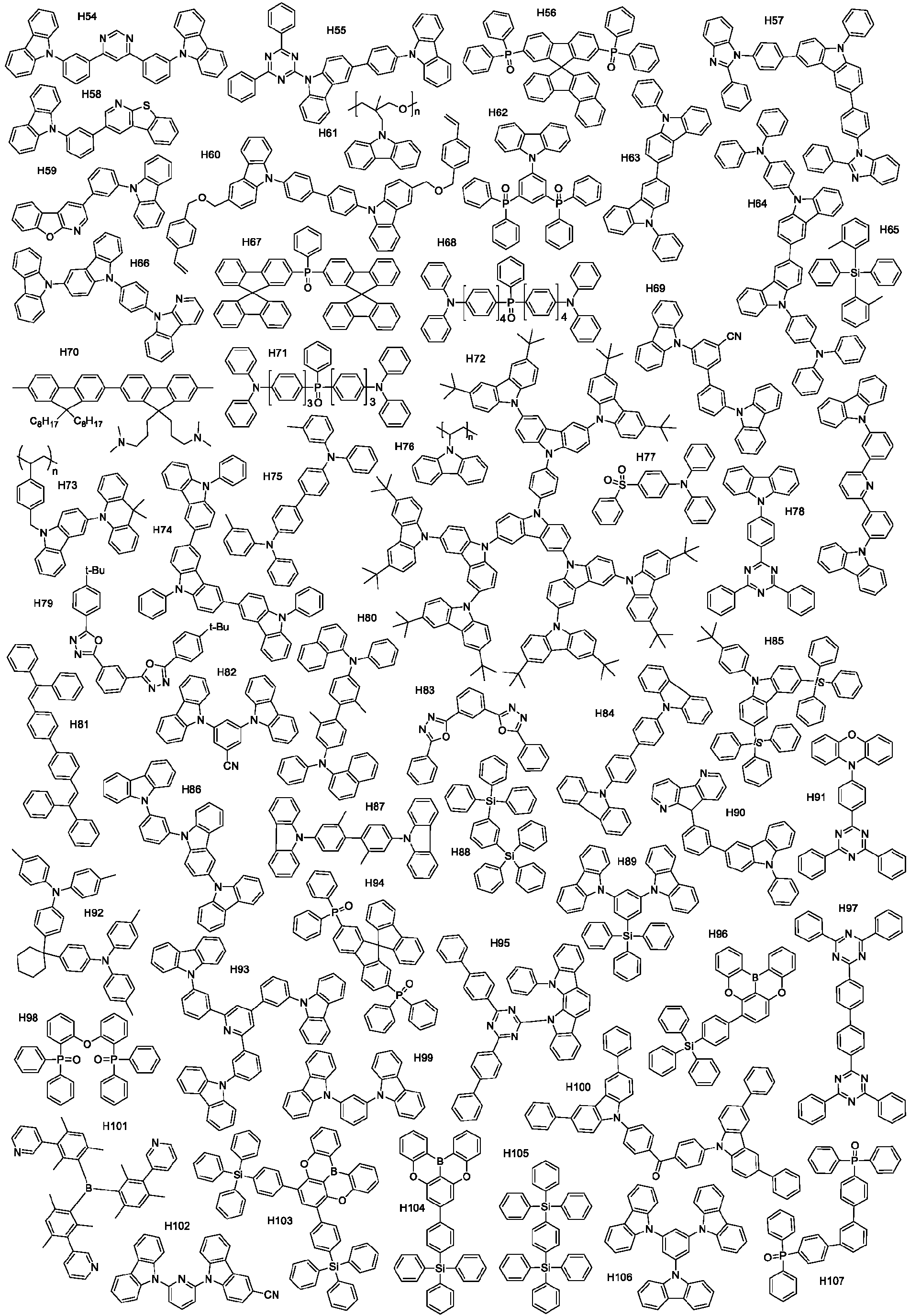 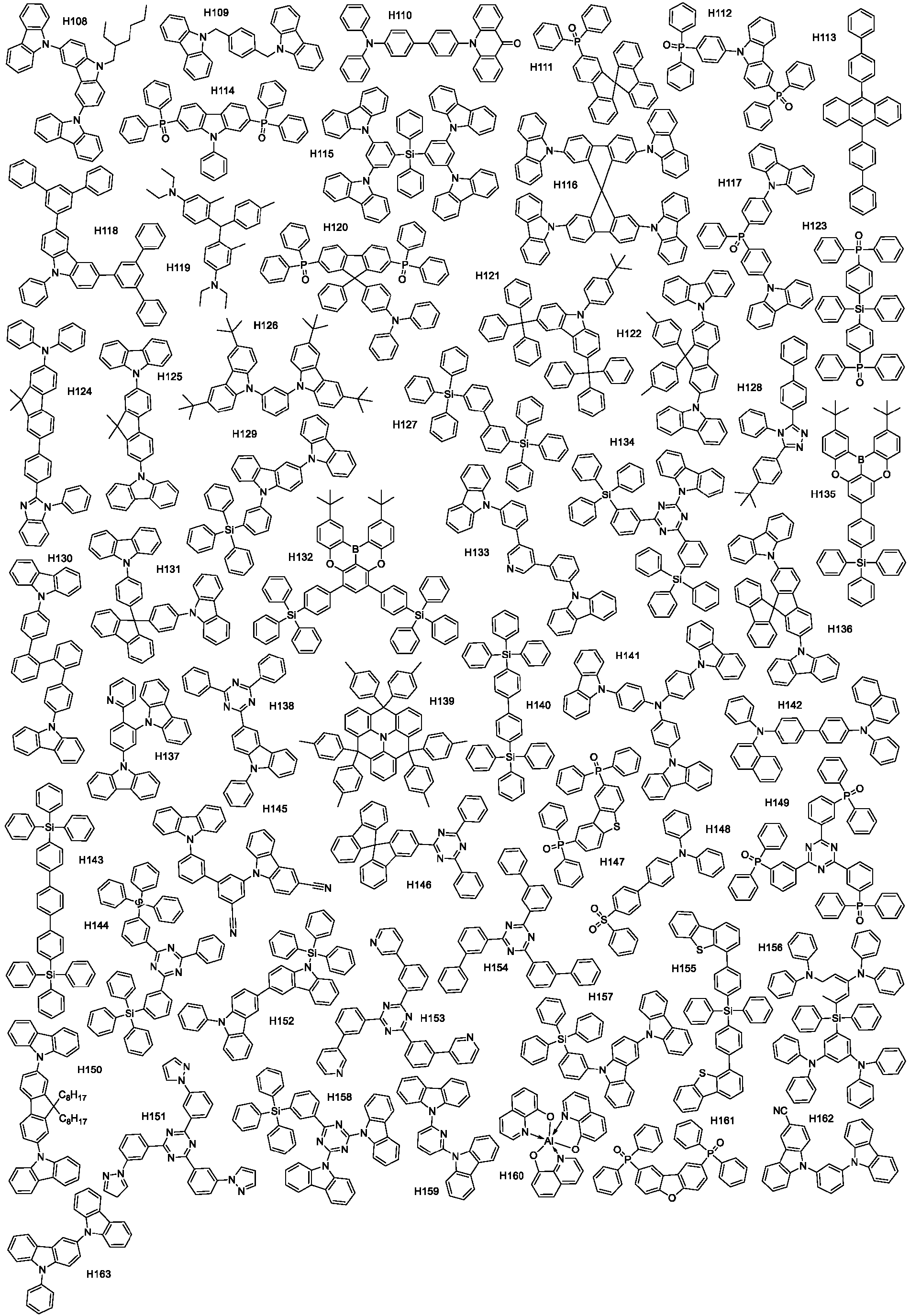 and deuterated analogues thereof. In the case of the hyper-fluorescence diodes, the fluorescent dopant is v-DABNA and an analogue deuterated analogue thereof.

The diode according to invention can comprise the host. The content of the host can constitute from 20.0% (w./w.) to 99.9% (w./w.) of the emissive layer. The content of the host can constitute from 20.0%, 22.5%, 25.0%, 27.5%, 30.0%, 35.0%, 40,0%, 45.0%, 50.0%, 55.0%, 60.0%, 62.5%, 65.0%, 70.0%, 75.0%, 80.0%, 85.0%, 87.5%, 90.0%, 92.5%, 95.0% (w./w.) to 99.0% (w./w.) of the emissive layer.

The diode according to invention can comprise the fluorescence dopant. The content of the fluorescence dopant can be from 0.01% (w./w.) to 99.9% (w./w.) of the emissive layer. The content of the fluorescence dopant or a can be from 0.05%, 0.1%, 1.0%, 1.5%, 2,0%, 2.5%, 3.0%, 5.0%, 7.0%, 8.0%, 9.0% (w./w.) to 9.5%, 10.0%, 15.0%, 20.0%, 25.0%, 30.0%, 35.0%, 40.0%, 45.0%, 50.0%, 55.0%, 60.0%, 65.0%, 70.0%, 75.0%, 80.0%, 85.0%, 90.0%, 95.0% (w./w.) of the emissive layer.

The diode according to the invention can comprise a matrix. The matrix can be passive or active. The diode according to invention can emit light with a maximum wavelength of 350 nm to 2 500 nm. The diode according to invention can emit light with a maximum wavelength of 400, 450, 500, 550, 600, 650, 700, 750, 800, 900 nm to 1000 nm.

The processes for obtaining chemical compounds presented in the diode according to the invention such as: dopants, hosts, the compounds of the hole injection layer, hole transport layer, electron transport layer, electron blocking layer, hole blocking layer, electron injection layer, are well known in the state of the art.

### The example 5: Electroluminescent diodes

Exemplary current-spectral measurements for exemplary electroluminescent diodes are presented in **Table 2.**

**Table 2. Current-spectral measurements for the diodes according to the invention**

| **No. of diode** | **Host/ Emitter weight ratio (wt %)** | **EQE (Max/@ 1000 cd/m²) (%)** | **EQE (Max/@ 5 000 cd/m²⁾ (%)** | **C.E. (Max/@ 1000 cd/A)** | **C.E. (Max/ @ 5000 cd/A)** | **EL max (nm)** | **CIE (x,y)** | **LT90 at 1000 cd/m² (h)** | **LT70 at 1000 cd/m² (h)** | **LT50 at 500 cd/m² (h)** | **FWHM (nm)** |
|---|---|---|---|---|---|---|---|---|---|---|---|
| **1** | DPFPO/ Compound 1 80/20 | 28.1/ 21.8 | | 37.8/ 27.6 | | 475 | (0.13, 0.20) | | | | 60 |
| **2** | DPFPO/ Compound 1 70/30 | 29.4/ 26.3 | | 41.9/ 35.1 | | 478 | (0.13, 0.23) | | | | 57 |
| **3** | DPFPO/ Compound 1 60/40 | 30.1/ 27.4 | | 43.1/ 38.1 | | 478 | (0.13, 0.25) | | | | 58 |
| **4** | DBFPO/ Compound 2 80/20 | 13.8/ 9.3 | | 21.6/ 13.2 | | 478 | (o.14, 0.24) | | | 1.0 | 62 |
| **5** | DBFPO/ Compound 1 70/30 | 11.5/ 8.9 | | 19.5/ 13.4 | | 481 | (0.14, 0.27) | | | 2.0 | 62 |
| **6** | NS60/ Compound 3 80/20 | | 13.2/ 10.7 | | 38.5/ 30.6 | 523 | (0.30/ 0.59) | | 67 | 233 | 80 |
| **7** | NS60/ Compound 3 70/30 | | 14.6/ 11.2 | | 43.0/ 32.5 | 524 | (0.30, 0.59) | | 67 | 223 | 80 |
| **8** | NS60/ Compound 3 60/40 | | 15.3/ 11.2 | | 45.0/ 32.5 | 525 | (0.30, 0.59) | | 67 | 233 | 80 |
| **9** | NS60/ Compound 3a 80/20 | | 13.8/ 11.2 | | 40.6/ 32.9 | 535 | | | | | 92 |
| **10** | NS60/ Compound 3a 70/30 | | 14.2/ 12.1 | | 41.6/ 34.2 | 537 | | | | | 92 |
| **11** | NS60/ Compound 3a 60/40 | | 14.4/ 10.3 | | 43.1/ 30.2 | 537 | | | | | 94 |
| **12** | NS60/ Compound 4 20/80 | | 9.2/ 2.8 | | 26.5/ 7.7 | 523 | (0.29, 0.59) | | 38 | 133 | 83 |
| **13** | NS60/ Compound 4 30/70 | | 12.0/ 6.5 | | 34.8/ 18.6 | 521 | (0.29, 0.58) | | 38 | 133 | 79 |
| **14** | NS60/ Compound 4 40/60 | | 14.0/ 9.0 | | 40.8/ 26.0 | 523 | (0.29, 0.59) | | 38 | 133 | 80 |
| **15** | DBFPO/ Compound 13 80/20 | | 8.9/ 5.3 | | 19.3/ 12.4 | | | | | | 88 |
| **16** | DBFPO/ Compound 13 70/30 | | 10.1/ 7.8 | | 20.8/ 11.7 | | | | | | 87 |
| **17** | 2CzPy/ Compound 14 80/20 | | 16.7/ 9.8 | | 29.6/ 22.3 | | | | | | 92 |
| **18** | 2CzPy/ Compound 14 70/30 | | 11.4/ 6.9 | | 23.4/ 14.7 | | | | | | 93 |
| **19** | DBFPO /Compound 15 80/20 | | 12.2/ 7.7 | | 25.3/ 19.0 | | | | | | 94 |
| **20** | DBFPO/ Compound 15 70/30 | | 10.8/ 5.4 | | 26.3/ 17.3 | | | | | | 94 |
| **21** | NS60/ Compound 16 20/80 | | 13.2/ 6.7 | | 31.9/ 16.4 | | | | | | 87 |
| **22** | NS60/ Compound 16 30/70 | | 14.1/ 8.3 | | 30.9/ 17.2 | | | | | | 86 |

| | | | | | | | | | | | |
|---|---|---|---|---|---|---|---|---|---|---|---|
| Abbreviations: EQE - external quantum efficiency; C.E.- Current Efficiency; ELₘₐₓ - a maximum wavelength of the diode emission; LT_{50/70/90} - operational lifetime of diode, wherein luminance decreases to 50%/70%/90% of the original, respectively; FWHM - full width at half maximum; CIE - CIE 1931 colour spaces. | | | | | | | | | | | |

The compounds according to the invention are characterized by high external quantum efficiency of electromagnetic emission, and high luminescence value, such that they can be used in electroluminescent devices, e.g. as emitters in OLED devices/diodes. The diodes can also provide an organic light emitting diode achieving high colour purity.

### Example 6: HF diodes

Even though the device performance of **Compound 1** as TADF emitter is better than TDBA-Ac, but the colour purity could be improved in pure blue region. Therefore, the compounds according to the invention can be a TADF emitter as TSH in an HF system using a well-known pure blue v-DABNA final emitter. Since the HF system works through a long-range Förster resonance energy transfer mechanism (depends on dipole-dipole interaction), spectral overlap between the TSH and final emitter is required. So, an effective FRET mechanism can be activated when a large portion of the final emitter's absorption spectra overlap with the emission spectra of TSH.

A similar device structure as above was constructed in Example 5 with only modifications in the emissive layer. This layer of the hyper-fluorescence system is as follows; DBFPO: TADF (40 wt%): v-DABNA (0.5 wt%) or TADF (40 wt%): 2CzPy (0.5 wt%). The hyper-fluorescence device exhibited excellent device properties, such as EQE maxima (36.2%) and current density (32.6 ca/A). Further, turn on voltage of both hyper-fluorescence and TADF devices are similar. Since the HOMO energy levels of TSH and v-DABNA are the same (5.5 eV), but the LUMO energy level of v-DABNA is slightly deeper than TSH, so there is a possibility of electron trapping in the hyper-fluorescence device. It is observed that the difference in plot between two devices are minimal, indicating charge trap emission is less. The hyper-fluorescence device achieved high colour purity (FWHM of 19 nm) in pure blue region (472 nm) with excellent device properties. The reference device using TDBA-Ac as TSH revealed poor device performances compared to the device using **Compound 1** as TSH (T**able 3**). Even for TDBA-Ac and **Compound 1,** the efficiency enhancement from TADF to HF device is quite comparable (~4-5%), suggesting that the FRET efficiency in both HF devices appears to be similar. Such good device performance of **Compound 1** can be attributed to high PLQY, the horizontal orientation of v-DABNA (0.90) and reduced concentration quenching. Exemplary current-spectral measurements for exemplary HF-OLED are presented in **Table 3.**

**Table 3. Summary of electroluminescence results of HF-OLED**

| **No. of diode** | **Host/Emitter/Dopant weight ratio (wt %)** | **EQE (Max/@1 000 cd/m2) (%)** | **EQE (Max/@5 000 cd/m2) (%)** | **C.E. (Max/@1 000 cd/A)** | **C.E. (Max/@5 000 cd/A)** | **ELₘₐₓ (nm)** | **FWHM (nm)** | **CIE (x,y)** |
|---|---|---|---|---|---|---|---|---|
| **21** | DBFPO/Compound 1/v-DABNA 59.5/40/0.5 | 36.2/30.3 | | 32.6/27.1 | | 472 | 19 | (0.12, 0.14) |
| **22** | 2CzPy /Compound 2/v-DABNA 59.5/40/0.5 | | 12.9/- | | 13.3/- | 472 | 19 | (0.12, 0.16) |
| **23** | 2CzPy/Compound 1/v-DABNA 59.5/40/0.5 | | 32.1/19.23 | | 27.1/16.74 | 471 | 19 | (0.11/0.13) |
| **24** | DBFPO/Compound 3/v-DABNA 59.5/40/0.5 | | 20.8/17.6 | | 31.0/26.3 | | 18 | |
| **25** | 2CzPy/Compound 13/v-DABNA 59.5/40/0.5 | 17.9/13.4 | | 24.5/18.6 | | | 20 | |
| **26** | DBFPO/Compound 13 /v-DABNA 59.5/40/0.5 | 14.8/ 11.6 | | 18.5/15.4 | | | 20 | |
| **27** | 2CzPy/Compound 14/v-DABNA 59.5/40/0.5 | | 13.6/10.1 | | 20.4/17.1 | | 19 | |
| **28** | DBFPO/Compound 14/v-DABNA 59.5/40/0.5 | | 11.6/7.5 | | 19.3/12.8 | | 19 | |
| **29** | 2CzPy/Compound 15/v-DABNA 59.5/40/0.5 | | 12.1/6.7 | | 20.1/15.6 | | | |
| **30** | DBFPO/Compound 15/v-DABNA 59.5/40/0.5 | | 10.8/6.3 | | 18.0/12.1 | | | |

### Example 7: Electroluminescent diodes

The diodes can comprise an anode, cathode, and organic emissive layer, disposed between an anode and cathode. The exemplary composition of these diodes according to the invention is presented in **Table 4**.

**Table 4. The exemplary composition of diodes the diodes according to the invention**

| **No. of diode** | **TSH** | **FD** | **H** | **HIL** | **HTL** | **HBL** | **ETL** | **EIL** | **EBL** |
|---|---|---|---|---|---|---|---|---|---|
| **31** | 1 | v-DABNA | H98 | HIL25 | HTL29 | H98* | ETL12 | EIL6 | - |
| **32** | 1 | | H98 | HIL25 | HIL25 | H154 | H154:EIL1 | H154:EIL1 | H30 |
| **33** | 2 | | H161 | HIL31 | HTL1:HTL15:H18 | H161 | ETL12 | EIL6 | - |
| **34** | 5 | | H161 | HIL4 | HTL1 | H161 | ETL12 | EIL6 | |
| **35** | 8 | | H162 | HIL4 | HIL25 | H54 | ETL18:EIL1 | EIL6 | HTL15 |
| **36** | 8 | | H69 | HIL4 | HIL25 | ETL15 | ETL16 | EIL6 | - |
| **37** | 13 | | H30 | HIL4 | HTL27 | H154 | ETL19 | EIL6 | H30 |
| **38** | 14 | | H30 | HTL11 | HTL30 | ETL11 | ETL17 | EIL6 | HTL18 |
| **39** | 15 | | H98 | HTL11 | HTL15 | H23 | ETL12 | EIL6 | H18 |
| **40** | 15 | | H69 | HIL4 | HTL15 | H161 | ETL9 | EIL1 | H163 |
| **41** | 15 | | H98 | HTL6 | H18 | - | ETL12 | EIL6 | H98 |

| | | | | | | | | | |
|---|---|---|---|---|---|---|---|---|---|
| * Host H98 can fulfil the function of a hole blocking layer and a host. | | | | | | | | | |

### Example 8: The use of electroluminescent diodes and a consumer product

A consumer product can comprise the diode according to the invention. The consumer product can be selected from a flat panel display, a computer monitor, a medical monitor, a television, a billboard, a light for interior or exterior illumination/signalling, a heads-up display, a fully or partially transparent display, a flexible display, a laser printer, a telephone, a cell phone, tablet, a phablet, a personal digital assistant, a wearable device, a laptop computer, a watch, a backlight, a digital camera, a camcorder, a 3-D display, a virtual reality or augmented reality display, a vehicle, a video walls comprising multiple displays tiled together, a theatre, stadium screen, and a sign. Use of the diode according to the invention can be as follows: a display screen or illumination device, an electroluminescent display device, an organic photovoltaic device, or a sensing device.

## Claims

1. A compound according to Formula I: 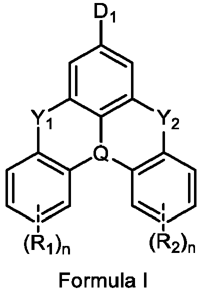 wherein:
Y₁, Y₂, together or independently, are O, S, Se, NR₉, C(R₉)₂ or Si(R₉)₂;
Q, together or independently, are B, P; P=O, Al, Ge, In;
R₁, R₂, R₃, R₄, R₅, R₆, R₇, R₈, together or independently, are hydrogen, deuterium, halogen, cyano, nitro, C₁-C₂₀ alkyl,
C₂-C₂₀ alkenyl, C₂-C₂₀ alkynyl, C₁-C₆₀ alkoxy group, C₃-C₆₀ cycloalkyl, C₃-C₆₀ cycloalkenyl, C₁-C₆₀ heterocycloalkyl,
C₁-C₆₀ heterocycloalkenyl group, C₃-C₆₀ cycloalkynyl group, C₁-C₆₀ heterocycloalkynyl group, hydroxyl, C₆-C₆₀ aryl,
C₁-C₆₀ heteroaryl group, C₅-C₆₀ aryloxy, C₁-C₆₀ heteroaryloxy group, C₂-C₆₀ ether group, C₆-C₆₀ thioaryl, C₁-C₆₀ heterothioaryl, C₁-C₆₀ amino, C₁-C₆₀ alkylamino, C₁-C₆₀ dialkylamino, C₆-C₆₀ arylamino, C₆-C₆₀ diarylamino, C₆-C₆₀ heteroarylamino group, C₆-C₆₀ heterodiarylamino group, C₄-C₆₀ non-aromatic fused polycyclic group, C₁-C₆₀ non-aromatic fused heteropolycyclic group or deuterated analogues thereof;
R₉, R₁₀, together or independently, are hydrogen, deuterium, C₁-C₂₀ alkyl, C₆-C₆₀ aryl group, C₁-C₆₀ heteroaryl group, C₅-C₆₀ aryloxy, C₁-C₆₀ heteroaryloxy group, C₆-C₆₀ arylamino, C₆-C₆₀ heteroarylamino, C₆-C₆₀ diarylamino, C₆-C₆₀ heteroarylamino group, C₆-C₆₀ diheterodiarylamino group, C₆-C₆₀ thioaryl group, C₁-C₆₀ heterothioaryl or deuterated analogues thereof;
an index n ranging from 0 - 4 indicates how many R₁ and R₂ may be attached to a particular ring of Formula I;
D₁ is selected from Formula IIa - Formula IIl: 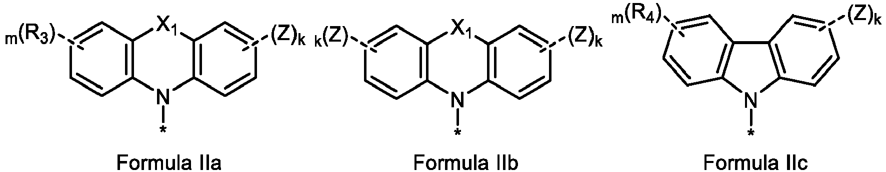 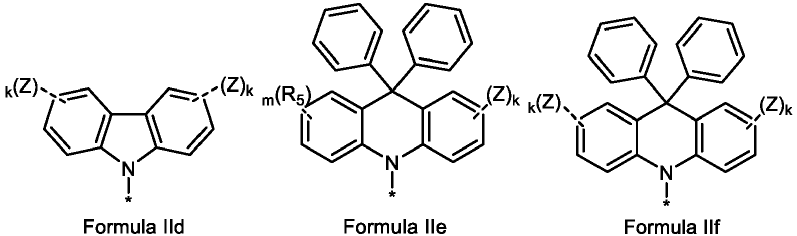 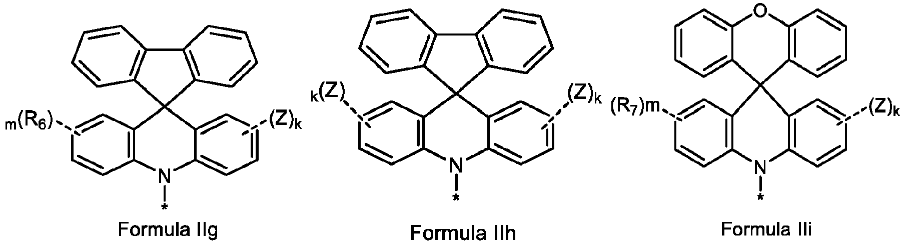 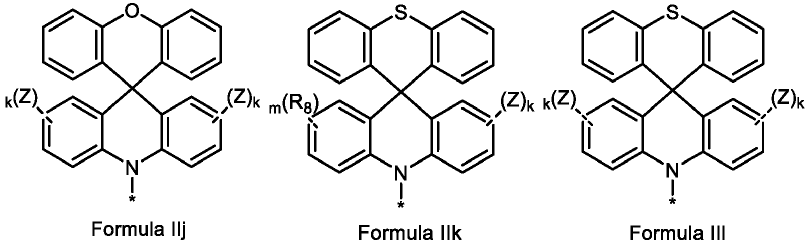 wherein:
X₁ is selected from C(R₁₀)₂, Si(R₁₀)₂, S, O, NR₁₀;
Z is: 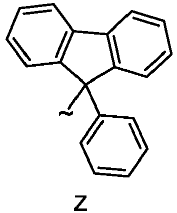
an index m ranging from 0 - 4 indicates how many R₃, R₄, R₅, R₆, R₇, R₈ may be attached to a particular ring of Formula IIa, Formula IIb, Formula IIc, Formula IId, Formula IIe, Formula IIf, Formula IIg, Formula IIh, Formula IIi, Formula IIj, Formula IIk, or Formula IIl;
an index k ranging from 0 - 4 indicates how many Z may be attacked to a particular ring of Formula IIa, Formula IIb, Formula IIc, Formula IId, Formula IIe, Formula IIf, Formula IIg, Formula IIh, Formula IIi, Formula IIj, Formula IIk, or Formula IIl;
~ each indicate a binding site of Z to Formula IIa, Formula IIb, Formula IIc, Formula IId, Formula IIe, Formula IIf, Formula IIg, Formula IIh, Formula IIi, Formula IIj, Formula IIk, and Formula IIl;
* each indicate a binding site of Formula IIa, Formula IIb, Formula IIc, Formula IId, Formula IIe, Formula IIf, Formula IIg, Formula IIh, Formula IIi, Formula IIj, Formula IIk, and Formula III to Formula I.

2. A compound according to claim 1, wherein the compound according to Formula I is selected from:

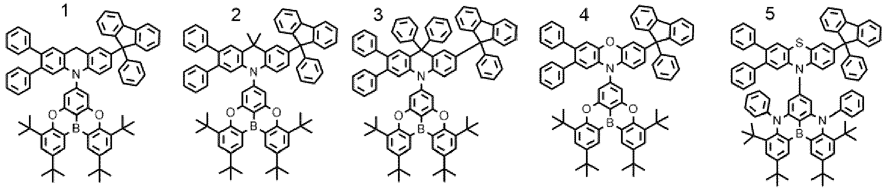

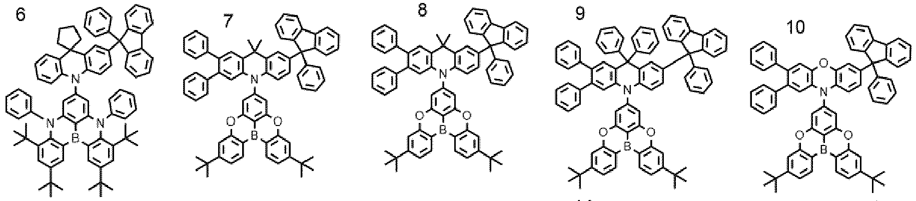

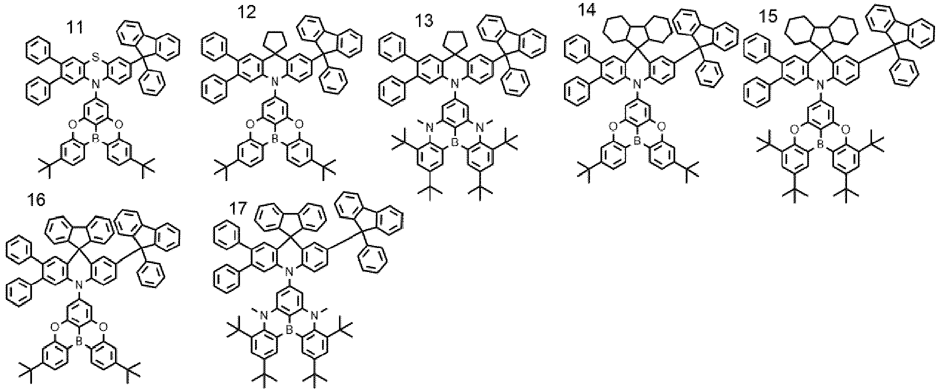

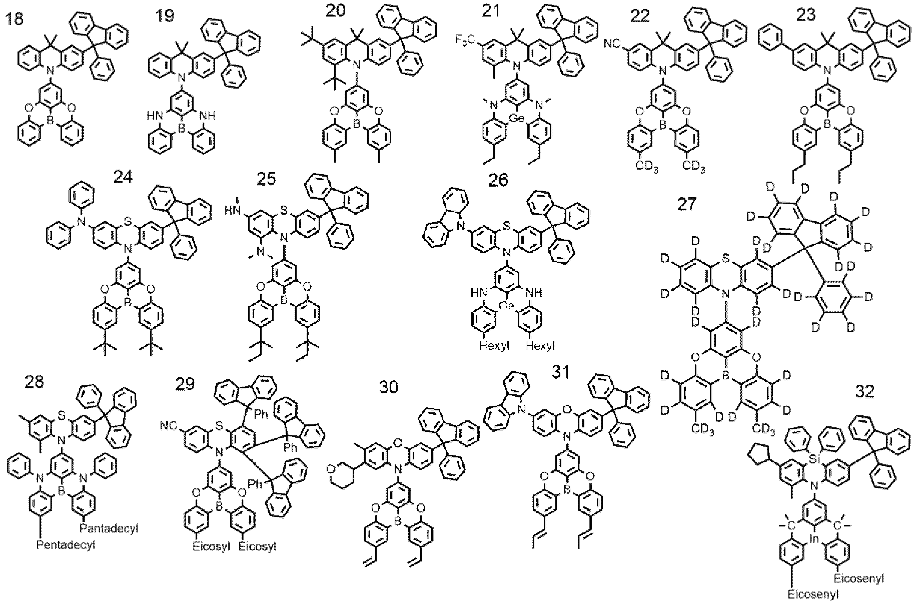

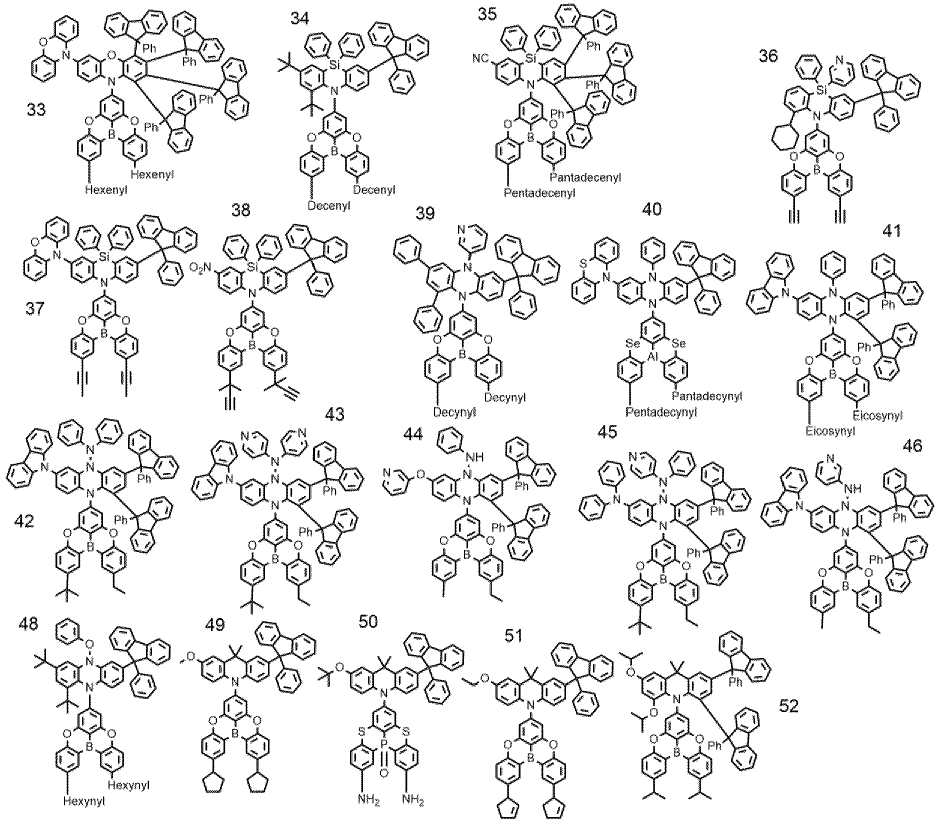

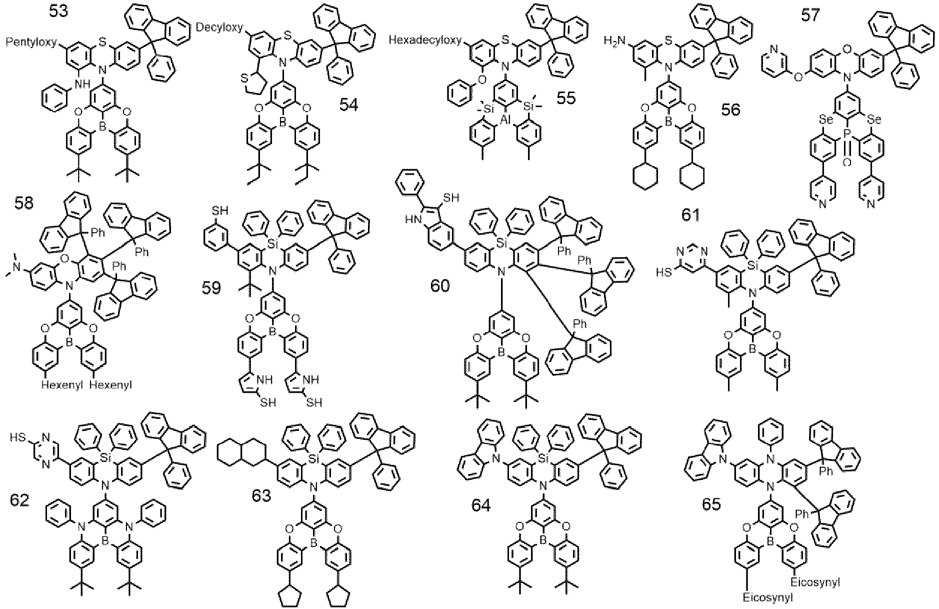

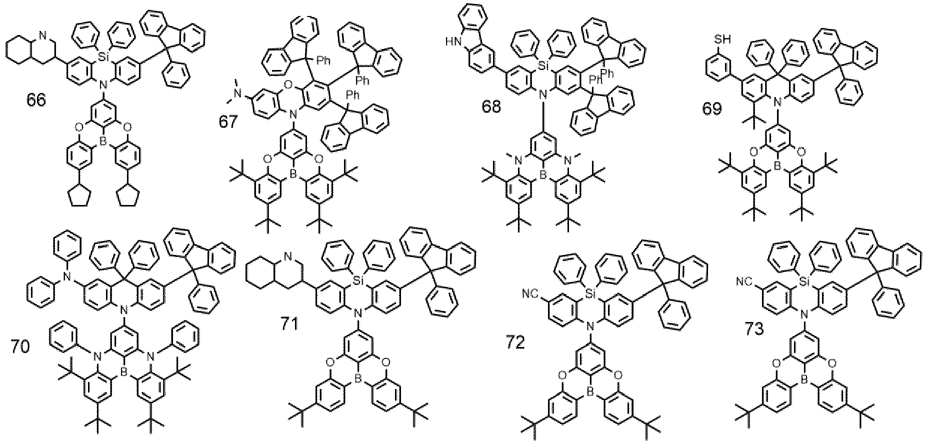

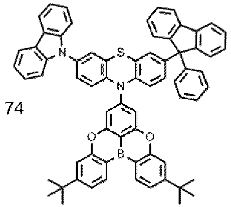

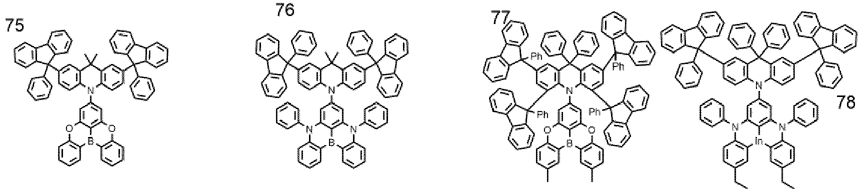

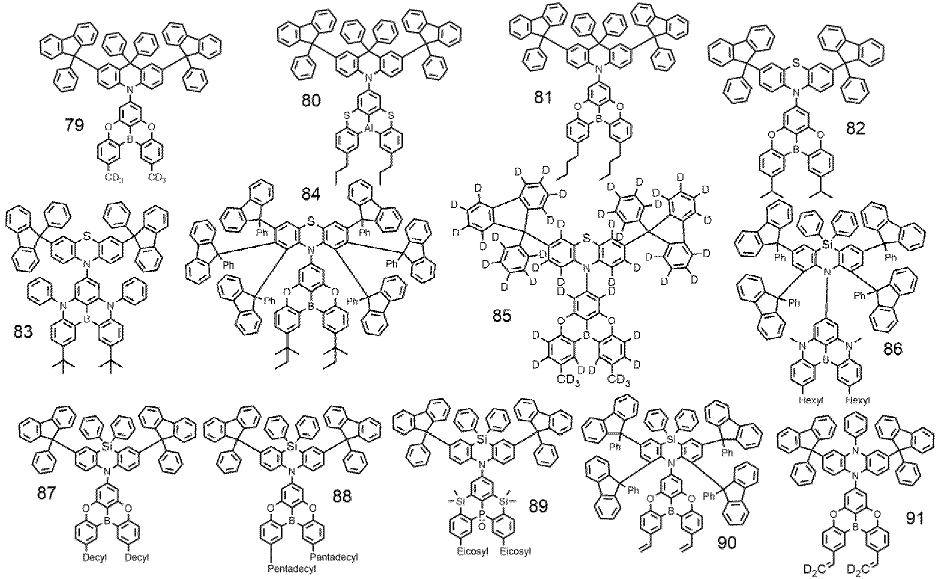

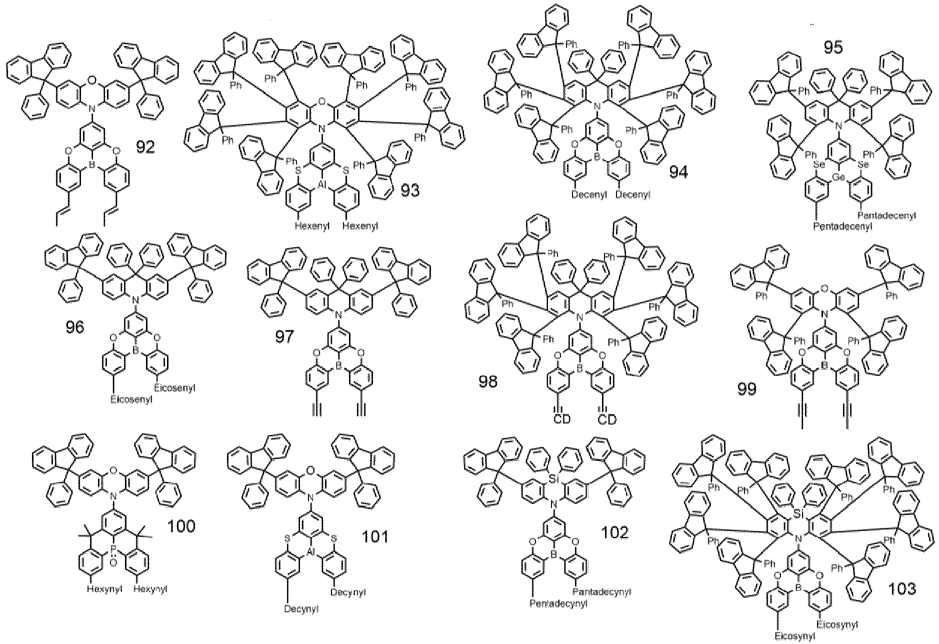

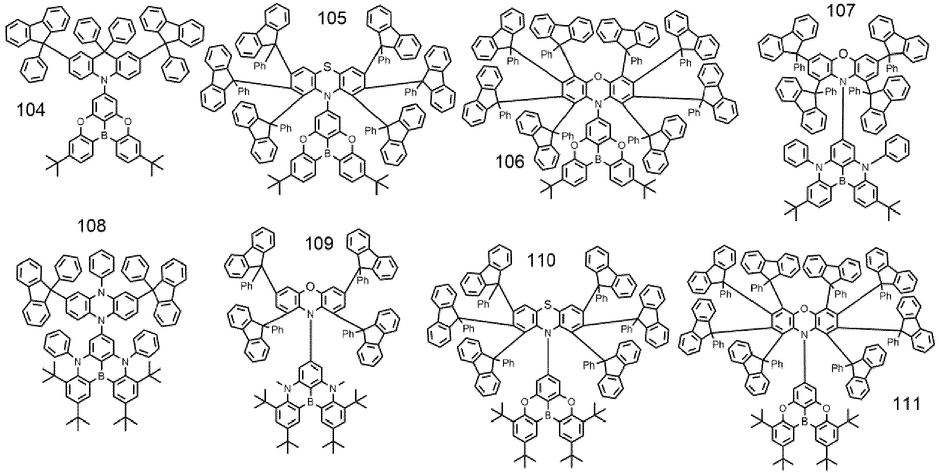

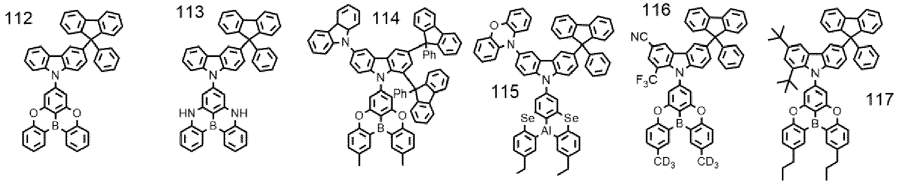

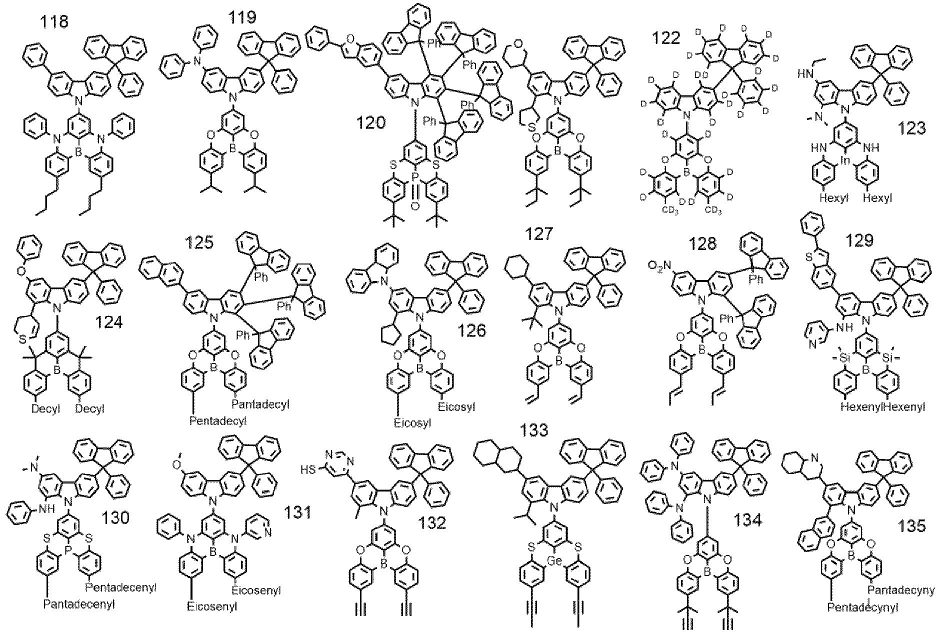

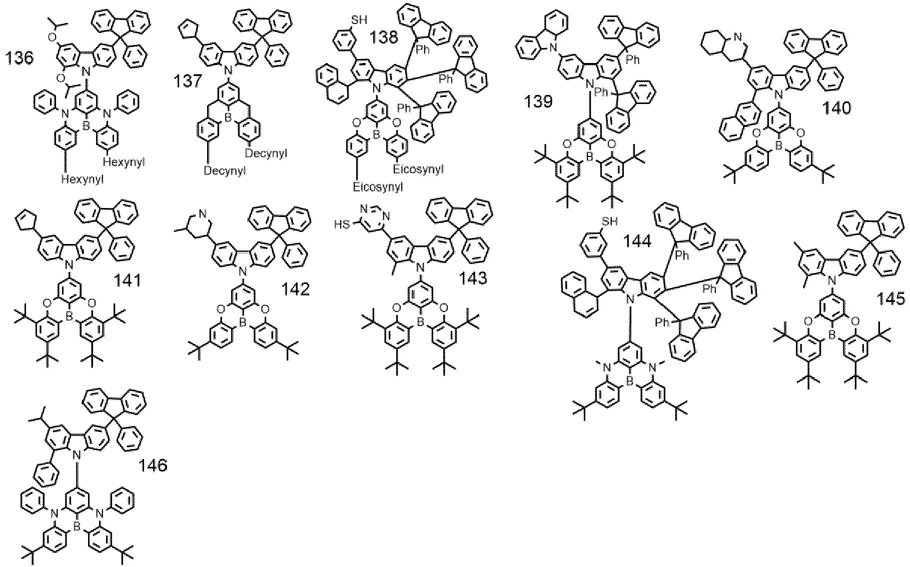

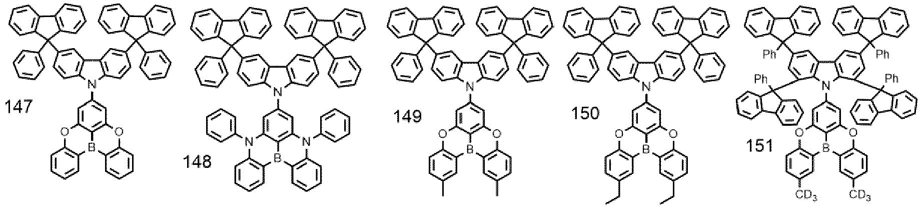

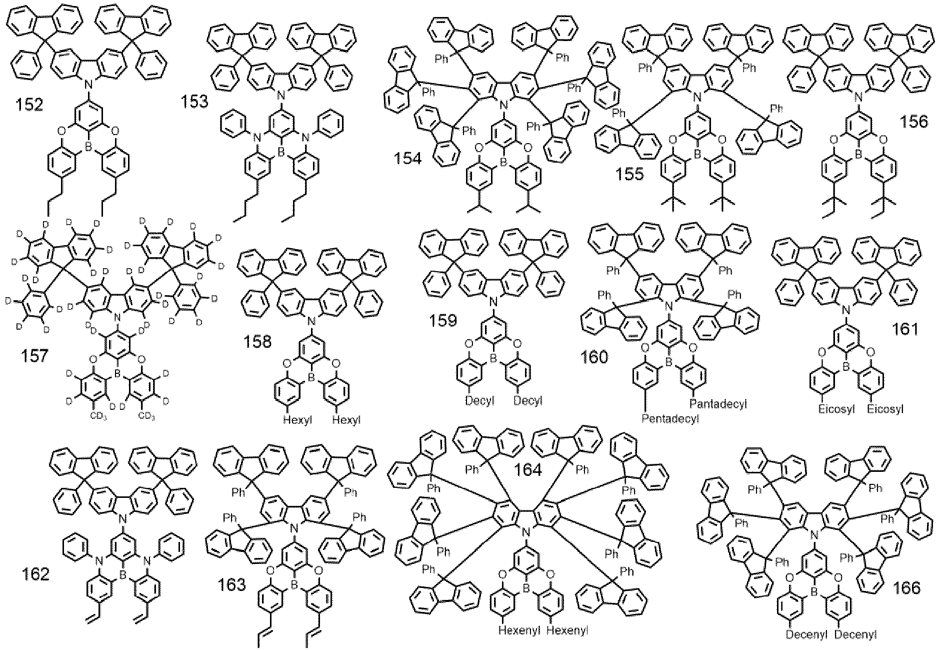

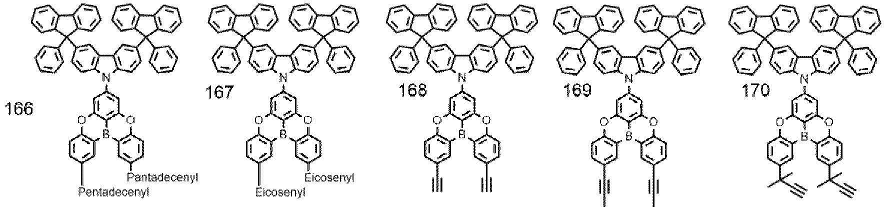

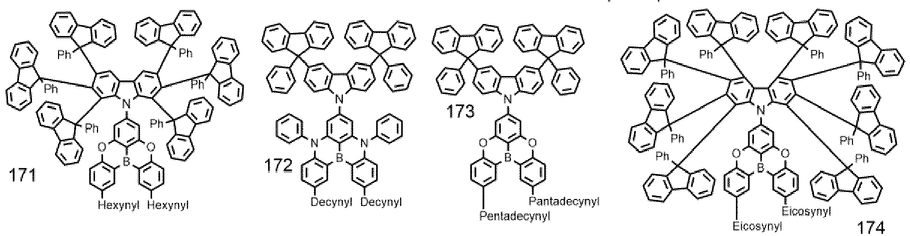

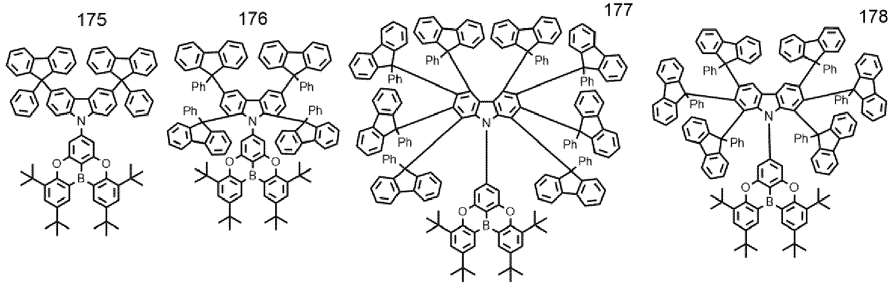

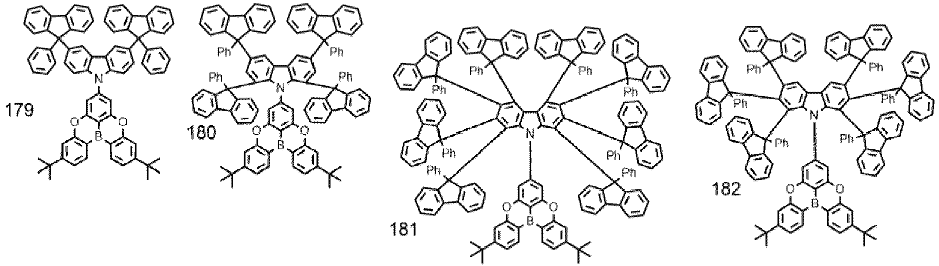

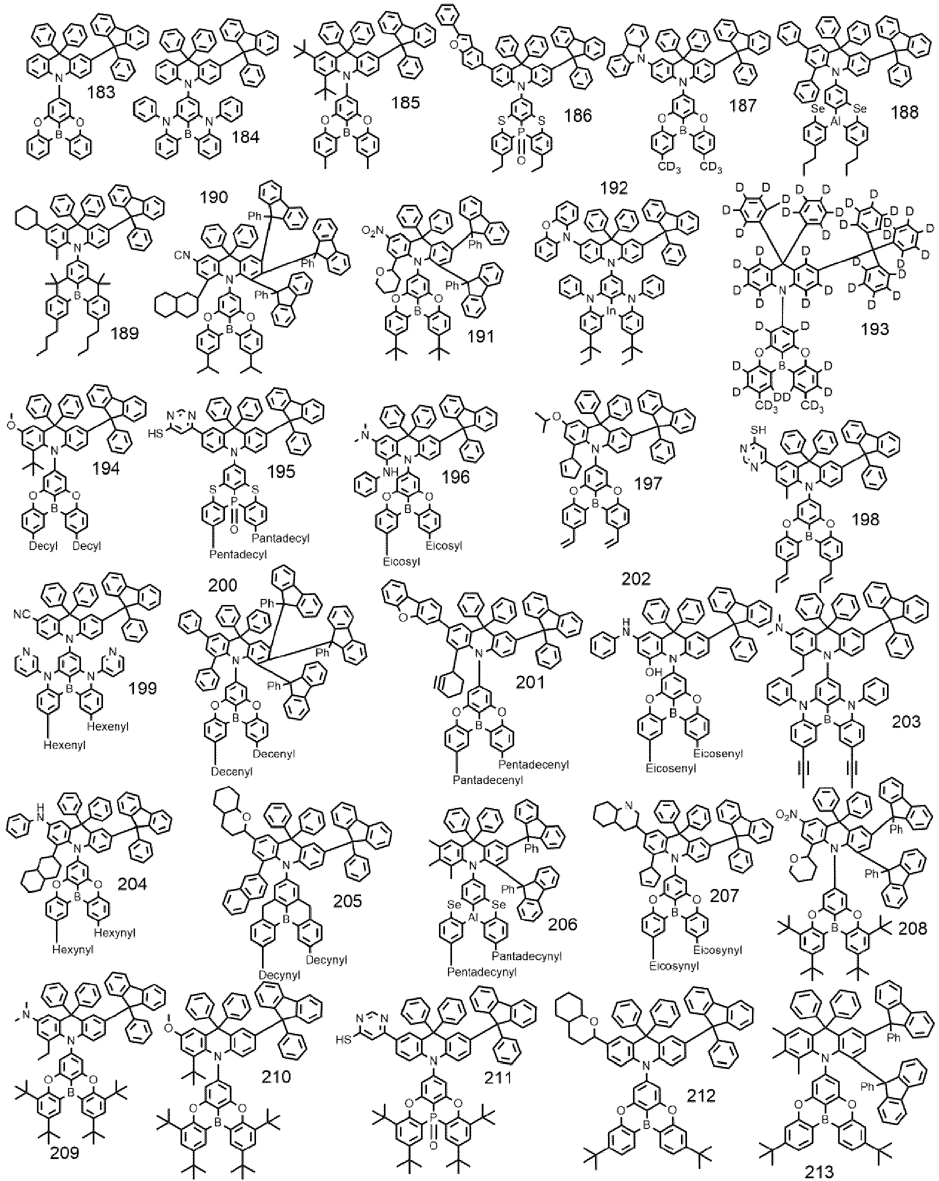

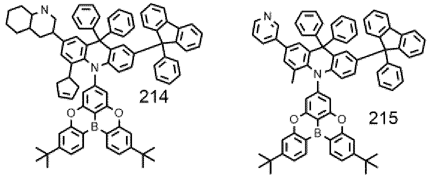

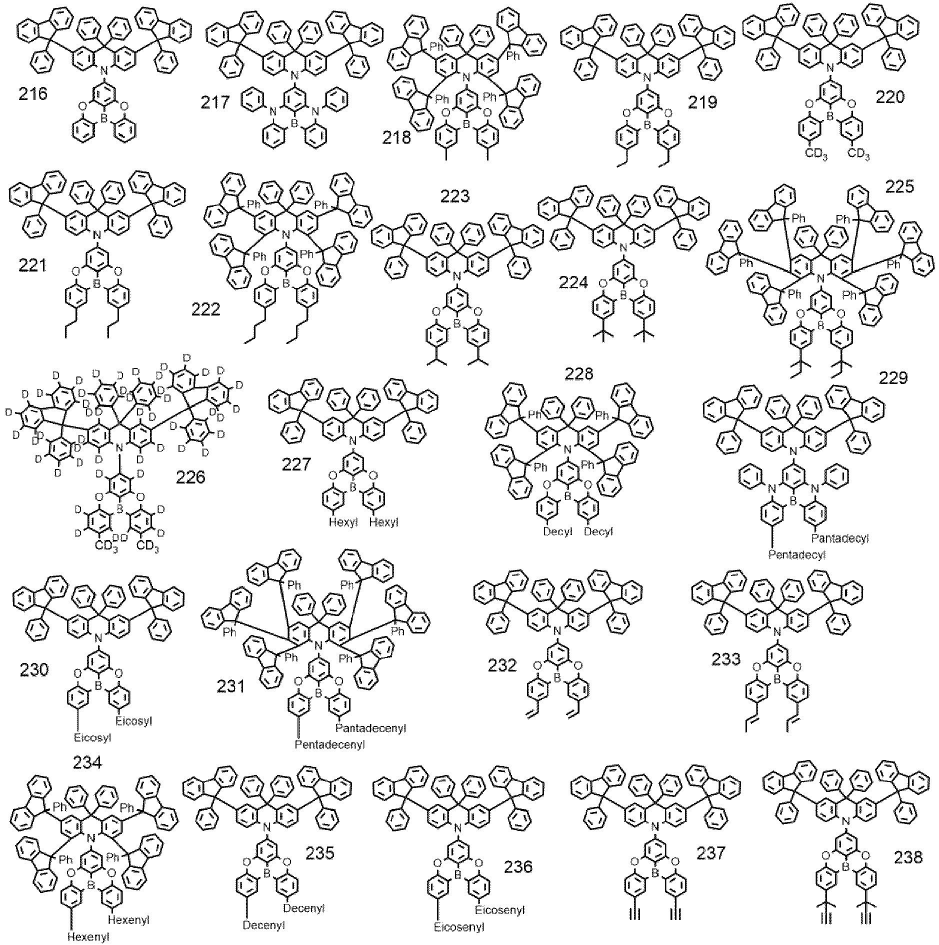

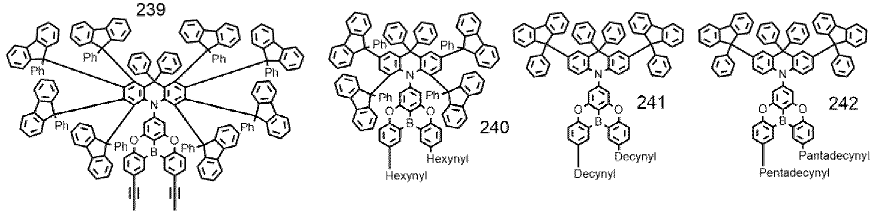

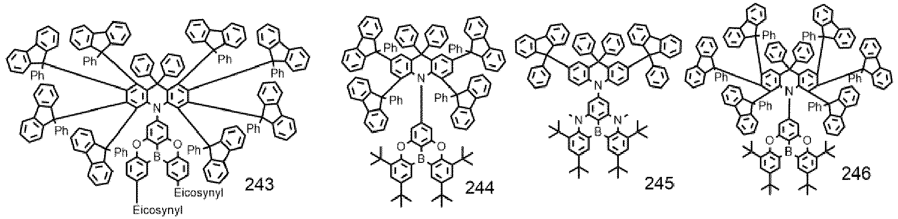

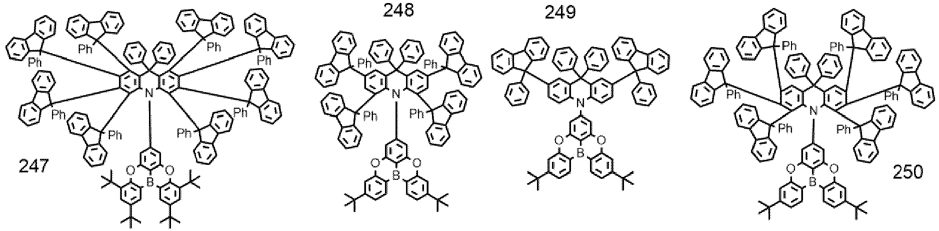

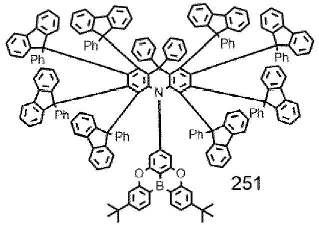

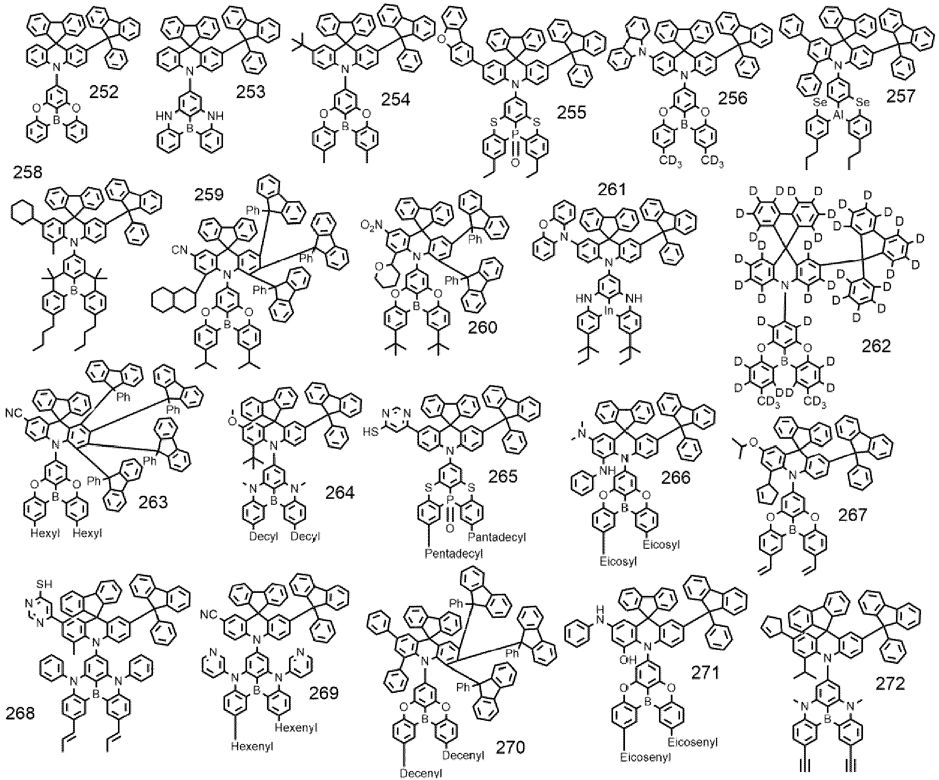

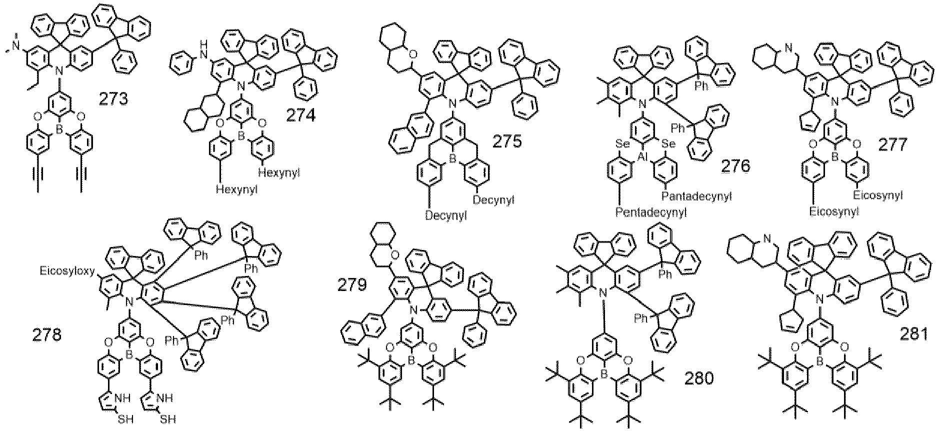

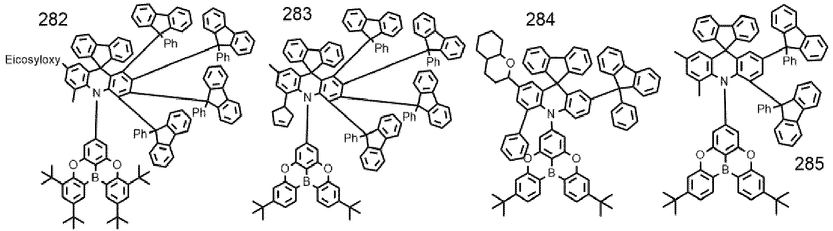

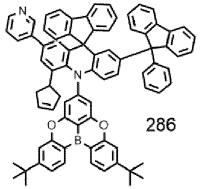

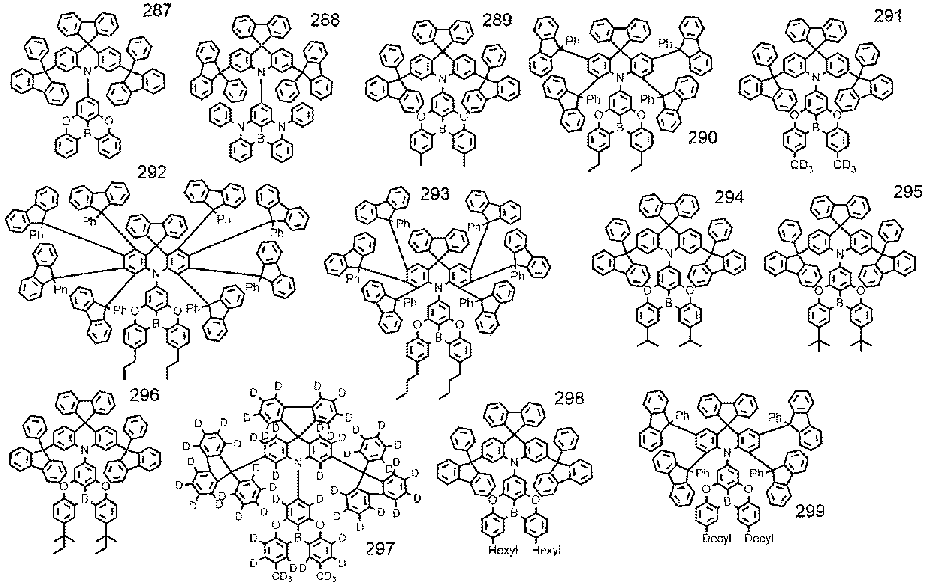

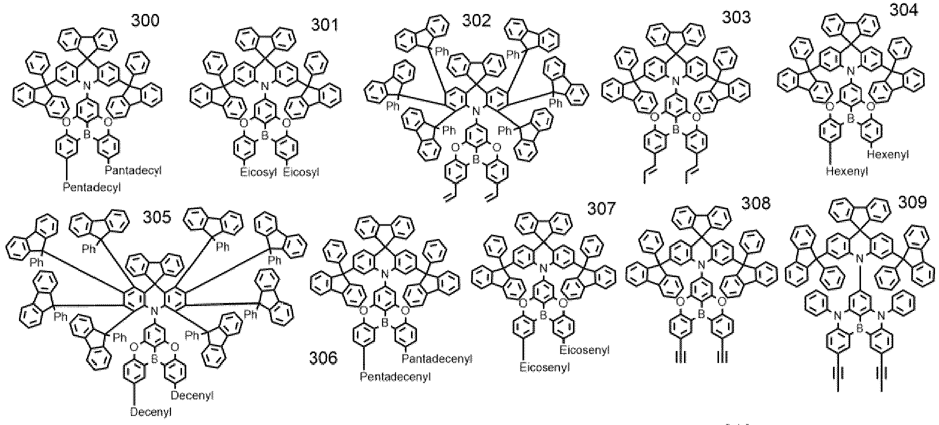

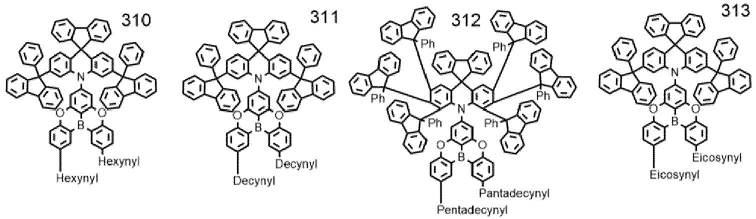

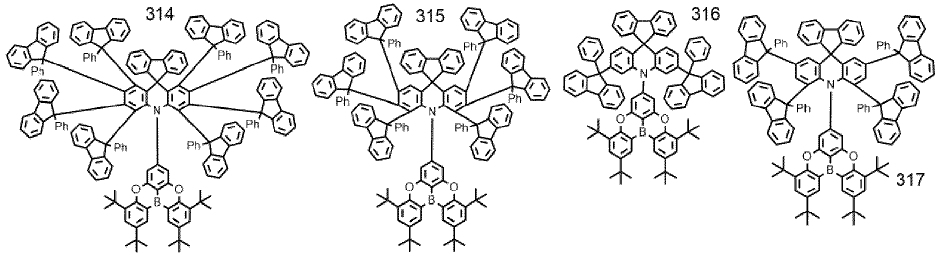

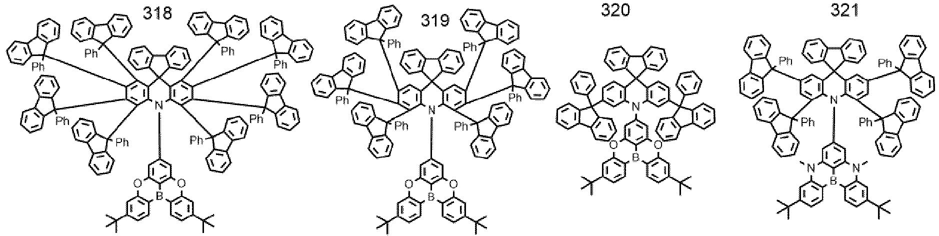

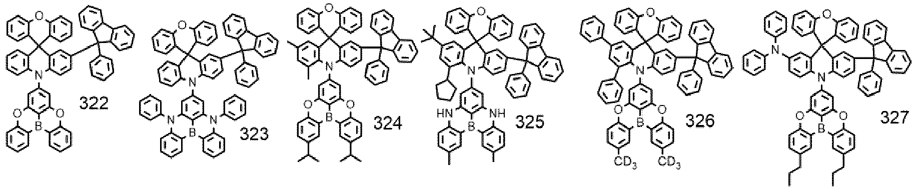

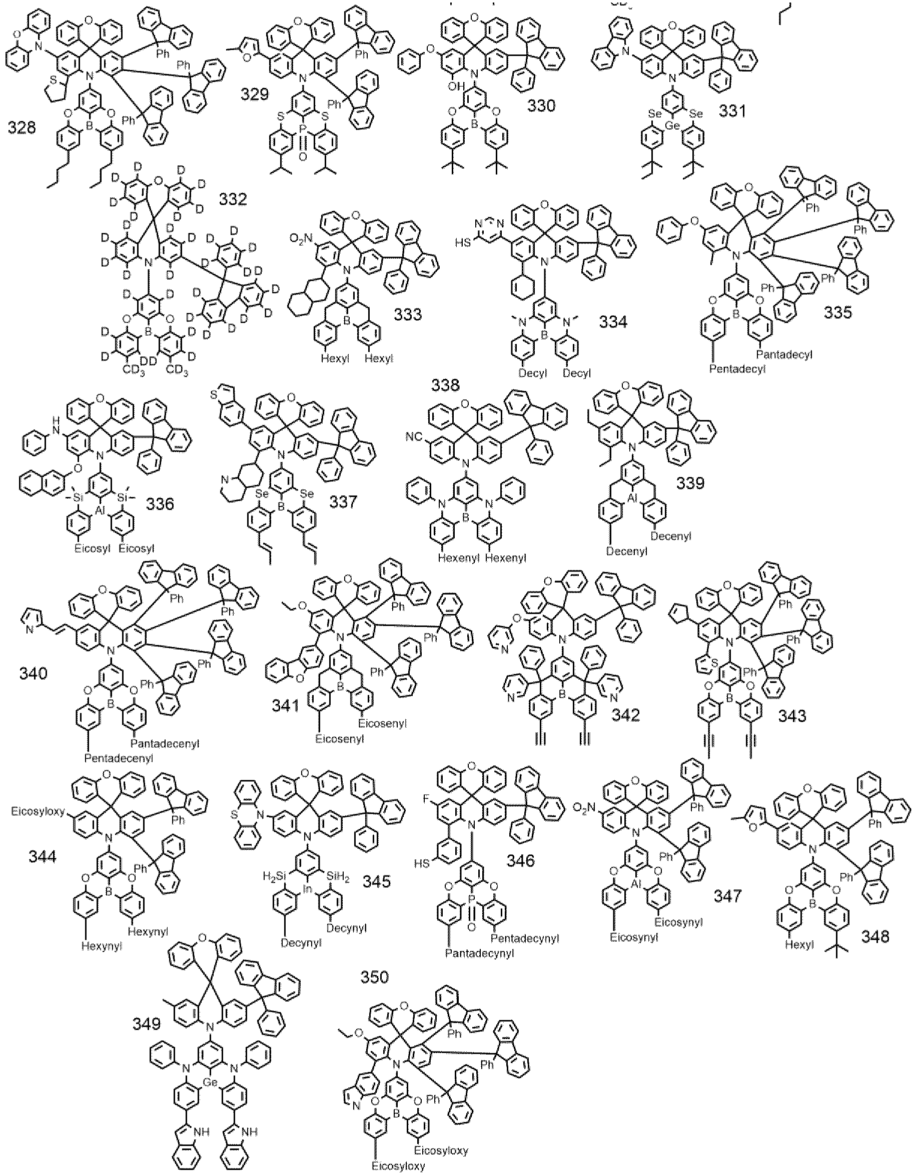

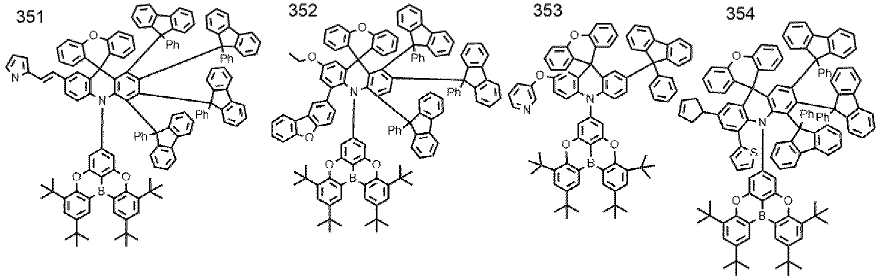

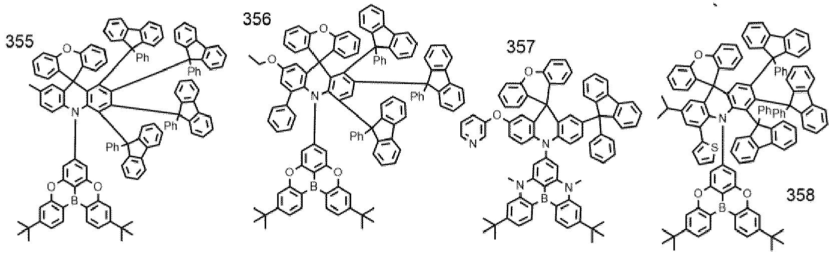

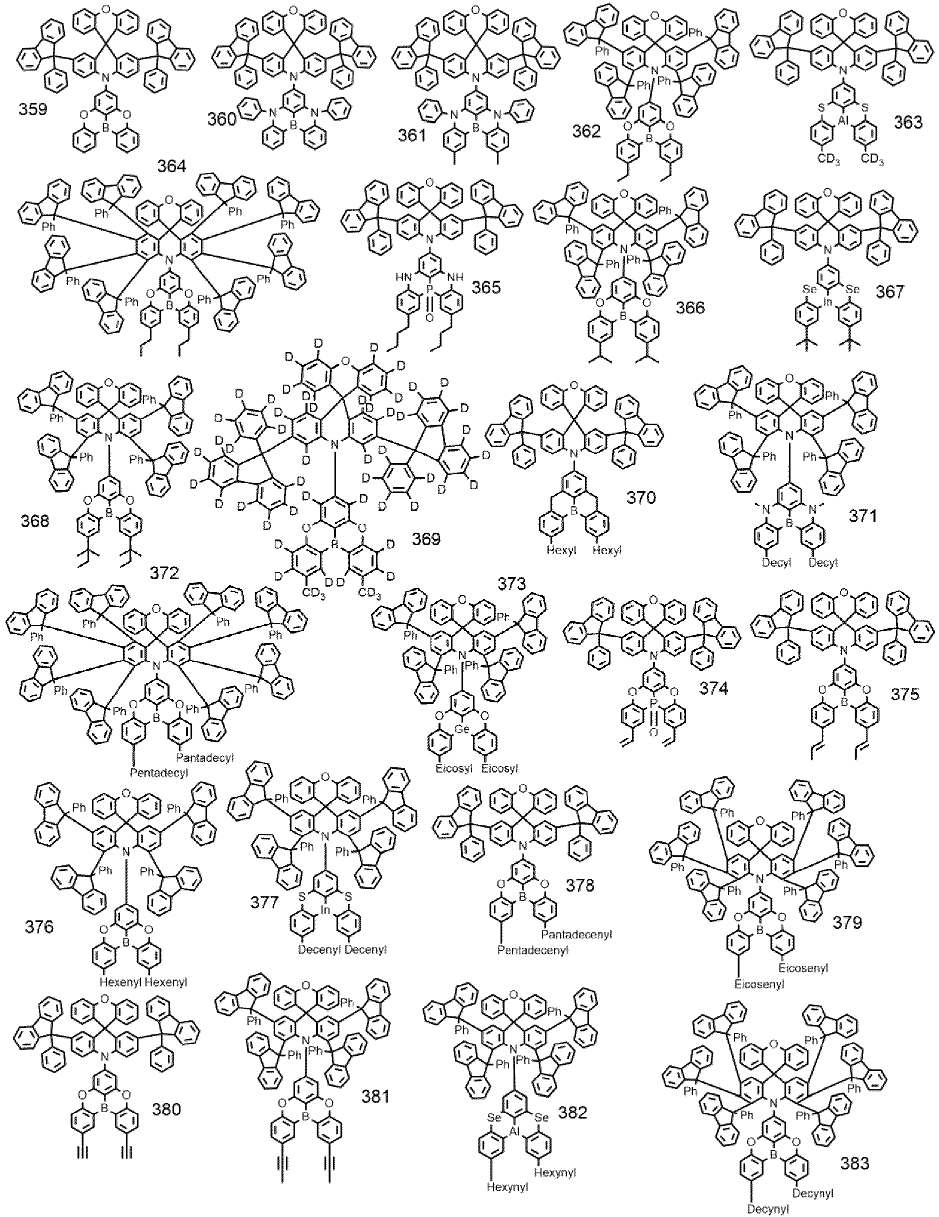

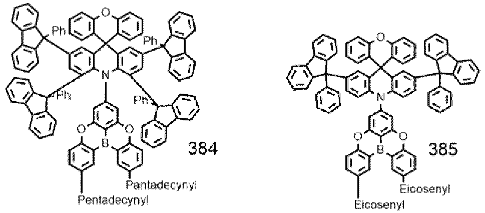

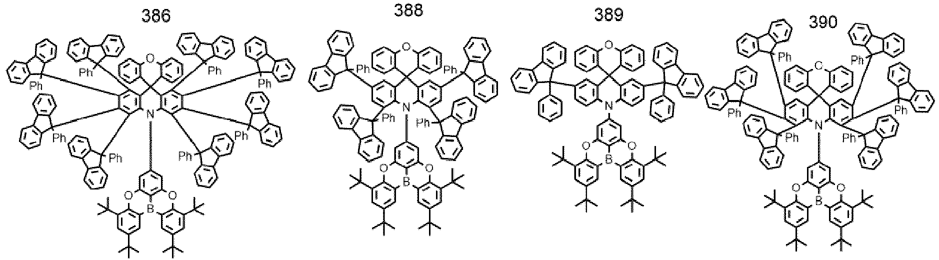

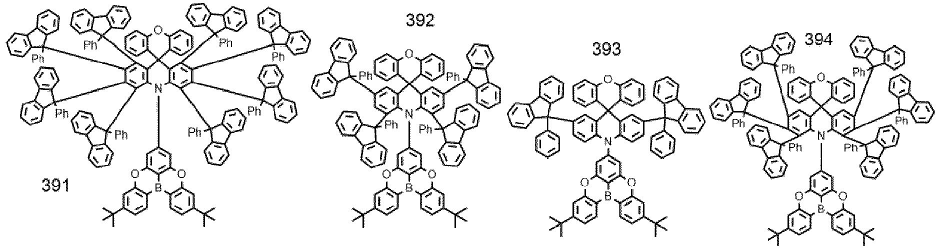

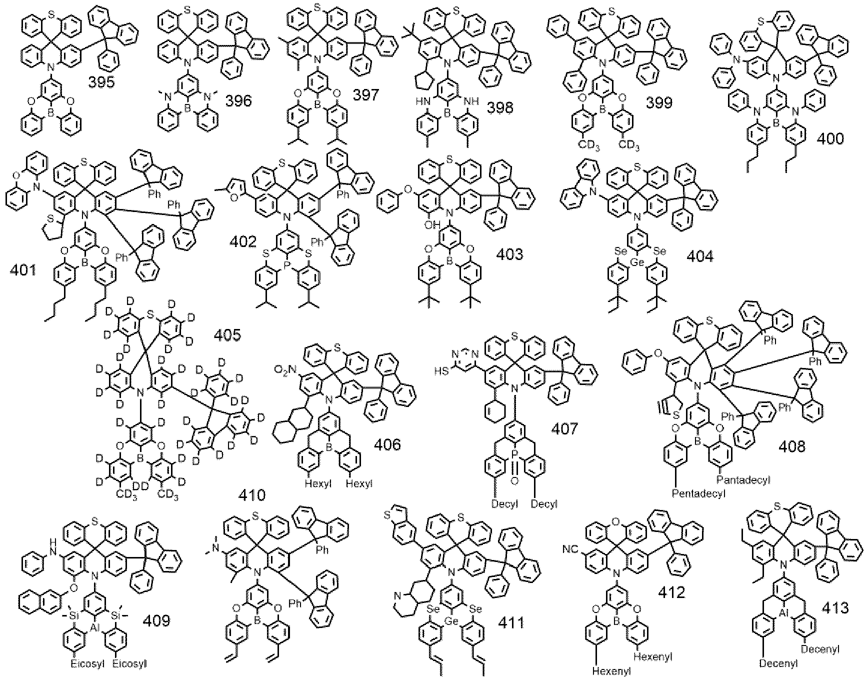

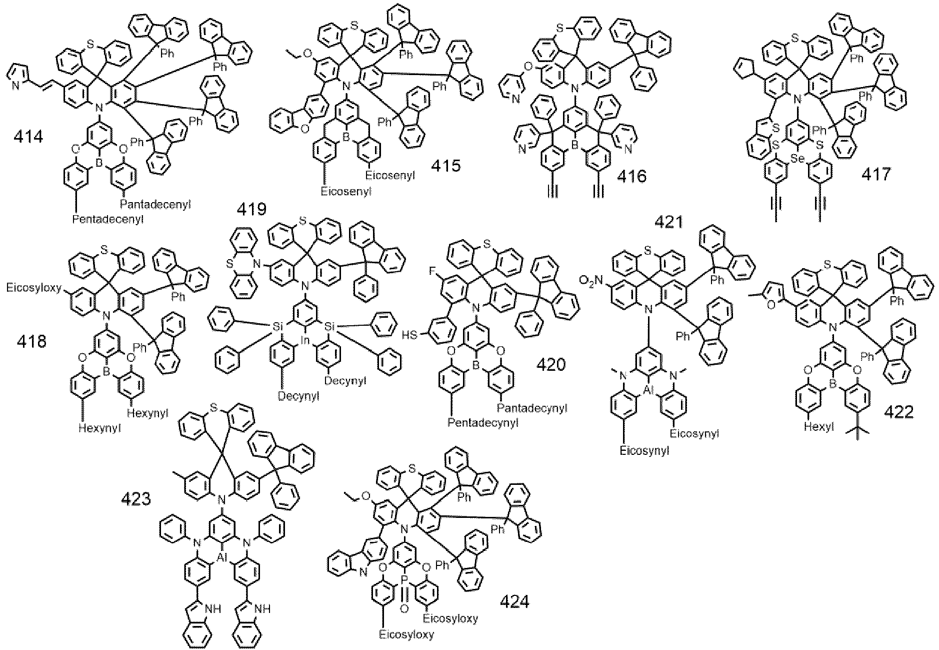

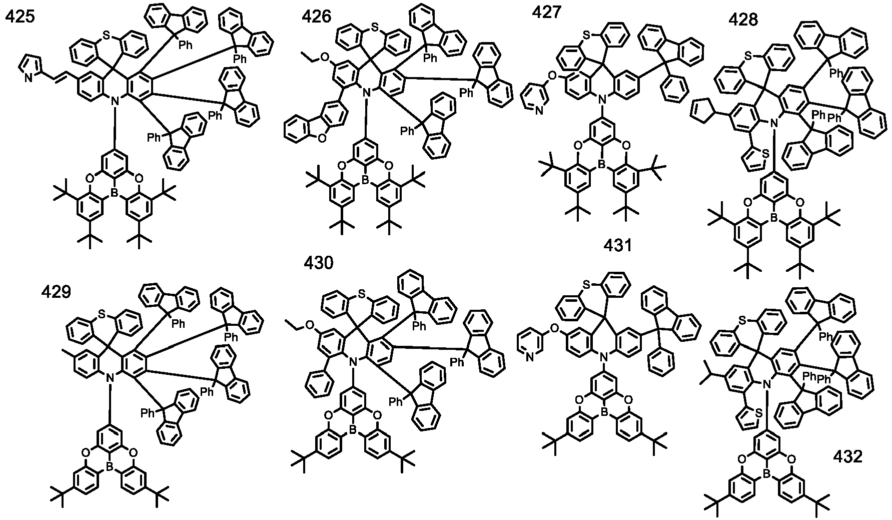

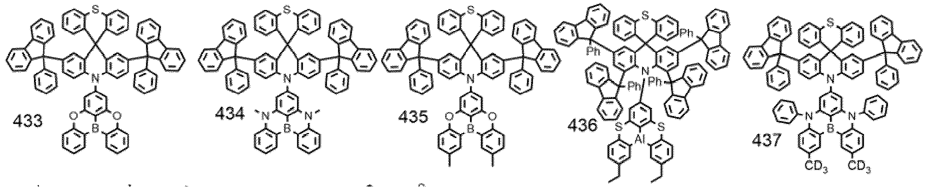

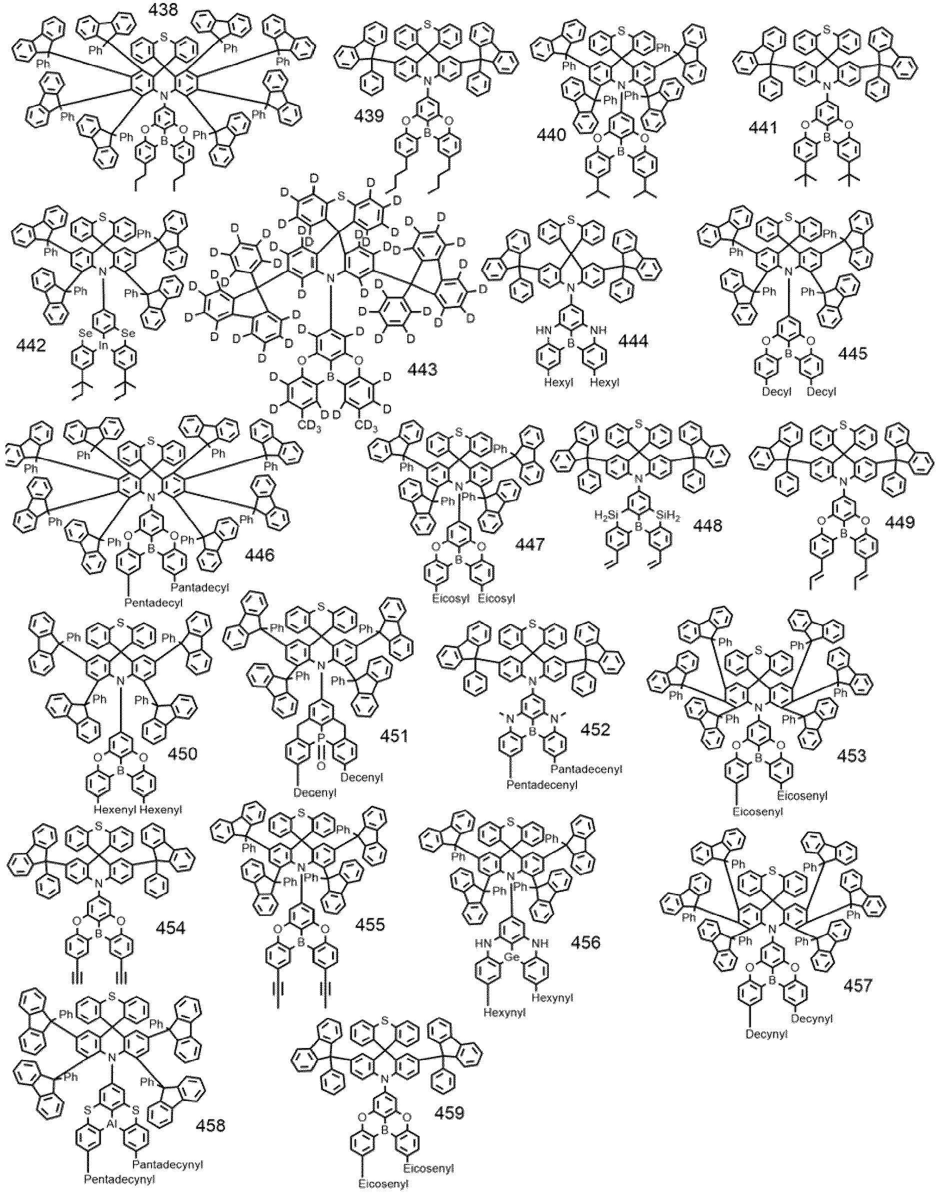

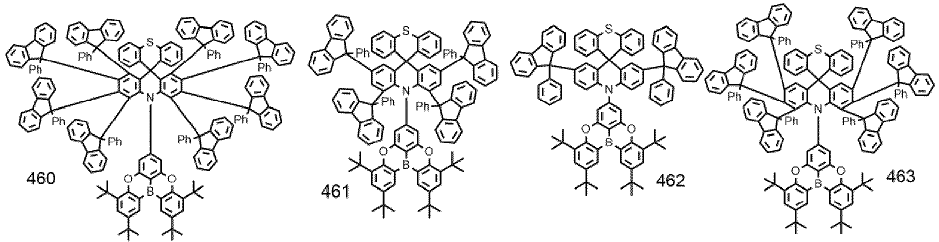

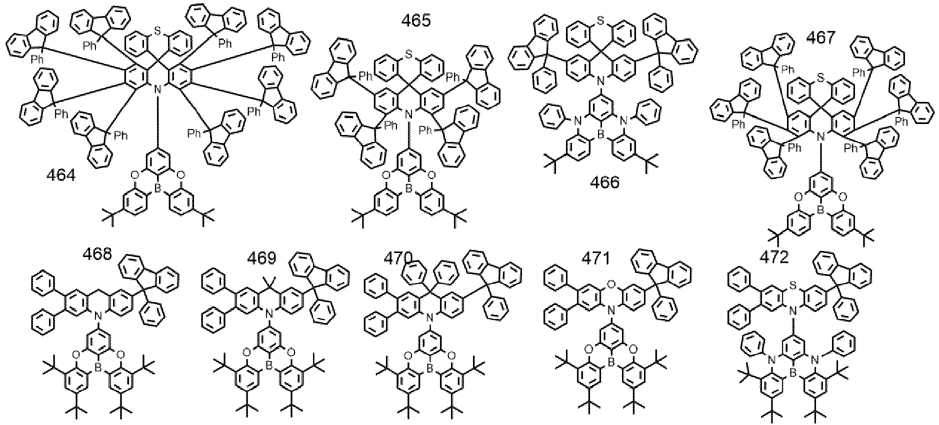

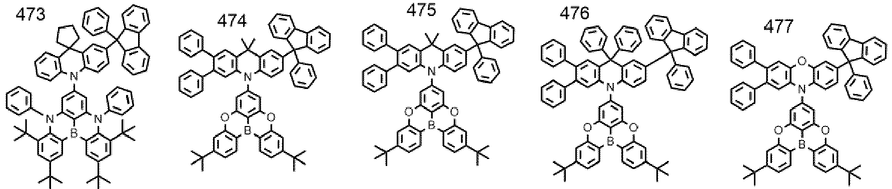

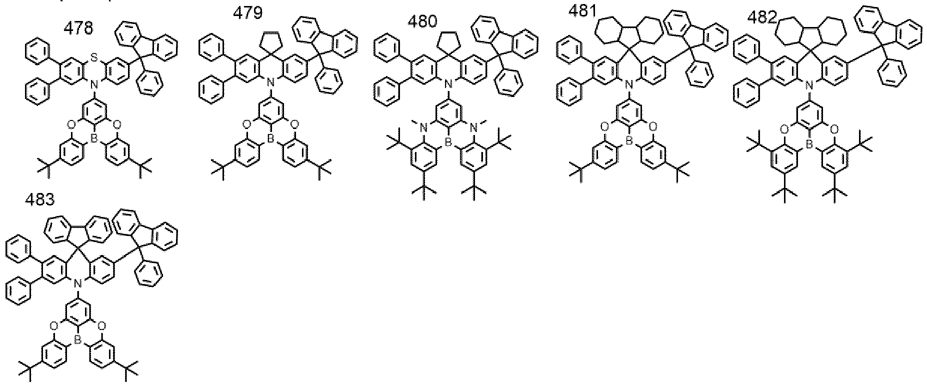

3. An organic light emitting diode (OLED) comprising an anode, cathode, and organic emissive layer, disposed between the anode and cathode, wherein the organic emissive layer comprises a compound according to claim 1 or 2.

4. An organic light emitting diode according to claim 3, wherein the organic emissive layer comprising v-DABNA as a fluorescent dopant.

5. An organic light emitting diode according to claim 3 or 4, wherein comprising a host selected from H1 - H163 and deuterated analogues thereof.

6. An organic light emitting diode according to any one claim 3-5, comprising at least one of layer between the anode and cathode selected from a hole injection layer; hole transport layer; electron transport layer; electron blocking layer; hole blocking layer; electron injection layer; or a combination thereof.

7. An organic light emitting diode according to any one of claims 3-6, comprising a matrix, preferably the matrix is passive or active.

8. An organic light emitting diode according to any one of claims 3 - 7, wherein the diode emits light with a maximum wavelength of 350 nm to 2 500 nm, more preferably with a maximum wavelength of 400 nm to 750 nm.

9. Use of the diode according to any one of claims 3 - 8 as a display screen or illumination device, an electroluminescent display device, an organic photovoltaic device, or a sensing device.

10. A consumer product comprises the diode according to any one of claims 3-8, wherein the product is selected from a flat panel display, a computer monitor, a medical monitor, a television, a billboard, a light for interior or exterior illumination/signalling, a heads-up display, a fully or partially transparent display, a flexible display, a laser printer, a telephone, a cell phone, tablet, a phablet, a personal digital assistant, a wearable device, a laptop computer, a watch, a backlight, a digital camera, a camcorder, a 3-D display, a virtual reality or augmented reality display, a vehicle, a video walls comprising multiple displays tiled together, a theatre, stadium screen, and a sign.
